# EUROPEAN PATENT APPLICATION

(11) **EP 2 202 221 A1**
(43) Date of publication of application: **30.06.2010**
(21) Application number: 08833577.3
(22) Date of filing: 25.09.2008
(51) Int. Cl.: C07C 275/42, C07D 209/48, C08G 73/18

(54) **ACETYLENE COMPOUND**

(30) Priority: 28.09.2007 JP 2007256372; 28.09.2007 JP 2007256501
(71) Applicant: Fujifilm Corporation, Tokyo 106-8620 (JP)
(72) Inventor: IMAKUNI, Akira, Odawara-shi Kanagawa 250-0001 (JP); NAKAYAMA, Masaya, Odawara-shi Kanagawa 250-0001 (JP); YAGIHARA, Morio, Odawara-shi Kanagawa 250-0001 (JP)
(74) Representative: HOFFMANN EITLE
(86) International application number: PCT/JP2008/067276
(87) International publication number: WO 2009/041482

(57) **Abstract**

The invention provides an acetylene compound represented by the following Formula (1), which is useful as a raw material for a polymer achieving high thermal resistance; 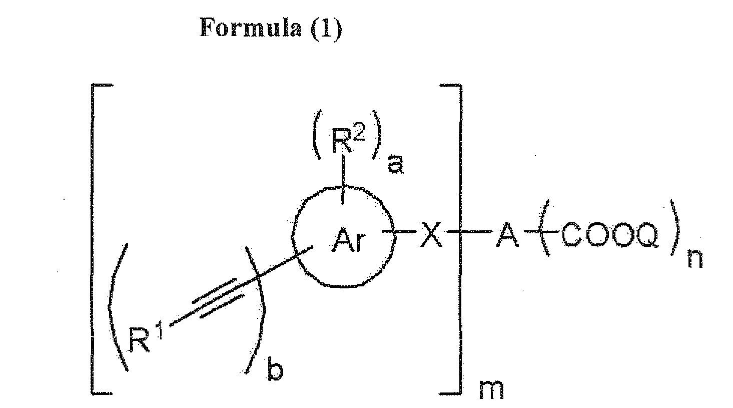 wherein, in Formula (1), the encircled Ar represents an aryl group or a heteroaryl group; X represents a divalent linking group; R, R' and R¹ each independently represent a hydrogen atom, a hydrocarbon group or a heterocyclic group; R² represents a hydrogen atom or a substituent substitutable on a benzene ring; A represents a hydrocarbon group or a heterocyclic group; Q represents a hydrogen atom, a hydrocarbon group, or a metal element capable of forming a monovalent metal salt; a represents an integer from 0 or more; b, m and n each independently represent an integer of 1 or more; when n, m and b are 1 at the same time, X is not -(C=O)O-; and when n is 2, and both m and b are 1, X is not -O-.

## Description

### [Technical Field]

The present invention relates to a novel acetylene compound which has a carboxylic acid or a derivative thereof as a functional group in the molecule and is useful for a raw material of functional material such as a thermosetting resin, a liquid crystal material, a nonlinear optical material, an electronic material, a material for adhesives, a material for sliding agents, a photographic additive or a material for a gas separation membrane, or for a raw material of an intermediate of medicinal and agricultural chemicals.

### [Background Art]

Compounds having an ethynyl group are important compounds as raw materials of functional materials such as a thermosetting resin, a liquid crystal material, a nonlinear optical material or the like, and in recent years, in particular, have attracted attention as the subject of research relating to various functional materials utilizing a carbon-carbon triple bond structure present in the molecule. For example, the compounds are used as materials for imparting heat resistance and oxidation resistance as well as thermosetting property to a polyimide oligomer (for example, U.S. Patent No. 5,567,800; POLYMER, Volume 35, pp. 4874-4880 and 4857-4864 (1994); FUNCTIONAL MATERIAL, Volume 20 (12), pp. 33-40 and the like). As known materials, although compounds represented by the following Formula (14), in which R⁵ represents a hydrogen atom or an alkyl group, and R⁶ represents an alkyl group or an aryl group, (for example Japanese Patent Application Laid-Open (JP-A) No. 2002-265414) are reported, there is a problem in that the selection of materials as the subject of research relating to functional materials is narrow.

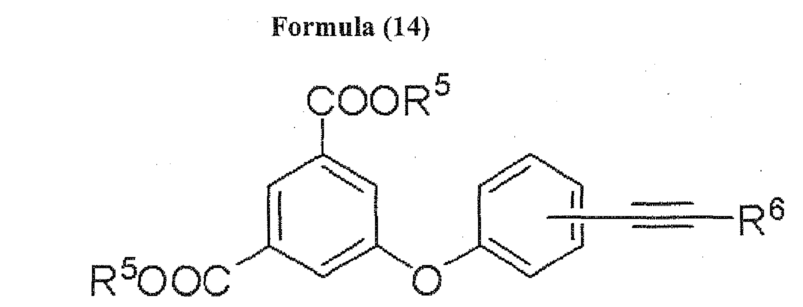

In the manufacturing process of such similar compounds, there has only been reported a compound obtained by allowing a leaving group L in the carboxylic compound represented by the following Formula (15) to react with a compound having an ethynyl group represented by the following Formula (16) to remove the hydrogen atom therefrom (for example, JP-A No. 2002-265414). For example, when a compound in which R⁵ is a hydrogen atom is synthesized, it is necessary to protect the ethynyl group, but there has been a problem in that the compound may not be obtained with sufficient yield resulting from the hydrolysis of the intended product at the time of the deprotecting reaction.
Further, there has been no example of a compound having plural carbon-carbon triple bond structures.

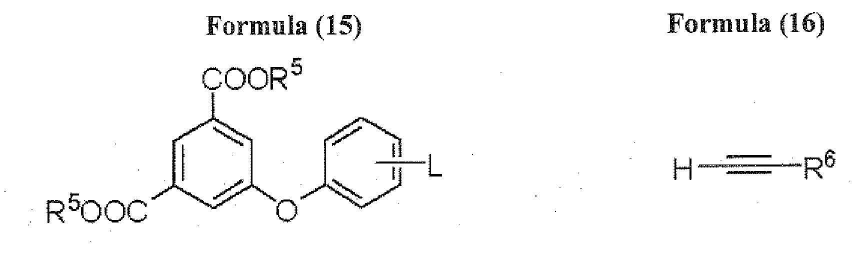

### [Disclosure of the Invention]

### [Problem to be Solved by the Invention]

An object of the present invention is to provide a novel acetylene compound having a structure in which a unit having a carboxylic acid or a derivative thereof as a functional group capable of being introduced into a condensed polymer is linked to a unit having one or more ethynyl groups via a linking group.

### [Means for Solving Problem]

As the result of intensive studies by the inventors in consideration of the above circumstances, a novel acetylene compound having a structure in which a unit having a carboxylic acid or a derivative thereof as a functional group is linked to a unit having one or more ethynyl groups via a linking group, derivatives of the compound, and a polymer having the compound as a structural unit have been found, and further a manufacturing method thereof has been found, thereby achieving the present invention. That is, specifically, the subject of the present invention has been achieved by the following means.

<1> A compound represented by the following Formula (1):

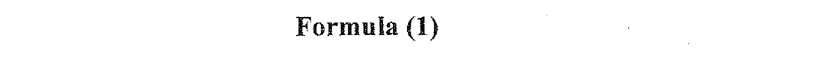

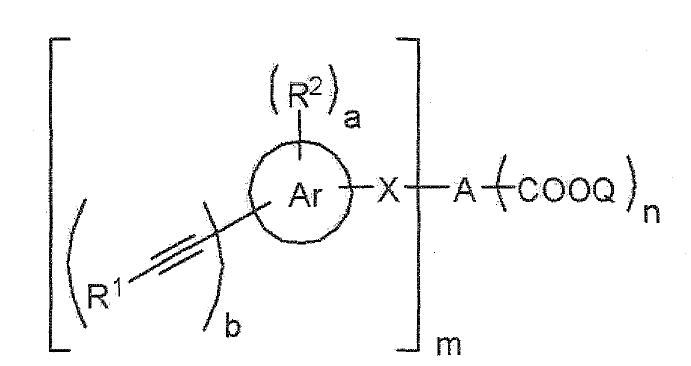

(in Formula (1), the encircled Ar represents an aryl group or a heteroaryl group; X represents a divalent linking group represented by -NR(C=O)-, -NR(C=O)O-, -NR(C=O)NR'-, -(C=O)-, -(C=O)O-, -O(C=O)O-, -(C=O)S-, -NR(C=S)-, -NR(C=S)NR'-, -O(C=S)O-, -O- or -S-; R, R' and R¹ each represent a hydrogen atom, a hydrocarbon group or a heterocyclic group. R² represents a hydrogen atom or a substituent substitutable on a benzene ring. A represents a hydrocarbon group or a heterocyclic group, and Q represents a hydrogen atom, a hydrocarbon group, or a metal element capable of forming a monovalent metal salt. a represents an integer of 0 or more, b represents an integer of 1 or more, m represents an integer of 1 or more, and n represents and integer of 1 or more. However, when n, m and b are 1 at the same time, X is not -(C=O)O-, and when n is 2, and both m and b are 1, X is not -O-).

<2> The compound according to <1>, characterized in that the compound according to <1> is represented by the following Formula (2);

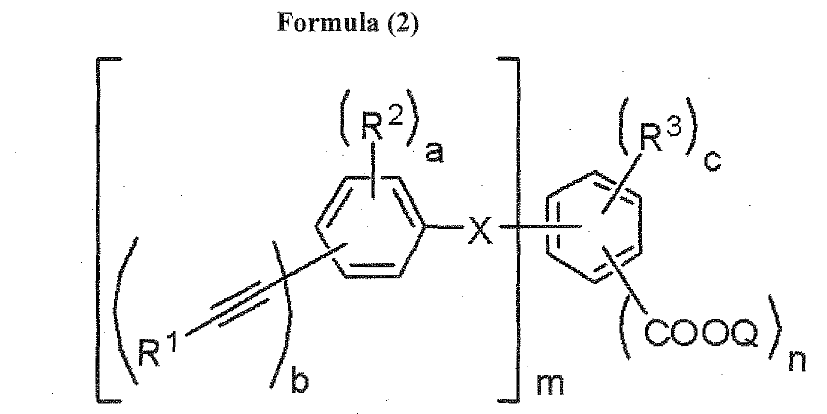

In Formula (2), X represents a divalent linking group represented by -NR(C=O)-, -NR(C=O)O-, -NR(C=O)NR'-, or -(C=O)O-. R³ represents a hydrogen atom or a functional group substitutable on the benzene ring, and R¹; R²; R, R' and Q each have the same definitions as those of R¹; R²; R, R' and Q in Formula (1). a represents an integer from 0 to 4, b represents an integer from 1 to 5, c represents an integer from 0 to 3, m represents an integer from 1 to 5, n represents an integer from 1 to 5, the sum of c, m and n is 6. However, when n, m and b are 1 at the same time, X is not -(C=O)O-.

<3> The compound according to <2>, characterized in that in Formula (2), m is an integer from 2 to 5, n is 1; and X represents a divalent linking group represented by -NR(C=O)-, -NR(C=O)O-, or -NR(C=O)NR'-.
<4> The compound according to <2>, characterized in that in Formula (2), m is 1, and n is an integer from 2 to 5.
<5> The compound according to <4>, characterized in that in Formula (2), n is 2.
<6> The compound according to <5>, characterized in that in Formula (2), b is 1.
<7> The compound according to <3>, characterized in that in Formula (2), m is 2.
<8> The compound according to <2>, characterized in that in Formula (2), b is 1, the substitution position of the ethynyl group substituted by R¹ is the meta-position or the para-position with respect to the linking group X.

<9> The compound according to <2>, characterized in that the compound represented by Formula (2) is represented by the following Formula (3); 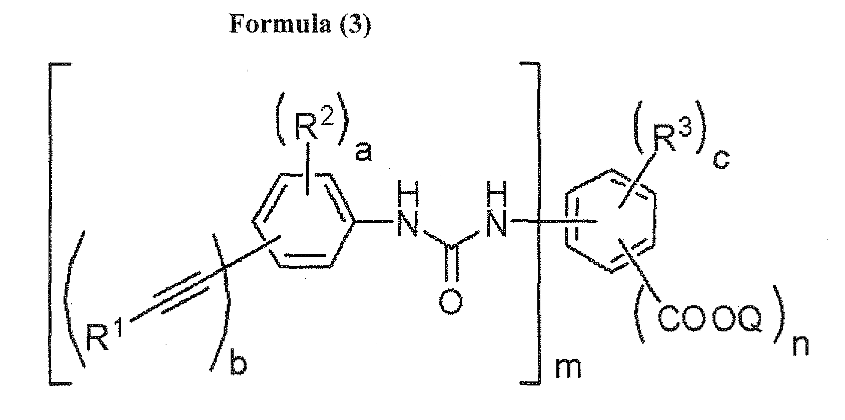 (in Formula (3), R¹, R², R³, Q, a, b, c, m and n each have the same definitions as those of R¹, R², R³, Q, a, b, c, m and n in Formula (2)).

<10> The compound according to <2>, characterized in that the compound represented by Formula (2) is represented by the following Formula (4); 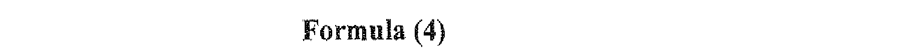 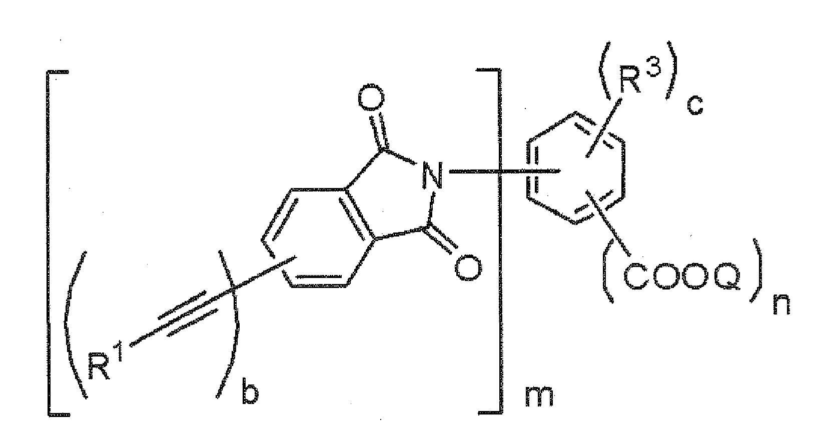 (in Formula (4), b represents an integer from 1 to 4; and R¹, R³, Q, c, m and n each have the same definitions as those of R¹, R³, Q, c, m and n in Formula (2)).

<11> The compound according to <9>, characterized in that in Formula (3), m is 2, and n is 1.
<12> The compound according to <10>, characterized in that in Formula (4), m is 2, and n is 1.

<13> A compound represented by the following Formula (5), characterized in that the compound is formed by allowing the compound represented by <1> to react with a compound having a functional group capable of reacting with a carboxyl group and an ethynyl group substituted with one or more R^{1e} groups: 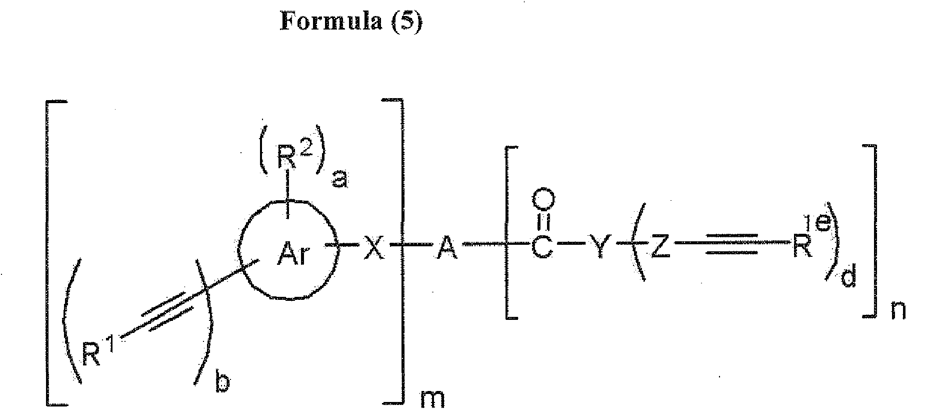 (in Formula (5), R¹, R², the encircled Ar, X, A, a, b, m and n each have the same definitions as those of R¹, R², the encircled Ar, X, A, a, b, m and n in Formula (1), d, Z and R^{1e} each have the same definitions as those of b, A and R¹ in Formula (1), and Y represents -NR-, -O- or -S-. R has the same definition as that of R in Formula (1).
<14> The compound according to <13>, characterized in that in Formula (5), m is 2 and n is 1.

<15> A polymer which contains at least a compound in any one of <1> to <12> as a constitutional unit.
<16> The polymer according to <15>, characterized in that in the polymer, a constitutional unit other than the constitutional units formed by the compounds recited in any one of <1> to <12> contains any of constitutional unit including a condensate of a dicarboxylic acid compound and a tetraamino compound, a condensate of a dicarboxylic acid compound and a bis(orthohydroxyamino) compound, a condensate of a dicarboxylic acid compound and a diamine compound, or a condensate of a dicarboxylic acid compound and a diol compound.
<17> The polymer according to <15>, characterized in that the polymer has a block copolymer structure of two or more kinds of polymers.
<18> The polymer according to <15>, characterized in that in the polymer, a constitutional unit other than the constitutional units formed by the compounds recited in any one of <1> to <12> contains any of constitutional unit including a condensate of a dicarboxylic acid compound and a tetraamino compound, or a condensate of a dicarboxylic acid compound and a bis(orthohydroxyamino) compound.

<19> A method of obtaining a carboxylic acid compound represented by the following Formula (10) which is a compound according to <9>, in which an amino group of an amino carboxylic ester represented by the following Formula (6) is converted into a carbamate by using a halogenocarbonate, and the carbamate is allowed to react with an amino group-containing acetylene compound represented by the following Formula (8) to synthesize an ester compound having an ethynyl group substituted with R¹ represented by the following Formula (9), and the ester compound is hydrolyzed:

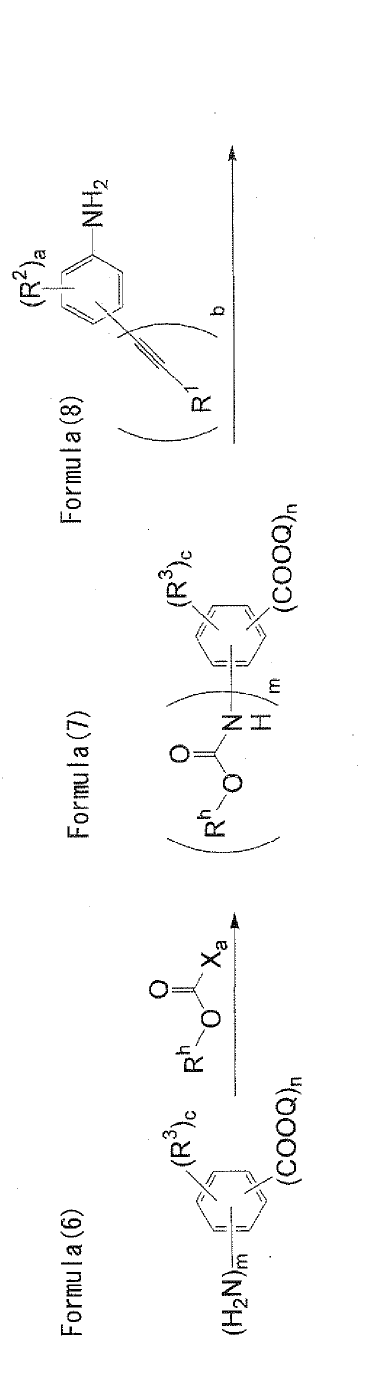

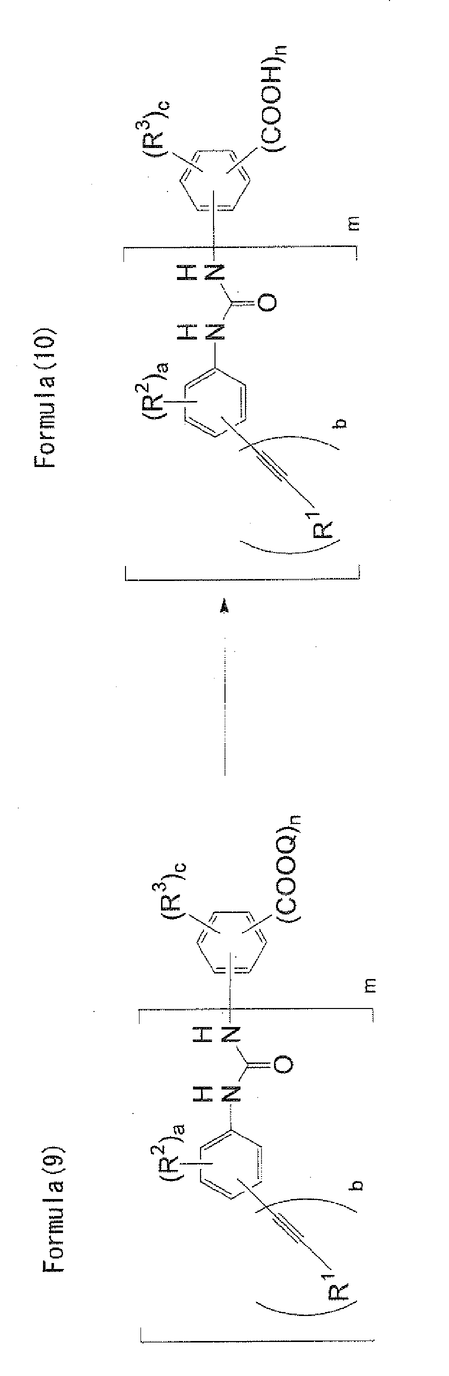

(here, R^{h} represents a hydrogen atom or a hydrocarbon group, and Xa represents a halogen atom. R¹, R², R³, Q, a, b, c, m and n each have the same definitions as those of R¹, R², R³, Q, a, b, c, m and n in Formula (3), respectively).

<20> The manufacturing method according to <19>, characterized in the carboxylic acid compound represented by the Formula (10) is continuously manufactured by without taking out an intermediate.
<21> The manufacturing method according to <19>, characterized in that m and n each are 1 or 2, and m+n =3.

<22> The manufacturing method according to <10>, characterized in that an amino carboxylic acid derivative represented by the following Formula (11) is allowed to react with an acetylene compound having an ethynyl group substituted with R¹ represented by the following Formula (12) to convert into an amic acid compound represented by the following Formula (13), and further the amic acid compound is cyclized, thereby synthesizing the compound represented by the Formula (4), and a series of these processes are continuously performed without taking out an amic acid compound represented by the Formula (13).

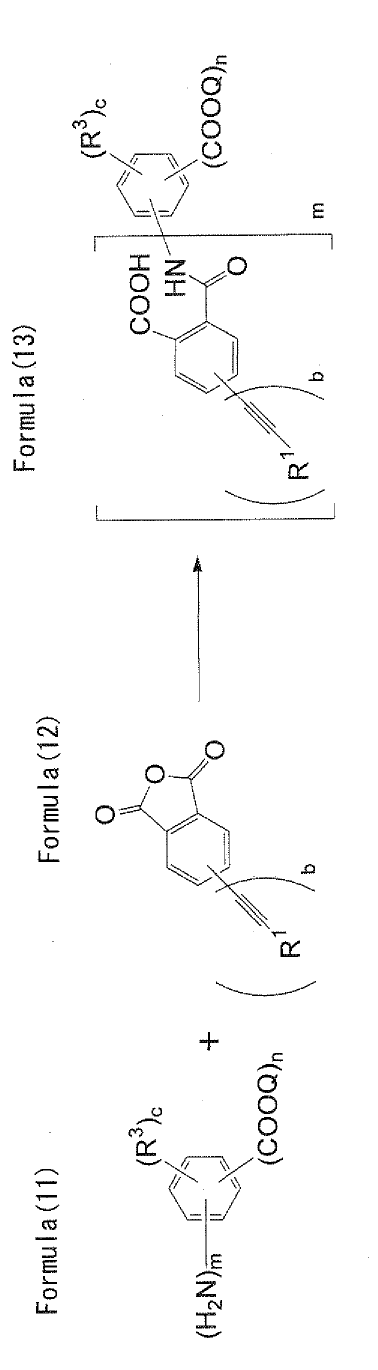

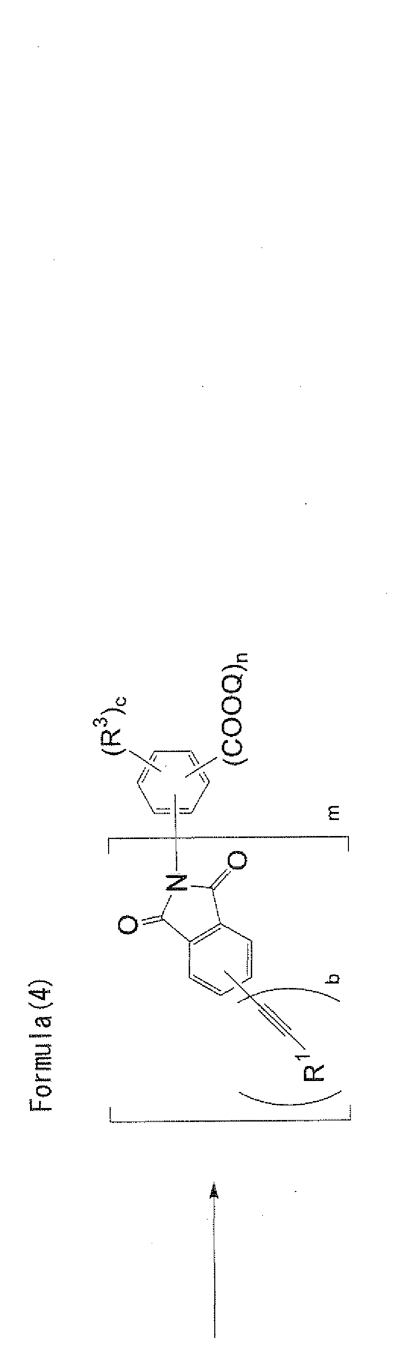

(here, R¹, R³, b, c, m and n each have the same definitions as those of R¹, R³, b, c, m and n in Formula (4)).
<23> The manufacturing method according to <22>, characterized in that m and n each are 1 or 2, and m+n=3.

<24> A composition containing a compound recited in at least any one of <1> to <14>, and/or a polymer recited in at least any one of <15> to <18>.
<25> A cured product formed by curing the composition recited in <24>.

### [Effect of the invention]

According to the invention, a novel acetylene compound having a structure in which a unit including a carboxylic acid or a derivative thereof as a functional group capable of being introduced into a condensed polymer is linked via a linking group to a unit having one or more ethynyl groups can be provided.

### [Best mode for carrying out of the invention]

Hereinafter, the invention will be explained in detail.

### <Explanation about an acetylene compound>

### Explanation of Formula (1)

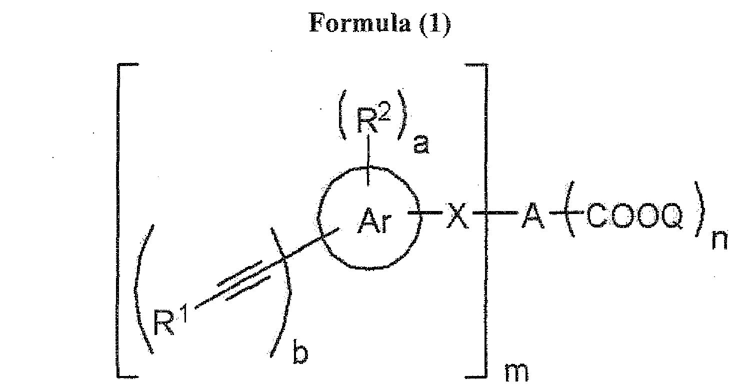

The encircled Ar represents a (a+b +1) valent aryl group or heteroaryl group which may be optionally substituted. Examples of the aryl group include phenyl, naphthyl, fluorenyl, anthranyl and the like. Examples of the heteroaryl group include pyridyl, furyl, thiophenyl, imidazolyl, indolyl and the like. An aryl group such as phenyl, naphthyl or the like is preferable, and a phenyl group is more preferable.

X represents a divalent linking group represented by -NR(C=O)-, -NR(C=O)O-, -NR(C=O)NR'-, -(C=O)-. -(C=O)O-, -O(C=O)O-, -(C=C)S-, -NR(C=S)-, -NR(C=S)NR'-, -O(C=S)O-, -O- or -S-; and X and R² may be linked each other to form a ring, and it is preferable that X be any one of -(C=O)O-, -NR(C=O)-, -NR(C=O)- and -NR(C=S)NR', or X be mutually linked with R² to form an imide ring, and it is more preferable that X be -NR(C=S)NR', or X be mutually linked with R² to form an imide ring. However, when n, m and b each are 1 at the same time, X is not -(C=O)O-, and when n is 2 and both m and b are 1, X is not -O-.

R, R' and R¹ each represent a hydrogen atom, or an unsubstituted or optionally substituted cyclic or non-cyclic hydrocarbon group, heterocyclic group or alkylsilyl group. As the unsbstituted hydrocarbon group, groups such as an alkyl group having 1 to 20 carbon atoms (for example, methyl, ethyl, butyl, octyl, hexadecyl or the like), an alicyclic group having 1 to 20 carbon atoms (for example, cyclopentyl, cyclohexyl, cyclohexenyl or the like), an alicyclic polycyclic group having 1 to 20 carbon atom (for example, bornyl, norbonyl, decanyl, adamantyl, diamantyl or the like), or a spiro ring having 1 to 20 carbon atoms (for example, spiro[3.4]octane, spiro[4.4]nonane, spiro[5.5]undecane or the like); or an aryl group having 1 to 20 carbon atoms (for example, phenyl, naphthyl, anthranyl or the like) may be exemplified. Examples of the optionally substituted hydrocarbon group include the hydrocarbon groups substituted with a halogen atom (for example a fluorine atom, a chlorine atom, a bromine atom or an iodine atom), a cyano group, a nitro group, an alkoxy group having 1 to 20 carbon atoms (for example, methoxy, butoxy or dodecyloxy), an aryl group such as phenyl, naphthyl or the like, a hydroxyl group, a silyl group and the like. Examples of the alkylsilyl groups include a trimethylsilyl, a triethylsilyl, a diisopropyl methylsilyl and the like. Further, examples of the hetero ring include, for example, pyridine, quinoline, pyrrole, furan, thiophene, imidazole, indole and the like. Among them, R, R' and R¹ each independently represent preferably a hydrogen atom, or a cyclic or non-cyclic hydrocarbon group which may be unsubstituted or optionally substituted, or an alkyl silyl group, more preferably an unsubstituted hydrocarbon group or a hydrocarbon group substituted with a hydroxyl group, a halogen atom (for example, a fluorine atom or a chlorine atom), or an alkoxy group having 1 to 4 carbon atoms; an alkyl silyl group having 1 to 6 carbon atoms; or a hydrogen atom, and even more preferably an unsbstituted hydrocarbon group having 1 to 6 carbon atoms, an alkyl silyl group having 1 to 6 carbon atoms, or a hydrogen atom, and particularly preferably a hydrogen atom.

R² represents a hydrogen atom; or a halogen atom (for example, a fluorine atom, a chlorine atom, a bromine atom or an iodine atom), a cyano group, a nitro group, a sulfonyl group, an amide group, an substituted amide group having 1 to 20 carbon atoms, an alkoxy group having 1 to 20 carbon atoms (for example, methoxy, butoxy or dodecyloxy), an alkyl group having 1 to 20 carbon atoms (for example, methyl, butyl, hexadecyl) or the like, which is a substitutable group on the benzene ring, and preferably a hydrogen atom, a halogen atom, an amide group, a substituted or unsubstituted alkyl group or an alkoxy group, and more preferably an alkyl group having 1 to 8 carbon atoms, or an alkoxy group having 1 to 8 carbon atoms, and even more preferably a hydrogen atom, a chlorine atom, a fluorine atom and an alkoxy group having 1 to 4 carbon atoms, and particularly preferably a hydrogen atom.

A represents a (m+n) valent hydrocarbon group or heterocyclic group which may be unsbstituted or optionally substituted, and examples of the unsubstituted hydrocarbon group include the above hydrocarbon groups. Examples of the unsubstituted hetrocyclic group include groups of a heteroaromatic group (for example, furan, thiophene, pyridine, imidazole, pyrazole, triazole, oxazole, carbazole, indole, chromene, chroman, quinoline, dibenzofuran, phthalimide, thiophthalimide, benzoxazole, benzimidazole, benzothiazole or the like), and groups of heteroalicyclic compounds (for example, oxetane, thietane, oxolane, thiolane, pyrroline, pyrrolidine, pyrazoline, imidazoline, oxane, thiane, piperidine, pyrrolidone or the like).

As a substituent of the optionally substituted hydrocarbon group and the optionally substituted hetrocyclic group, a halogen atom (for example, a fluorine atom, a chlorine atom, a bromine atom and an iodine atom), a cyano group, a nitro group, an alkoxy group having 1 to 20 carbon group (for example, methoxy, butoxy, dodecyloxy), an aryl group such as phenyl, naphthyl or the like, a hydroxyl group, and the like may be exemplified. Among them, A is preferably an aryl group, an alicyclic group or an aliphatic polycyclic group which are unsbstituted or optionally substituted, more preferably a benzene ring group which is unsbstituted or substituted with a halogen atom (for example, a fluorine atom, or a chlorine atom), an alkoxy group having I to 4 carbon atoms or a hydrocarbon group having 1 to 6 carbon atoms, and particularly preferably an unsubstituted benzene ring group.

Q represents a hydrogen atom, a cyclic or non-cyclic hydrocarbon group, or a metal element which can form a monovalent metal salt. Examples of the cyclic or non-cyclic hydrocarbon group include an alkyl group having 1 to 20 carbon atoms (for example, methyl, butyl, octyl, hexadecyl), a phenyl group, a naphthyl group and the like. Examples of the metal element which can form a monovalent metal salt include lithium, sodium, potassium and the like. Q is preferably a hydrogen atom, sodium, potassium or an alkyl group having 1 to 20 carbon atoms (for example, methyl, butyl, octyl, hexadecyl, or the like), and more preferably a hydrogen atom, sodium or a methyl group.

a represents an integer from 0 or more, preferably from 0 to 4, and more preferably 0 or 1.
b represents an integer from 1 or more, preferably from 1 to 5, and more preferably 1.
m represents an integer from 1 or more, preferably from 1 to 4, and more preferably I or 2.
n represents an integer from 1 or more, preferably from 1 to 5, and more preferably 1 or 2.
When a, b, n and m each are 2 or more, plurally present R²(s), R¹(s) and Q(s), and the ethynylaryl group-containing moieties in the square bracket, may be the same with one another, or may be different from one another, respectively.

Explanation of Formula (2)

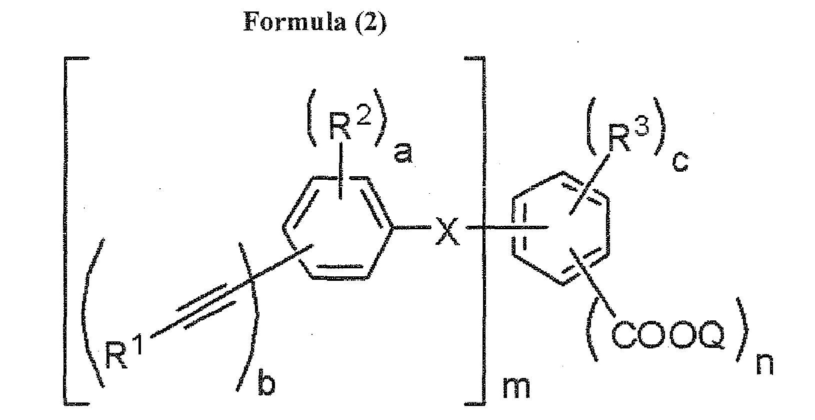

In Formula (2), X represents a divalent linking group represented by -NR(C=O)-, -NR(C=O)O-, -NR(C=O)NR'- or -(C=O)O-. R and R' each have the same definitions as those of R and R' in Formula (1). It is preferable that X be any one of an ester, amide, urethane and urea, or an imide ring or urea ring group formed by mutually connecting X with R²; or X be an imide ring group formed by mutually connecting X with R³. R³ represents a hydrogen atom, or a halogen atom (for example, a fluorine atom, a chlorine atom, a bromine atom, an iodine atom), a cyano group, a nitro group, an alkoxy group having 1 to 20 carbon atoms (for example, methoxy, butoxy, dodecyloxy), an alkyl group having 1 to 20 carbon atoms (for example, methyl, butyl, octyl, hexadecyl), and the like, which are substituents substitutable on the benzene ring. R³ represents preferably a hydrogen atom, a halogen atom, a nitro group, a sulfonyl group, an amide group, a substituted or unsbstituted hydrocarbon group or an alkoxy group, more preferably a hydrogen atom, a halogen atom, an alkyl group having 1 to 8 carbon atoms, or an alkoxy group having 1 to 8 carbon atoms, and even more preferably, a hydrogen atom, a chlorine atom, a fluorine atom, an alkyl group having 1 to 4 carbon atoms, or an alkoxy group having 1 to 4 carbon atoms, and particularly preferably a hydrogen atom. R¹, R², a, b and Q each have the same definitions as those of R¹, R², a, b and Q in Formula (1), and the preferable ranges thereof are also the same as those of R¹, R², a, b and Q in Formula (1).

c represents an integer from 0 to 3, and preferably 3. m represents an integer from 1 to 5, and preferably 1 or 2. n represents an integer from 1 to 5, and preferably 1 or 2. When a, b, c, n and m each are 2 or more, plurally present R²(s), R¹(s), R³(s) and Q(s), and an ethynylphenyl group-containing moieties in the square brackets, may be the same with one another, or may be different from one another, respectively. In addition, the sum of c, m and n is 6.

Explanation of Formula (3)

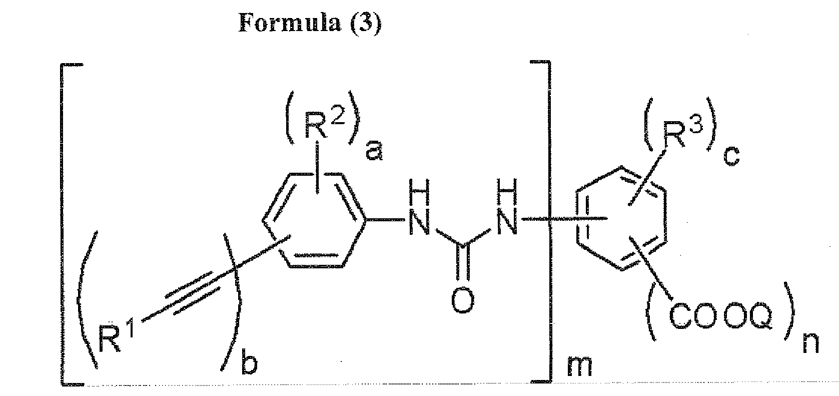

R¹, R², R³, Q, a, b, c, m and n each have the same definitions as those of R¹, R², R³, Q, a, b, c, m and n in Formula (2), and the preferable ranges thereof are also the same as those of R¹, R², R³, Q, a, b, c, m and n in Formula (2). When a, b, c, n and m each are 2 or more, plurally present R²(s), R¹(s), R³(s) and Q(s), and an ethynylphenyl group-containing moieties in the square brackets, may be the same with one another, or may be different from one another, respectively.

Explanation of Formula (4)

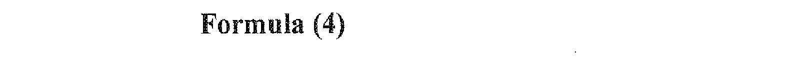

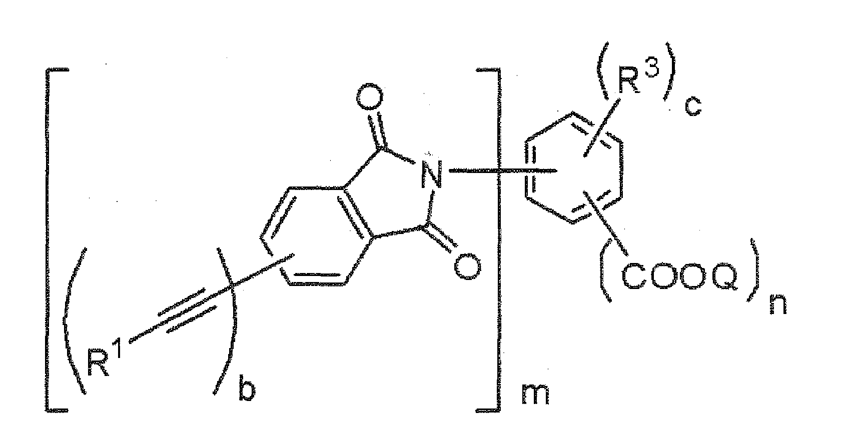

b represents an integer from I to 4, and R¹, R³, Q, c, m and n each have the same definitions as those of R¹, R³, Q, c, m and n in Formula (2), and the preferable ranges thereof are also the same as those of R¹, R³, Q, c, m and n in Formula (2). When b, c, n and m each are 2 or more, plurally present R¹(s), R³(s) and Q(s), and an ethynylphthalimide group-containing moieties in the square brackets may be the same with one another, or may be different from one another, respectively.

Explanation of Formula (5)

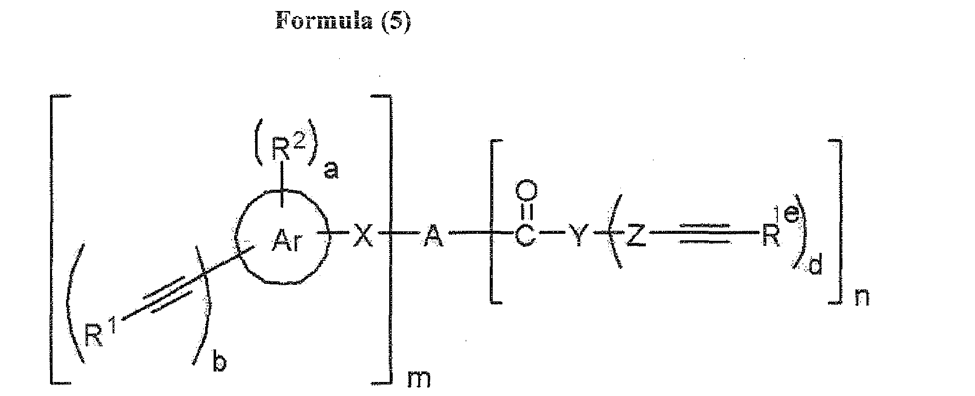

The compound of Formula (5) is a compound formed by allowing the compound represented by Formula (1) to react with a compound which has a functional group capable of reacting with a carboxyl group and has an ethynyl group substituted with one or more R^{1e}. R¹, R², the encircled Ar, X, A, a, b, m and n each have the same definitions as those of R¹, R², the encircled Ar, X, A, a, b, m and n in Formula (1), and the preferable ranges thereof are also the same as those of R¹, R², the encircled Ar, X, A, a, b, m and n in Formula (1). d has the same definition as that of b in Formula (1), Z has the same definition as that of A in Formula (1), and R^{1e} has the same definition as that of R¹ in Formula (1), respectively, and the preferable ranges thereof are also the same as those of R¹ in Formula (1). Y represents -NR- or -O-. R has the same definition as that of R in Formula (1), and the preferable ranges thereof are also the same as those of R in Formula (1).

As the a compound which has a functional group capable of reacting with a carboxyl group, and has an ethynyl group substituted with one or more R^{1e}, compounds which have an ethynyl group substituted with one or more R^{1e}, and have an amino group, a hydroxy group or a mercapto group may be exemplified. Further, anilines, phenols, mercaptobenzenes, naphthols, aminopyridines, naphthyl amines, amino furyls, hydroxyfuryls or the like, which has an ethynyl group substituted with one or more R^{1e}, may be exemplified. Among them, anilines, phenols and mercaptobenzenes having an ethynyl group substituted with one or more R^{1e} are preferable.

As specific compounds of the compound which has a functional group capable of reacting with a carboxyl group, and has an ethynyl group substituted with one or more R^{1e}, for example, anilines (such as m-ethynylaniline, p-ethynylaniline, o-ethynylaniline, 5-ethynyl-2-methylaniline, 3-ethynyl-4-methylaniline, 5-ethynyl-3-fluoroaniline, 3-etlaynyl-4-fluoroaniline, 3-ethynyl-4-methoxyaniline, 3-ethynyl-4-ethoxyaniline, 2,6-dimethyl-4-ethynylaniline, 2,3-diethynylaniline, 3,4-diethynylaniline, 3,5-dietlaynylaniline, 3,6-diethynylaniline, 2,4,6-triethynylaniline, m-propynylaniline, m-butynylaniline, m-hexynylaniline, m-dodeeylethynylaniline, m-t-butylethynylaniline, m-cyclohexylethynylaniline, m-3-pyridylethynylanilne, m-2-pyridylethynylaniline, m-naphthylethynylaniline, m-quinolinylethynylaniline, m-(3-hydroxy-3-methyl-1-buthyryl)aniline, 3-(3-hydroxy-3-methyl-1-butynyl)-5-methylaniline, m-trimethylsilylethynylaniline, m-ethynyltoluidine, p-ethynyltoluidine, o-ethynyl-p-chloroaniline, 2,3-diethynyl-5-methylaniline, 3,4-diethynyltoluidine, 3,5-diethynyltoluidine, 4-chloro-3,6-diethynylaniline, m-propynyltoluidine, m-butynyltoluidine, m-hexynyltoluidine, 3-dodecylethynyl-5-inethoxyaniline, 3-t-butylethynyl-5-chloroaniline, 3-cyclohexylethynyl-5-chloroaniline, m-(2-hydroxypropyl-2-ethynyl)toluidine, and m-trimethylsilylethynyltoluidine);

phenols (such as m-ethynylphenol, p-ethynylphenol, o-ethynylphenol, 5-ethynyl-2-methylphenol, 3-ethynyl-5-fluorophenol, 2,3-diethynylphenol, 3,4-diethynylphenol, 3,5-diethynylphenol, 3,6-diethynylphenol, 2,4,6-triethynylphenol, m-propynylphenol, m-butynylphenol, m-hexynylphenol, m-dodecylethynylphenol, m-t-butylethynylphenol, m-cyclohexylethynylphenol, m-3-pyridylethynylphenol, m-2-pyridylethynylphenol, m-naphthylethynylphenol, m-quinolinylethynylphenol, m-(2-hydroxypropyl-2-ethynyl)phenol, m-trimethylsilylethynylphenol, m-ethynylcresol, p-ethynylcresol, o-ethynyl-p-chlorophenol, 3-ethynyl-4-methylphenol, 3-ethynyl-4-methoxyphenol, 3-ethynyl-4-ethoxyphenol, 3-ethynyl-4-fluorophenol, 4-ethynyl-2,6-dimethylphenol, 2,3-diethynyl-5-methylphenol, 3,4-diethynylphenol, 3,5-diethynylphenol, 4-chloro-3,6-diethynylphenol, m-propynylcresol, m-butynylcresol, m-hexynylcresol, 3-dodecylethynyl-5-methoxyphenol, 3-t-butylethynyl-5-chlorophenol, 3-cyclohexylethynyl-5-chlorophenol, m-(2-hydroxypropyl-2-ethynyl)cresol, m-trimethylsilylethynylcresol, and m-(3-hydroxy-3-methyl-1-butynyl)phenol).

mercaptobenzenes (for example, m-ethynylmercaptobenzene, p-ethynylmereaptobenzene, o-ethynylmercaptobenzene, 5-ethynyl-2-methyl mercaptobenzene, 3-ethynyl-5-fluoro mercaptobenzene, 2,3-diethynyl mercaptobenzene, 3,4-diethynyl mercaptobenzene, 3,5-diethynyl mercaptobenzene, 3,6-diethynyl mercaptobenzene, 2,4,6-triethynyl mercaptobenzene, m-propynyl mercaptobenzene, m-butynyl mercaptobenzene, m-hexynyl mercaptobenzene, m-dodecylethynyl mercaptobenzene, m-t-butylethynyl mercaptobenzene, m-cyclohexylethynyl mercaptobenzene, m-3-pyridylethynyl mercaptobenzene, m-2-pyridylethynyl mercaptobenzene, m-naphthylethynyl mercaptobenzene, m-qumolinylethynyl mercaptobenzene, m-(2-hydroxypropyl-2-ethynyl) mercaptobenzene, m-trimethylsilylethynyl mercaptobenzene, m-ethynyl-p-methyl mercaptobenzene, o-ethynyl-p-chloro mercaptobenzene, 3-ethynyl-4-methoxy mercaptobenzene, 3-ethynyl-4-fluoro mercaptobenzene, 4-ethynyl-2,6-dimethyl mercaptobenzene, 2,3-diethynyl-5-methyl mercaptobenzene, 3,4-diethynyl mercaptobenzene, 3,5-diethynyl mercaptobenzene, 4-chloro-3,6-diethynyl mercaptobenzene, m-propynyl-p-methyl mercaptobenzene, 3-t-butylethynyl-5-chloro mercaptobenzene, 3-cyclahexylethynyl-5-chloro mercaptobenzene, m-(2-hydroxypropyl-2-ethynyl) mercaptobenzene, or the like may be exemplified.

Among them, from the viewpoints of the availability of raw materials, and the reactivity, m-ethynylaniline, p-ethynylaniline, o-ethynylaniline, 2,3-diethynylaniline, 3,4-diethynylaniline, 3,5-diethynylaniline, 3,6-diethynylaniline, m-propynylaniline, m-hexynylaniline, m-t-butytethynylaniline, m-cyclohexylethynylaniline, m-3-pyridylethynylaniline, m-trimethylsilylethyhylaniline, m-ethynyltoluidine, m-(3-hydroxy-3-methyl -1-butynyl)aniline, m-ethynylphenol, p-ethynylphenol, o-ethynylphenol, 2,3-diethynylphenol, 3,4-diethynylphenol, 3,5-diethynylphenol, 3,6-diethynylphenol, m-propynylphenol, m-hexynylphenol, m-t-butylethynylphenol, m-cyclohexylethynylphenol, m-3-pyridylethynylphenol, m-trimethylsilylethynylphenol, m-ethynylcresol, m-(3-hydroxy-3-methyl-1-butynyl)phenol, and the like are desirable, and in particular, m-ethyhylaniline, p-ethyhylaniline, 3,4-diethynylaniline, 3,5-diethynylaniline, m-propynylaniline, m-cyclohexylethyhylaniline, m-(3-hydroxy-3-mcthyl-1-butynyl)aniline, m-ethynylphenol, p-ethynylphenol, 3,4-diethynylphenol, 3,5-diethynylphenol, m-propynylphenol, m-cyclohexylethyhylphenol, and m-(3-hydmxy-3-methyl-1-butynyl)phenol are desirable.

Specific examples of the acetylene compound of the invention are shown below, but the present invention is not limited to these examples.

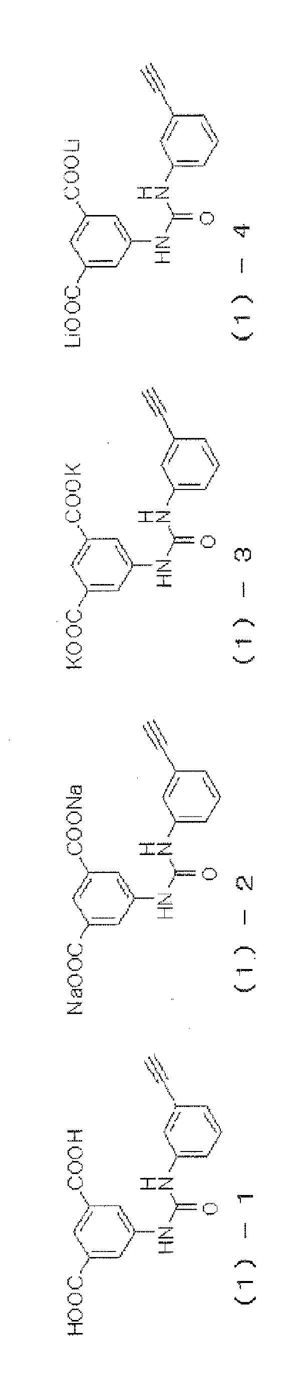

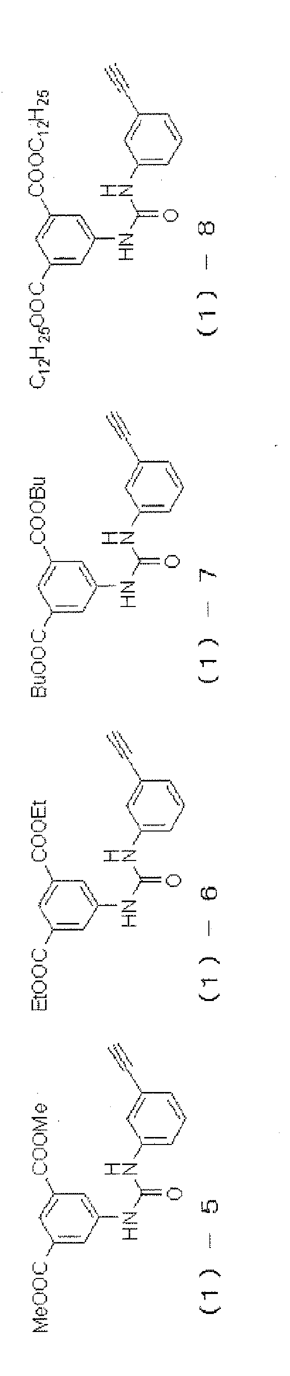

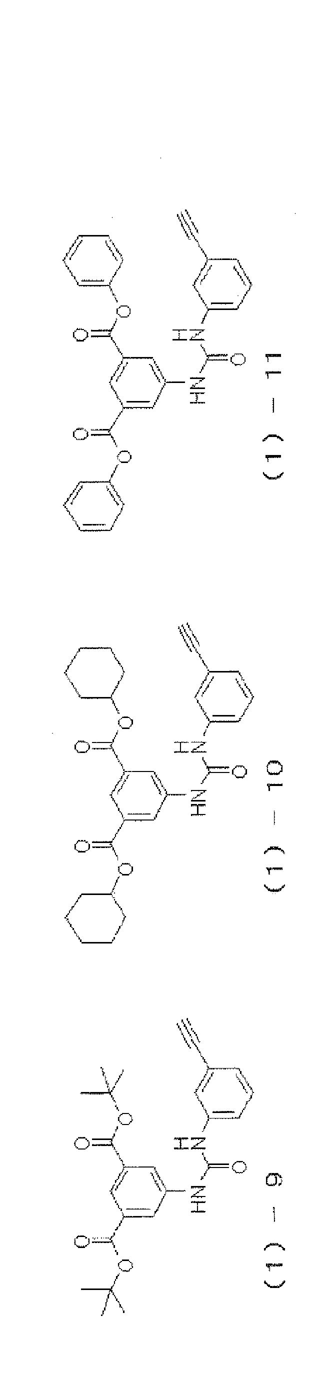

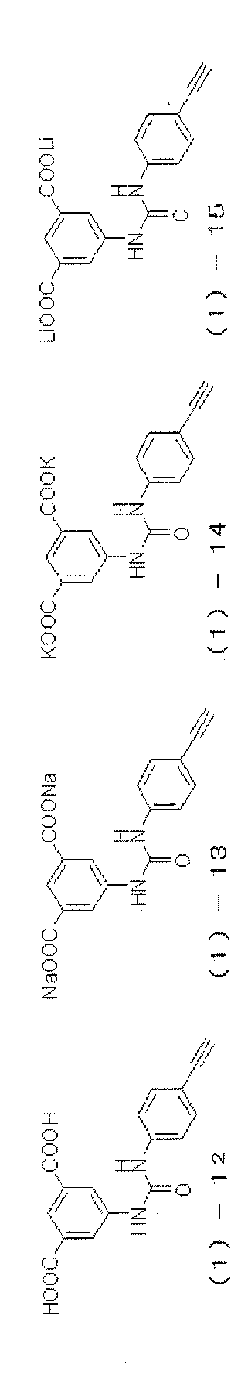

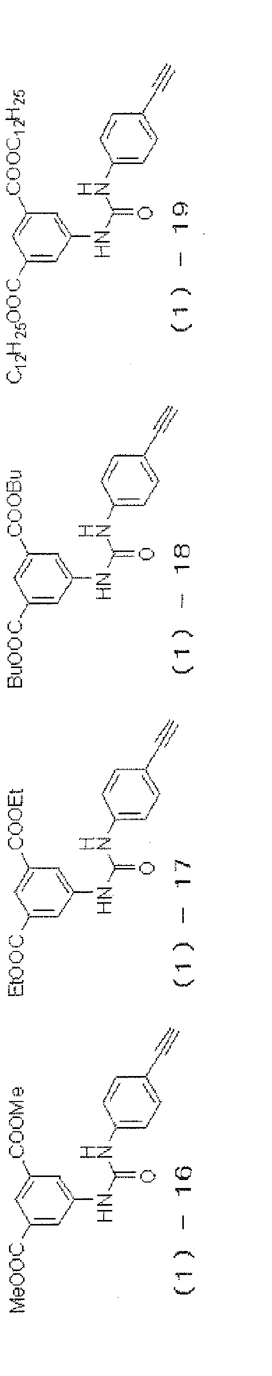

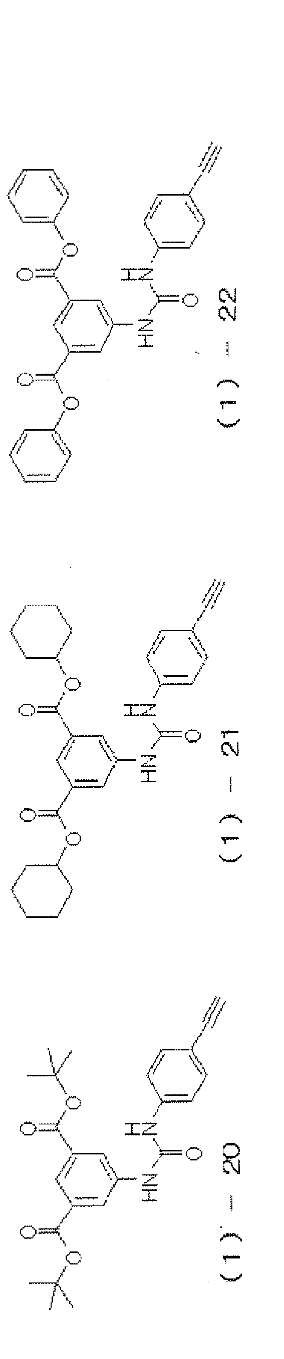

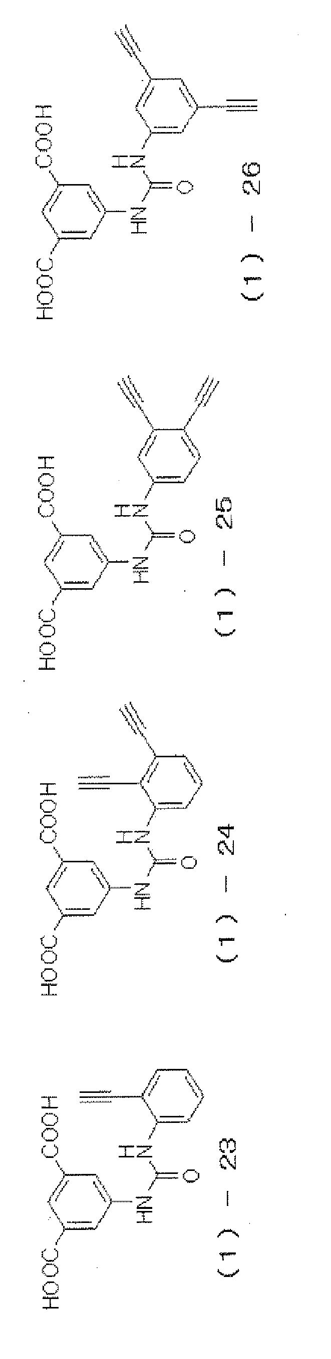

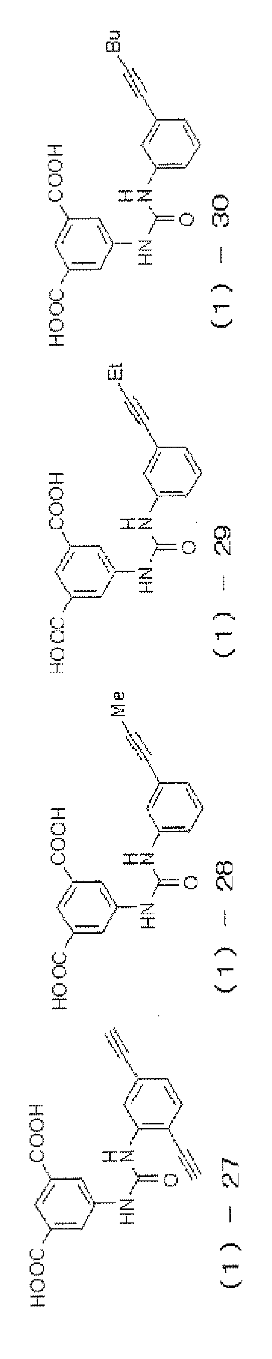

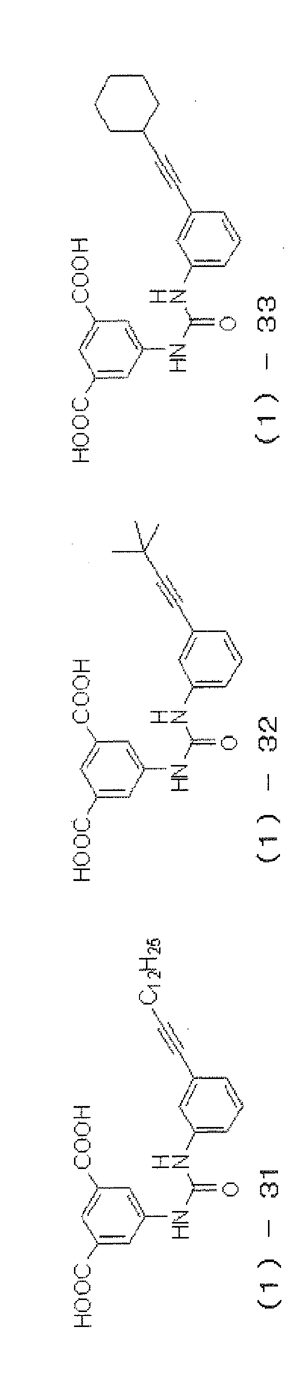

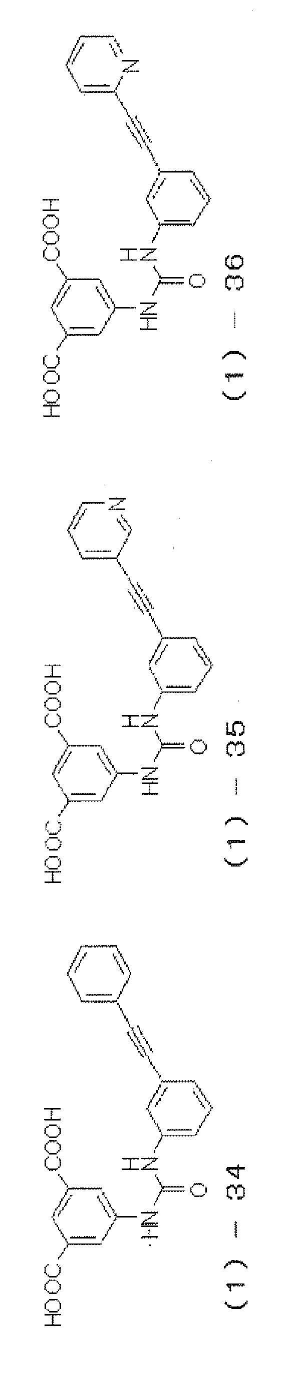

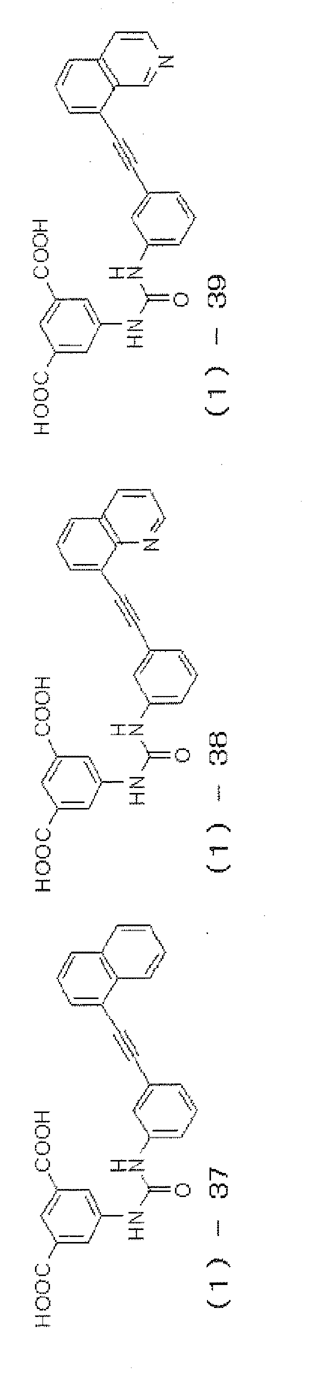

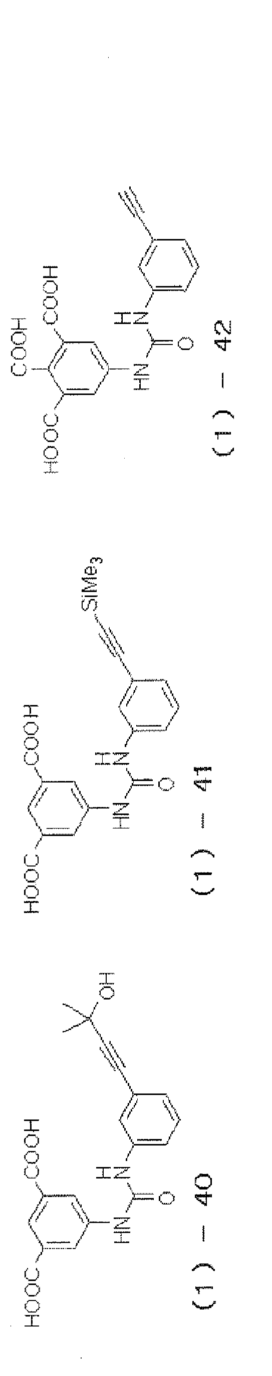

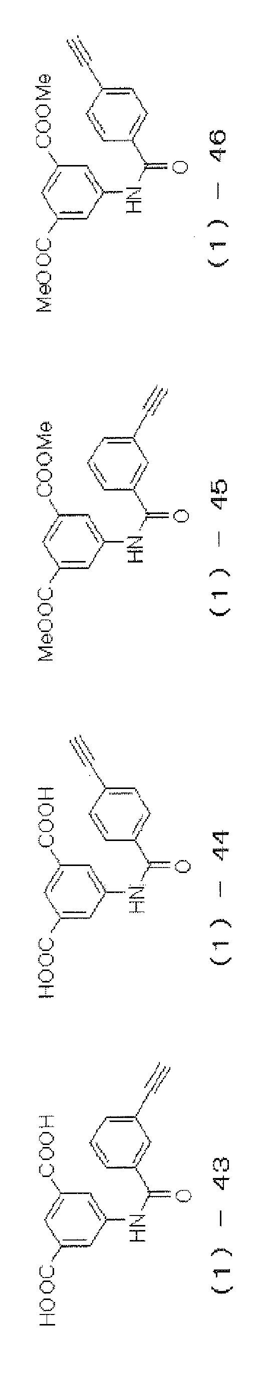

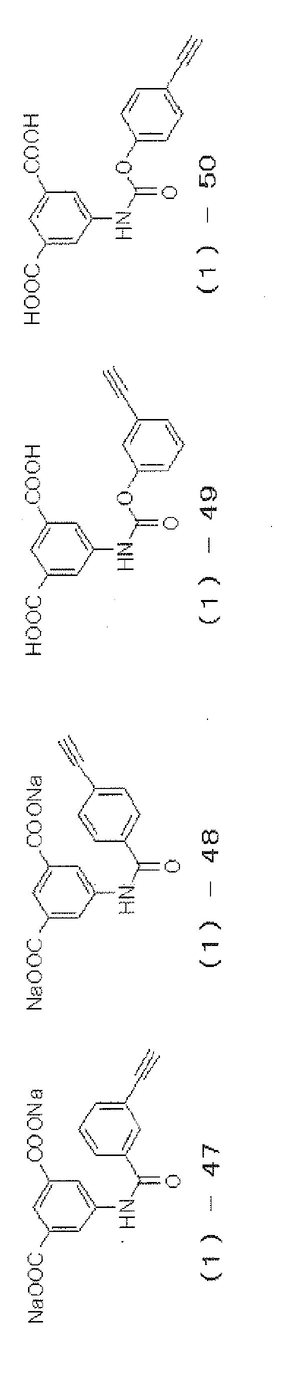

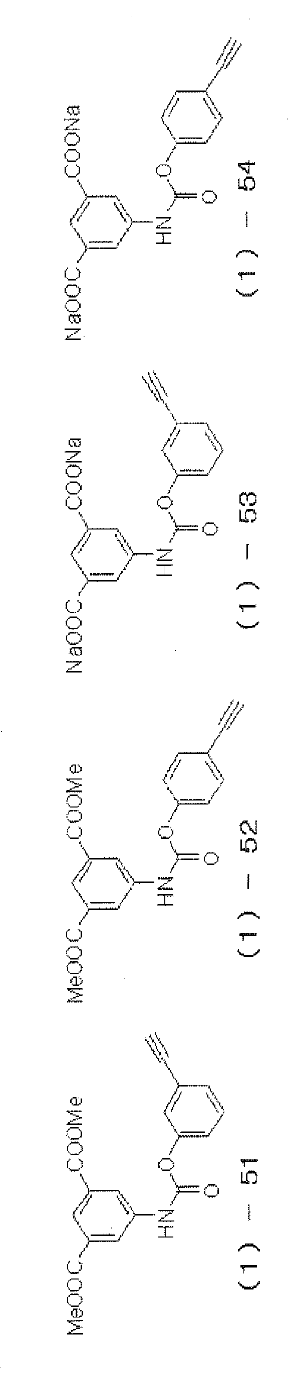

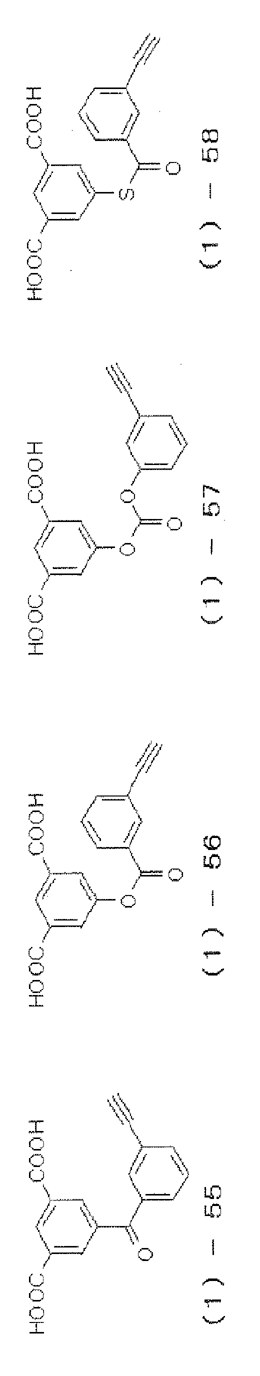

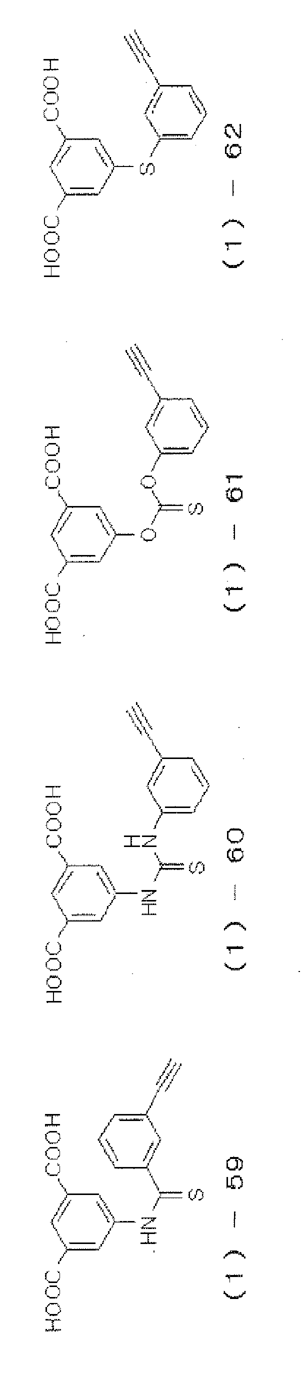

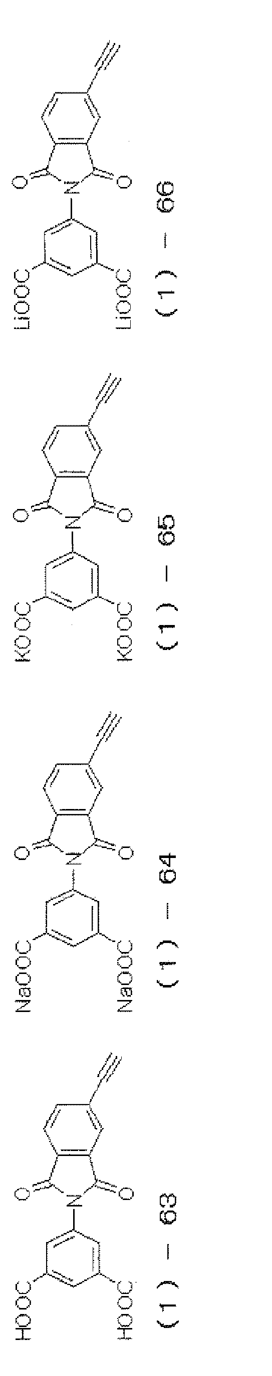

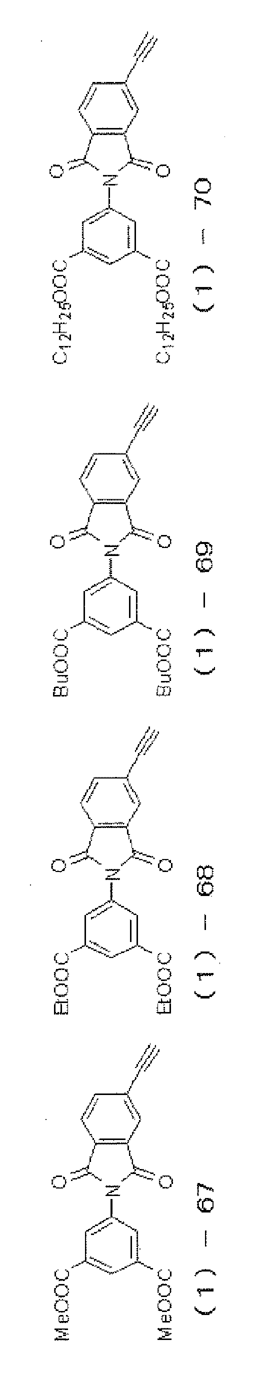

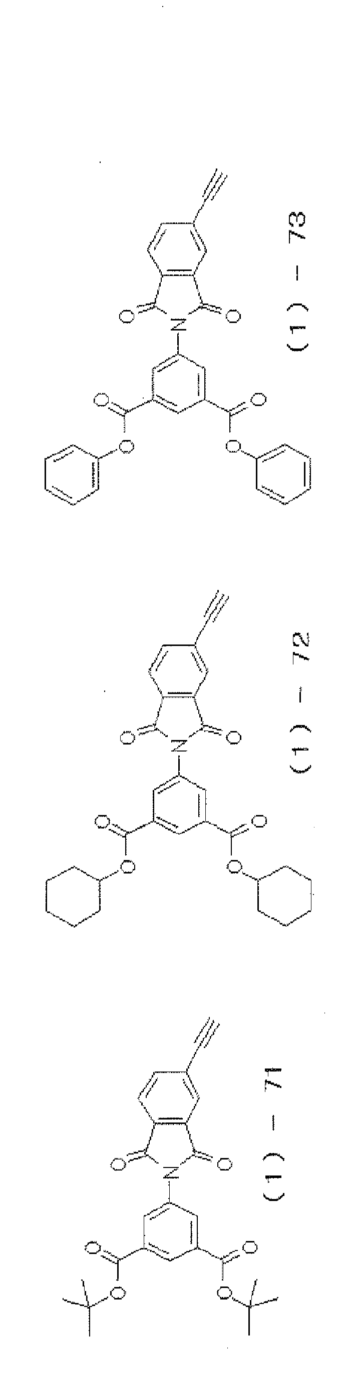

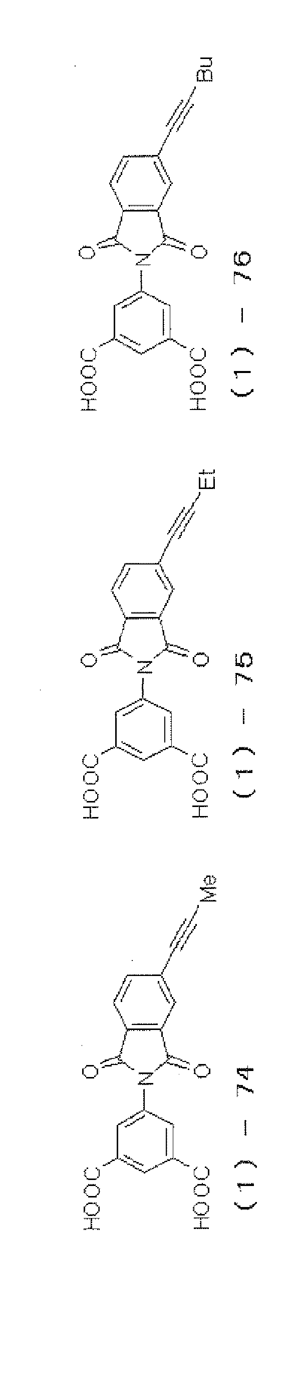

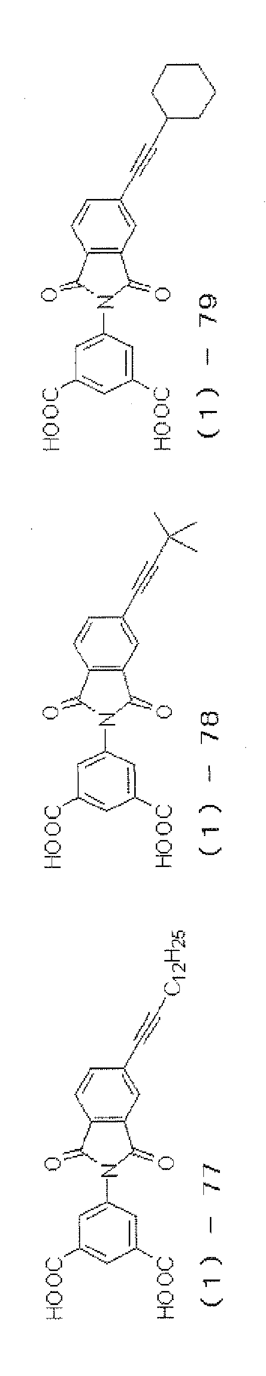

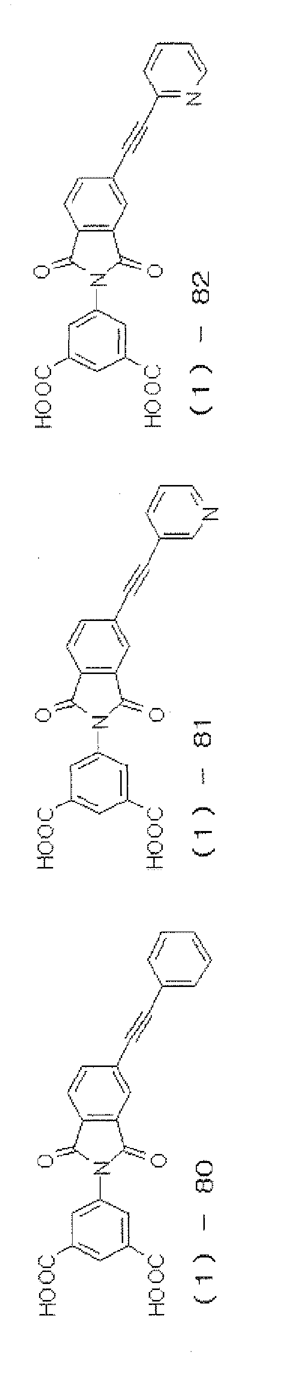

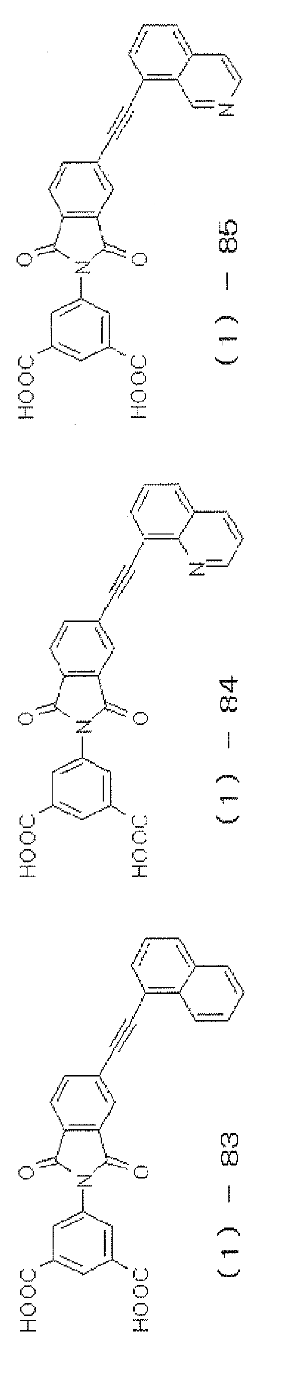

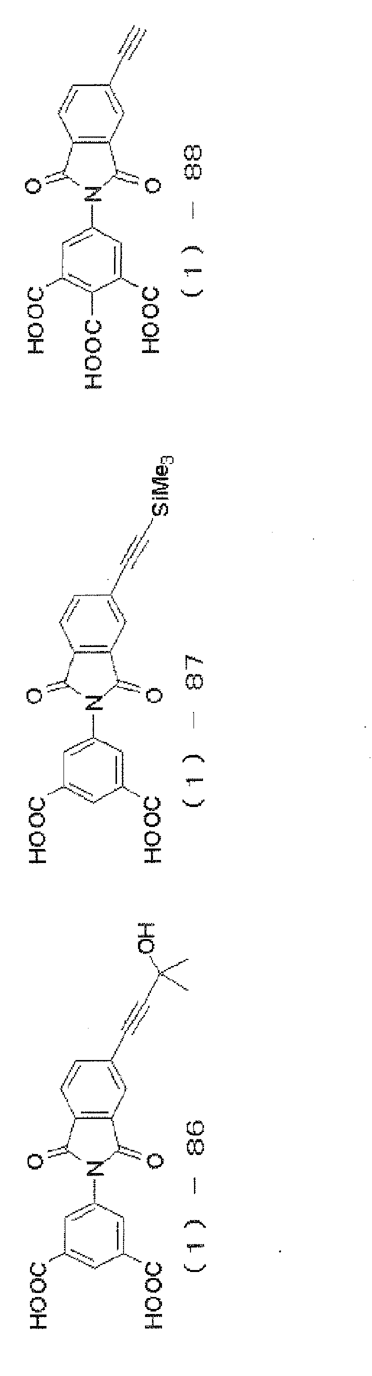

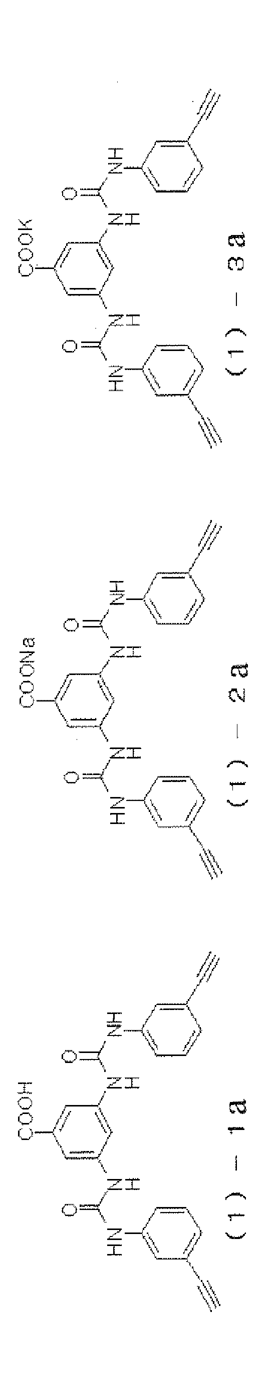

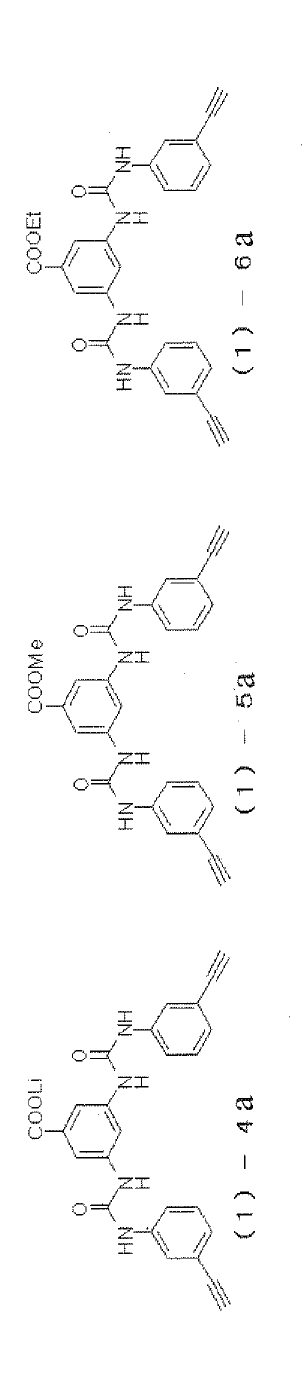

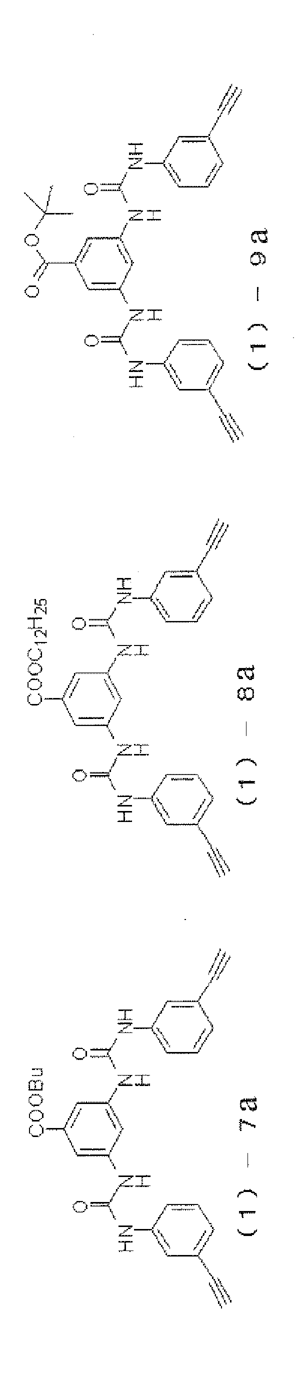

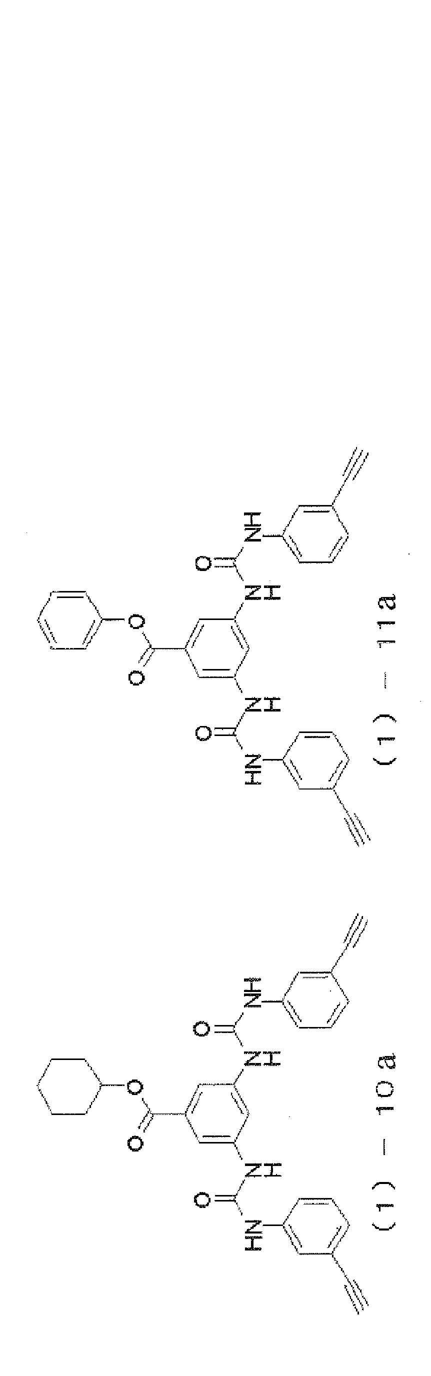

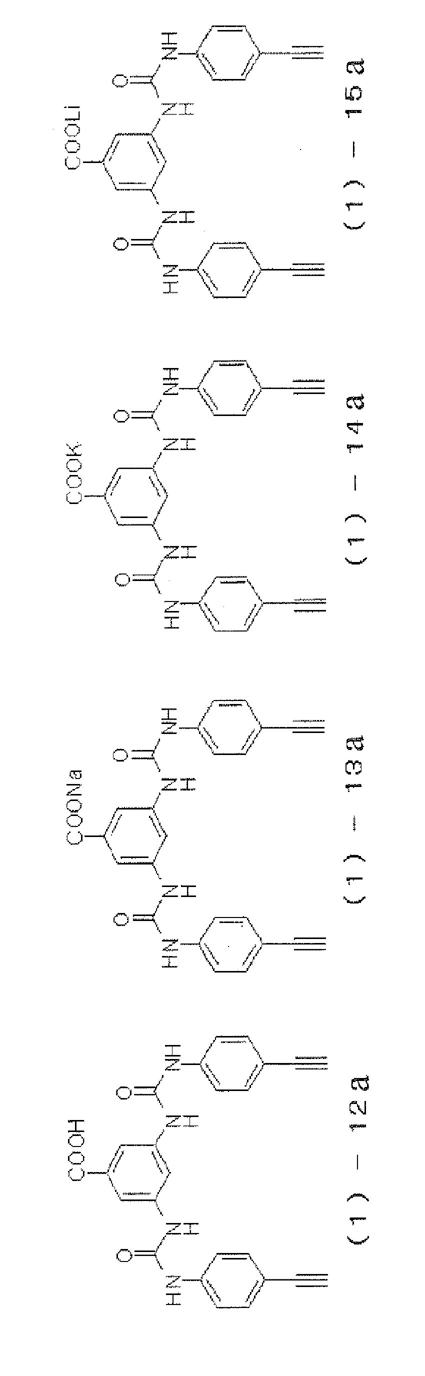

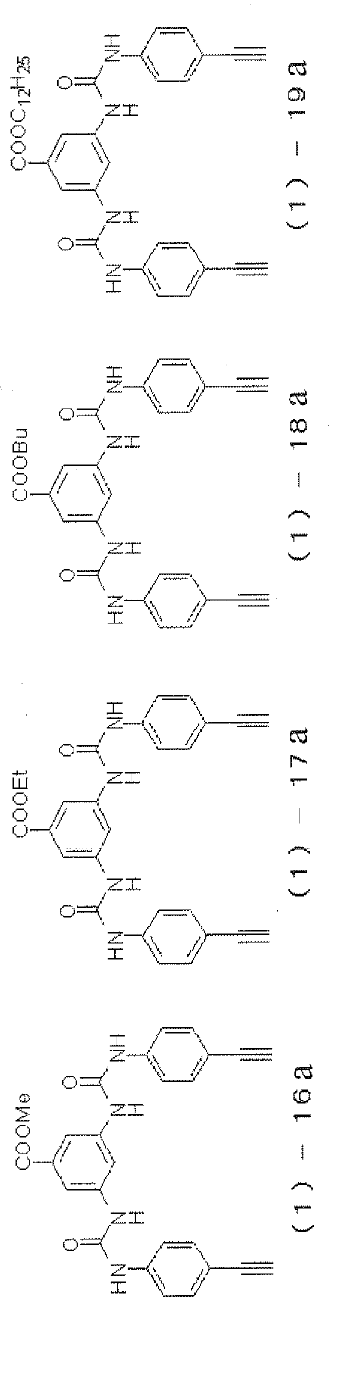

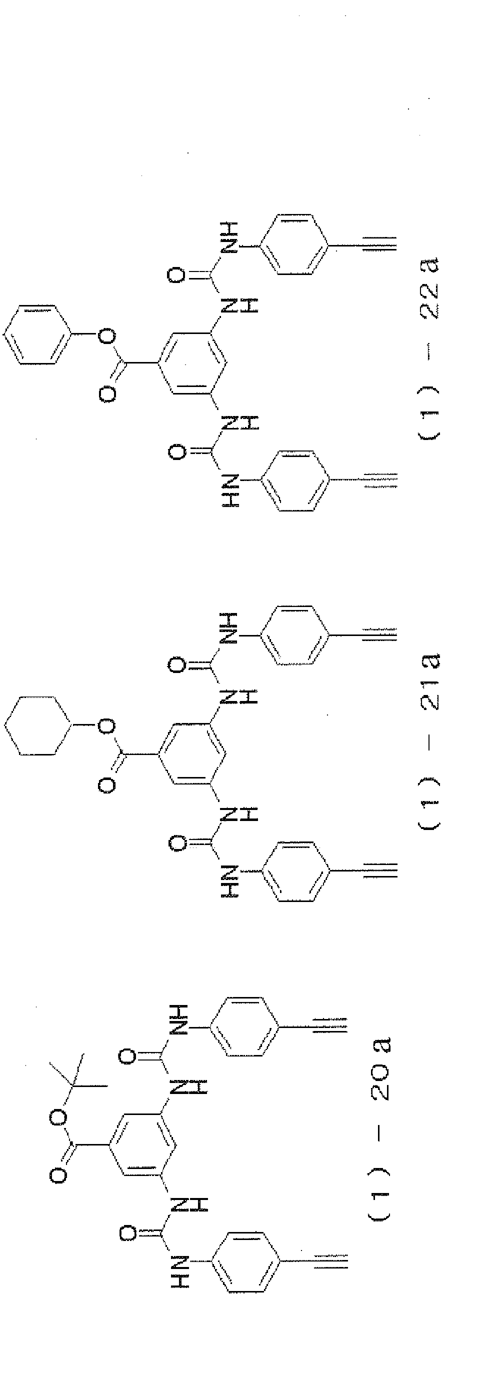

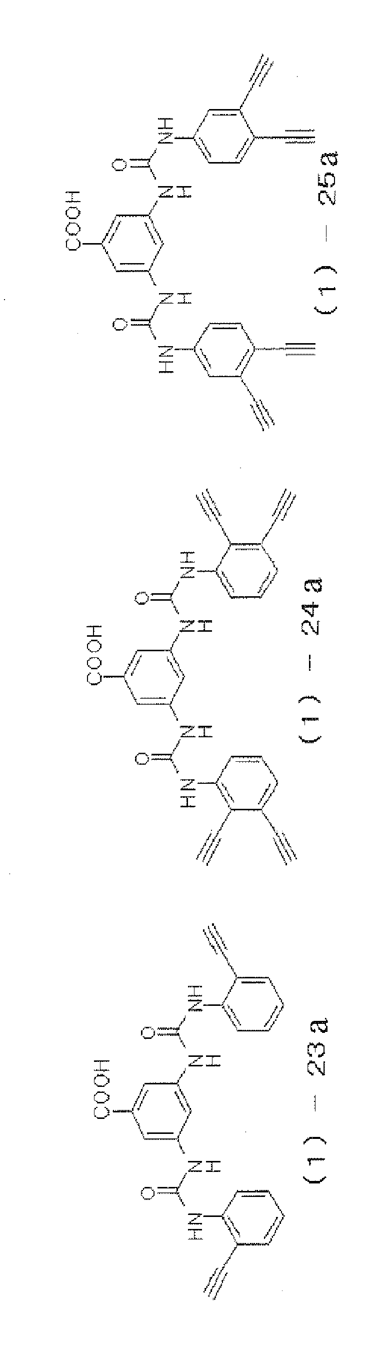

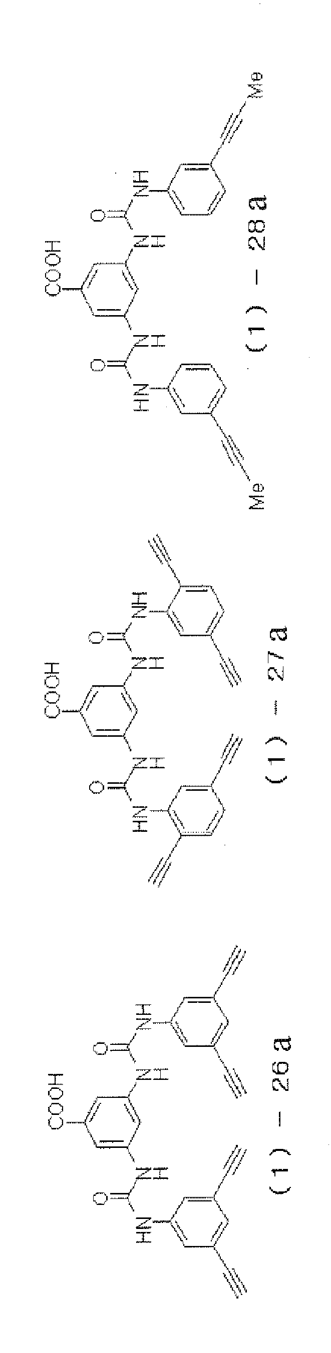

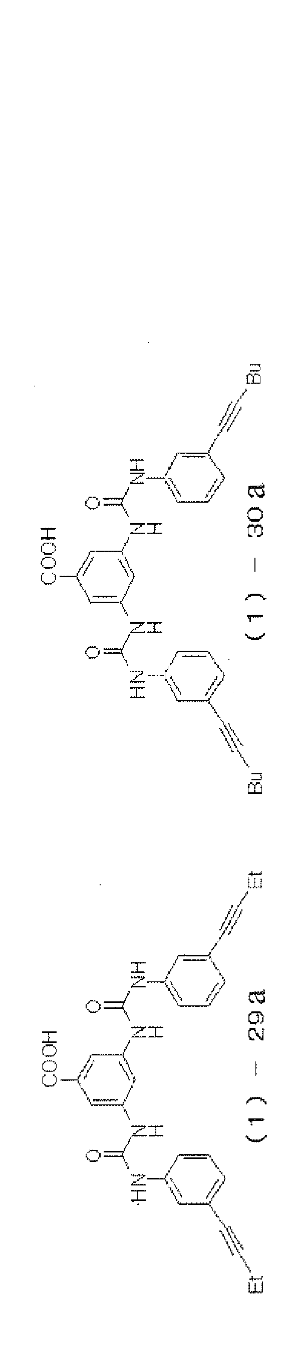

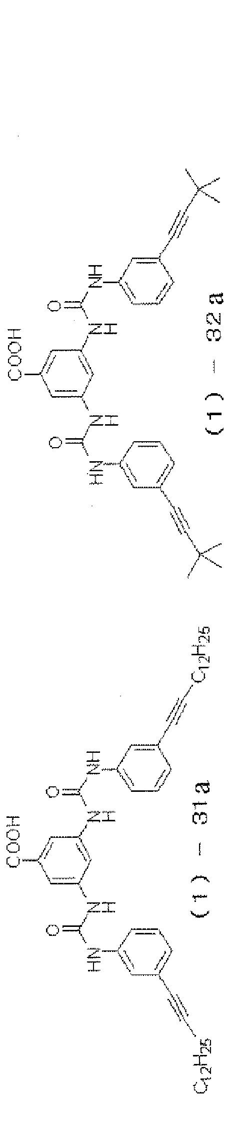

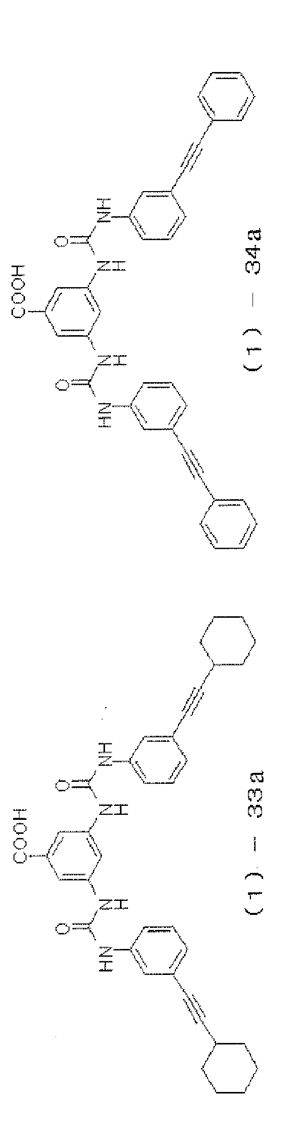

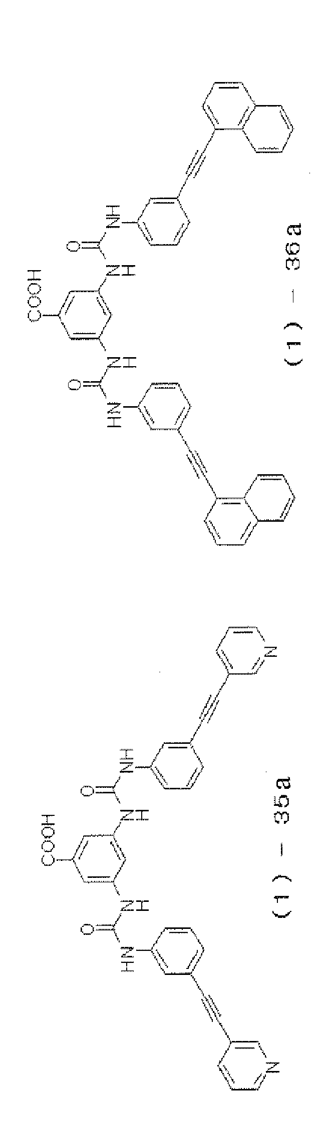

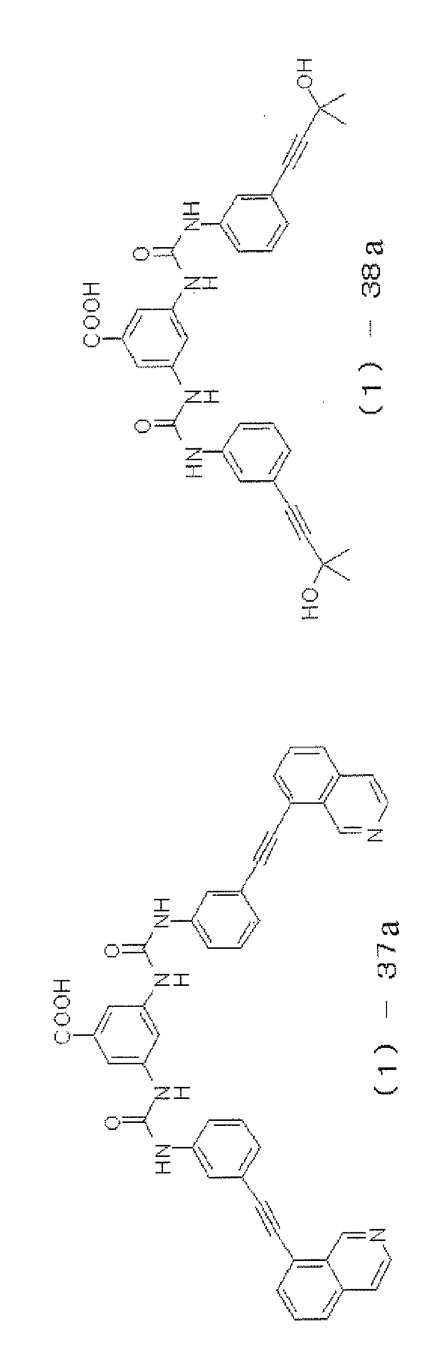

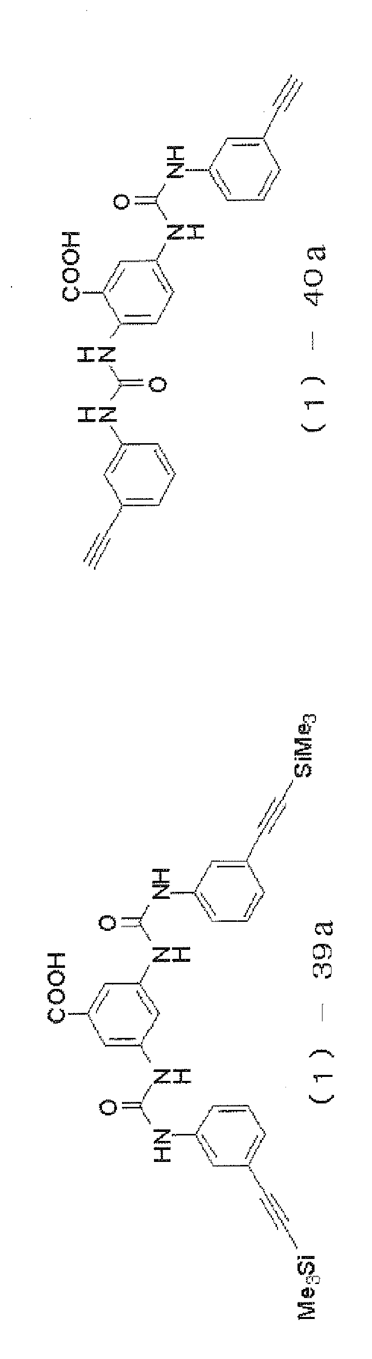

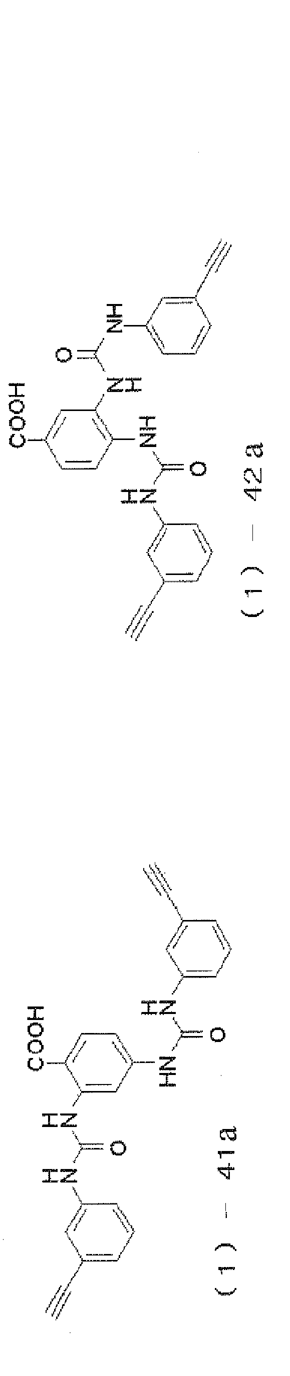

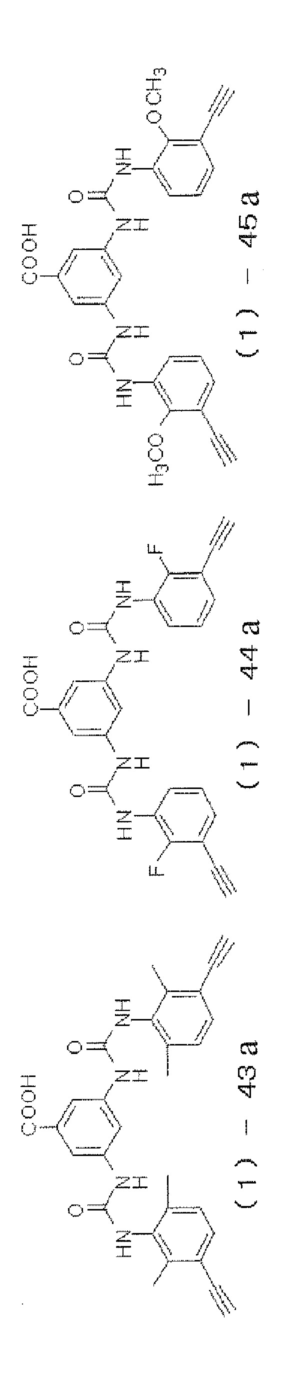

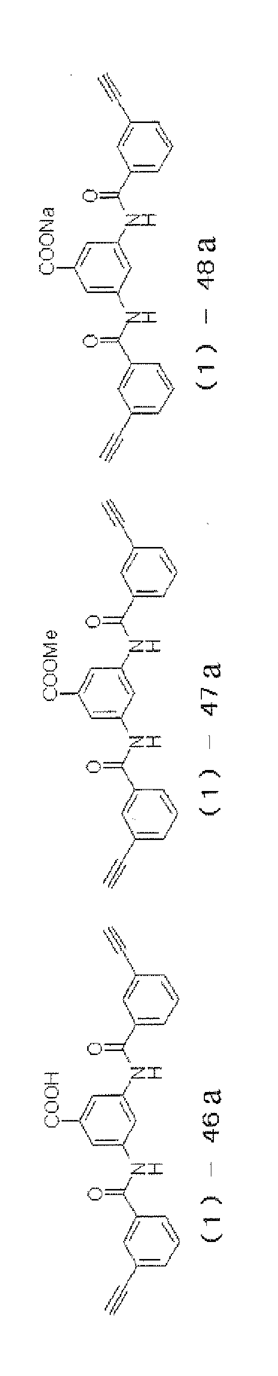

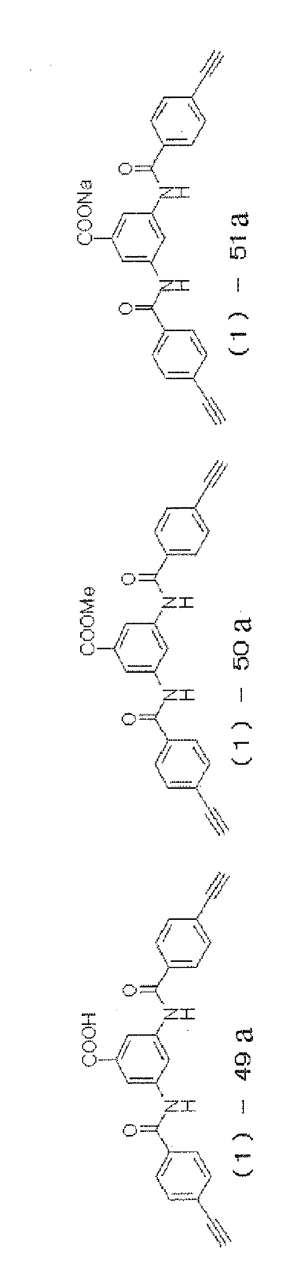

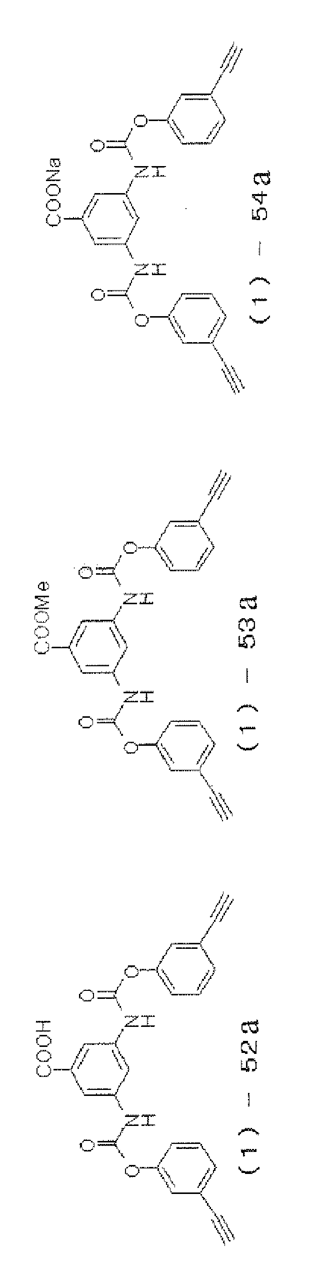

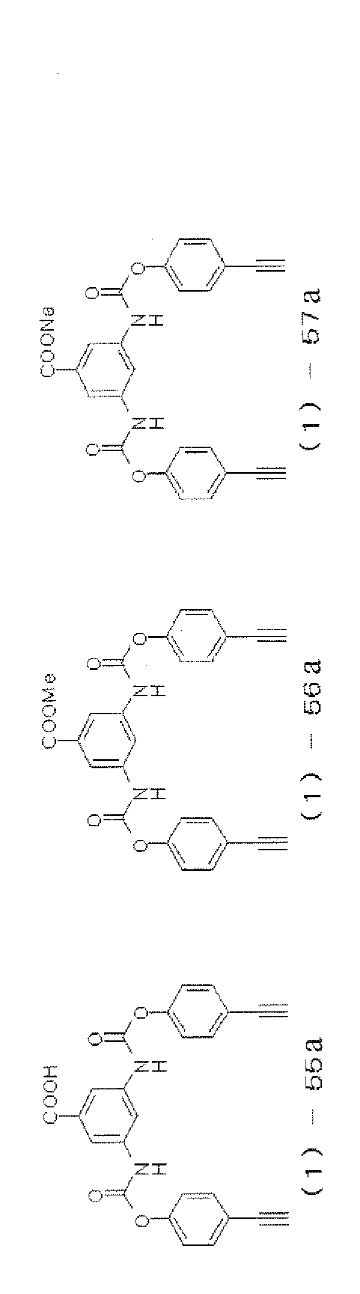

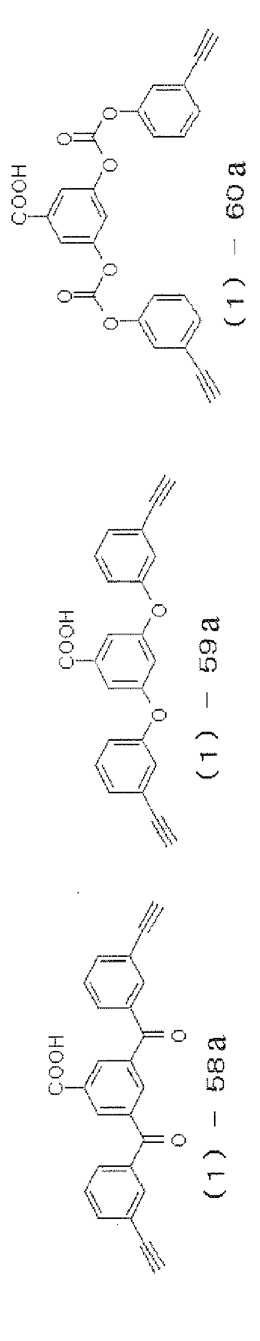

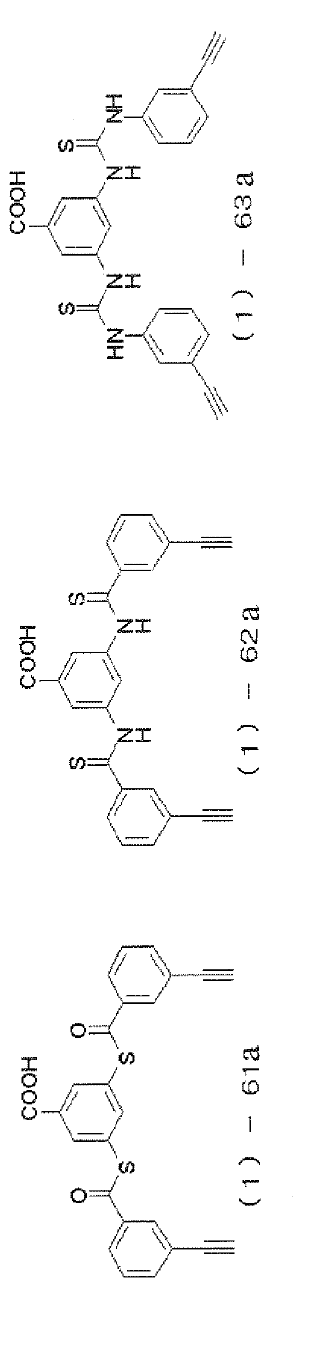

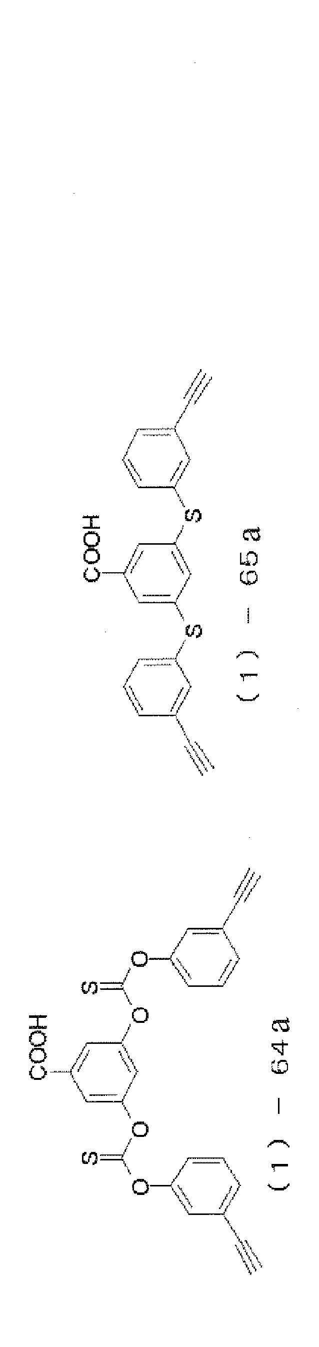

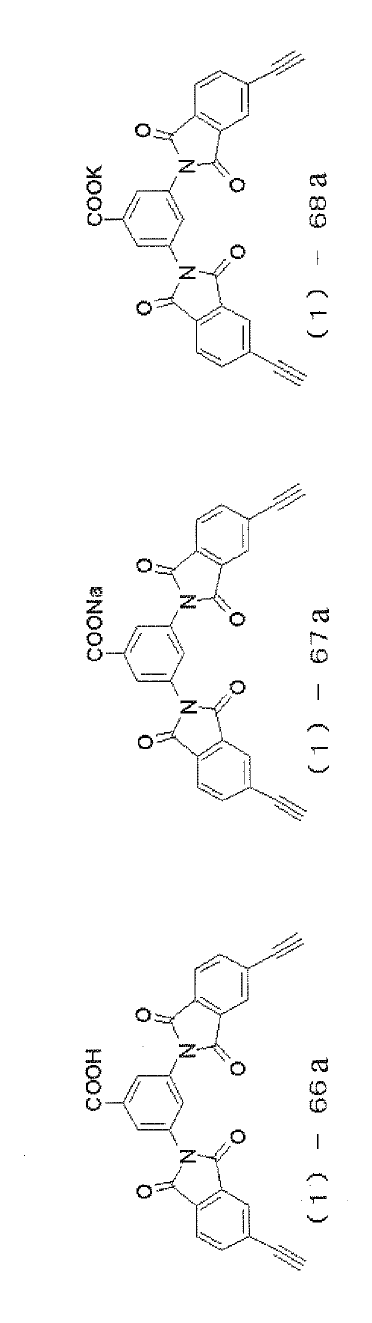

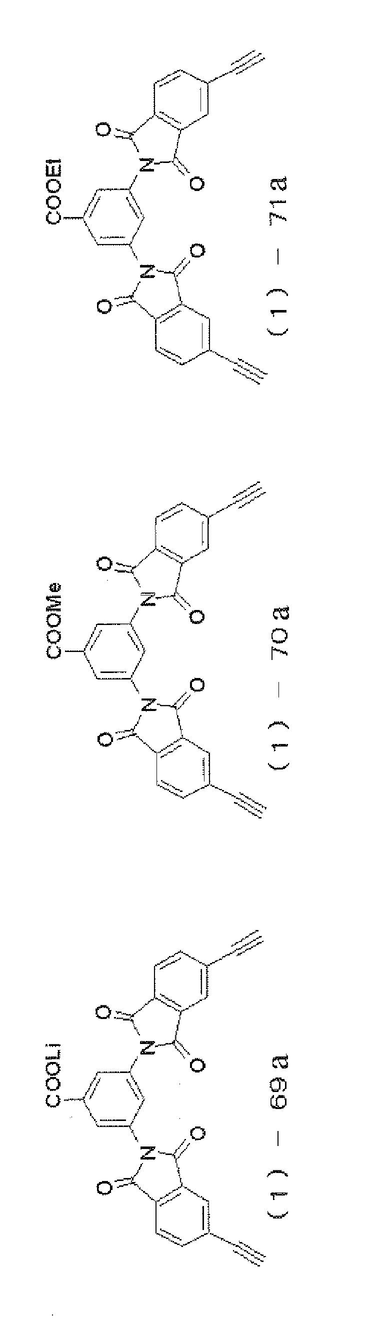

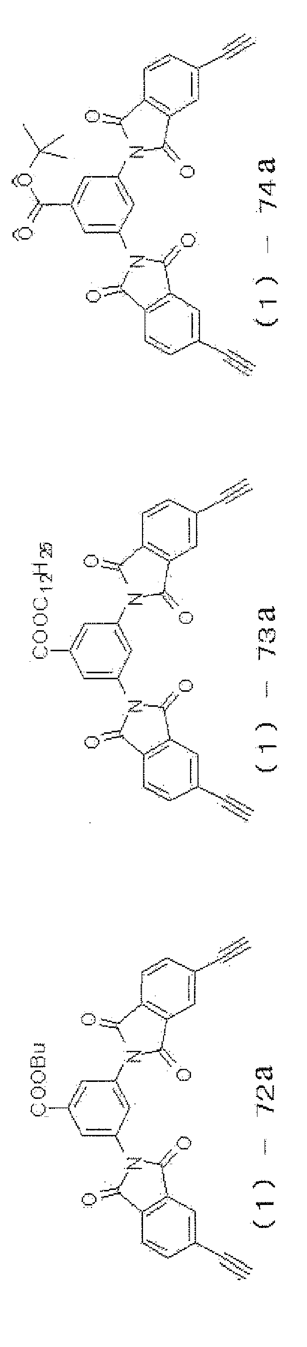

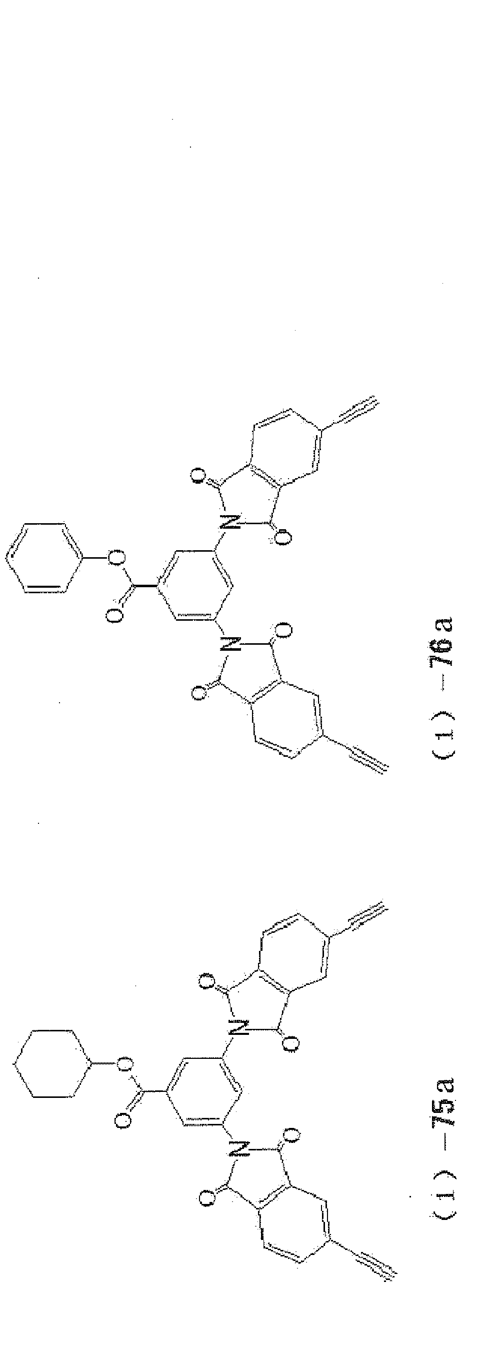

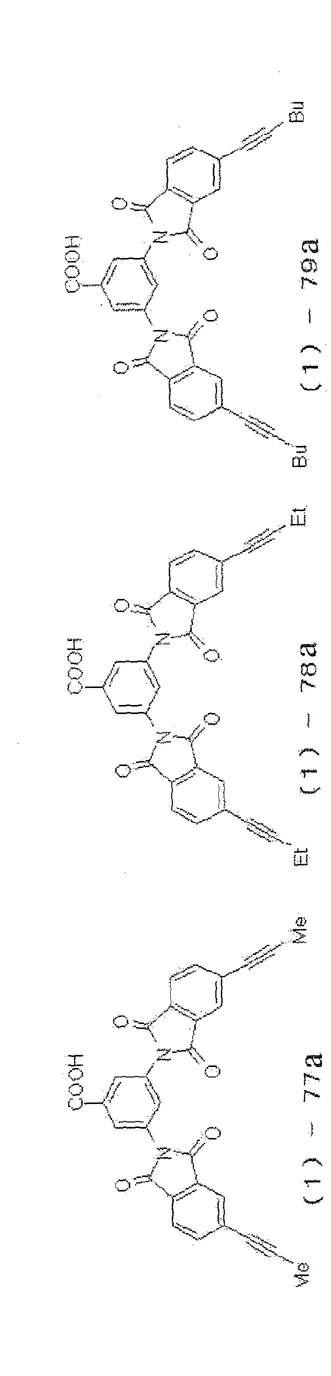

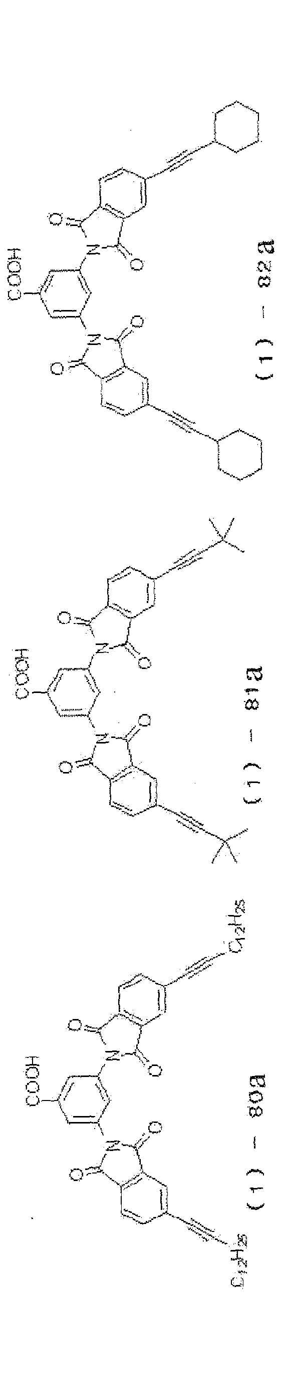

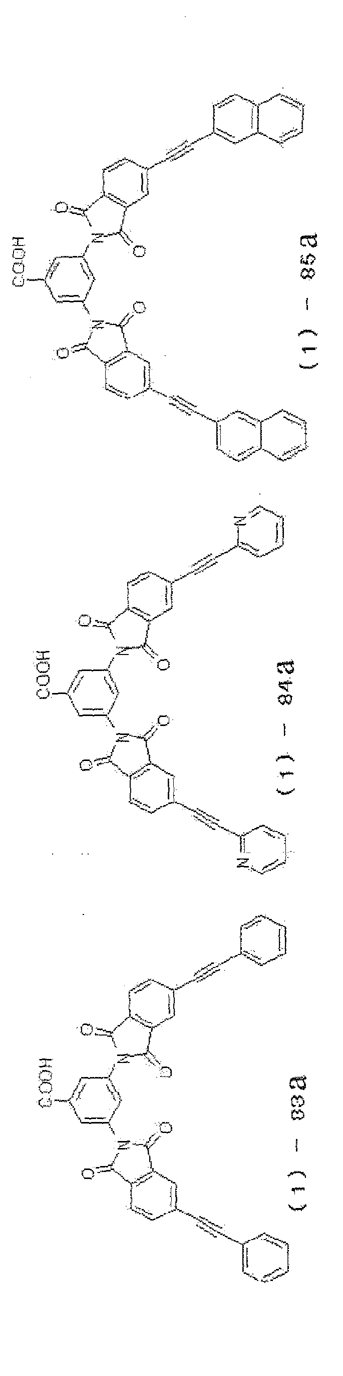

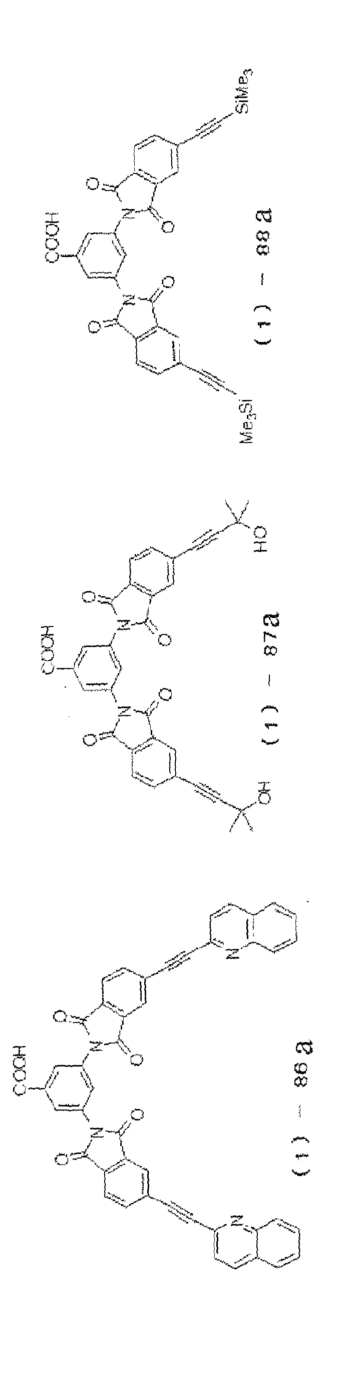

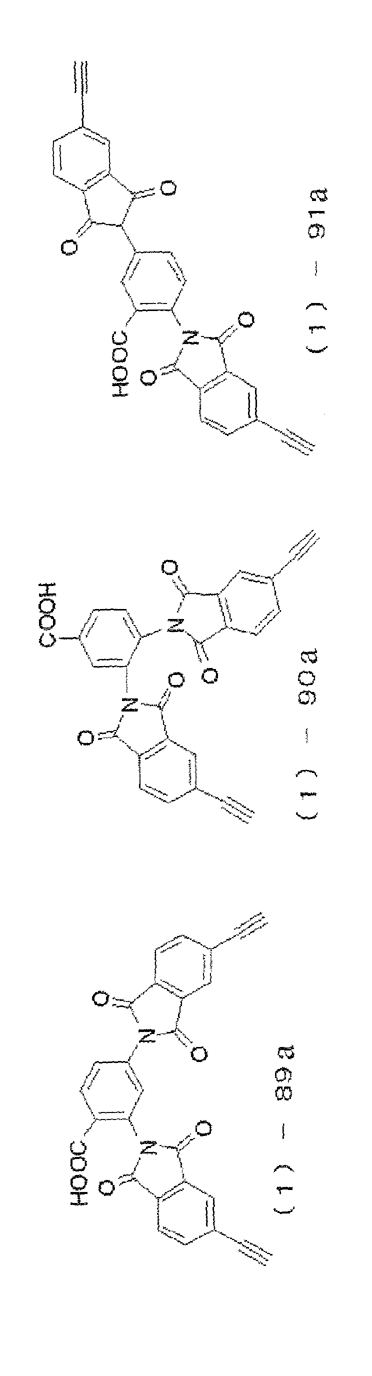

As specific exemplary compounds represented by Formula (5) of the invention, specific examples of compounds formed by condensing the compounds represented by Formula (1) with compounds which have one or more substituted or unsbstituted ethynyl groups, and have any of the structures of -NH₂, -OH and -SH in a molecule are shown below, but the invention is not limited to these examples.

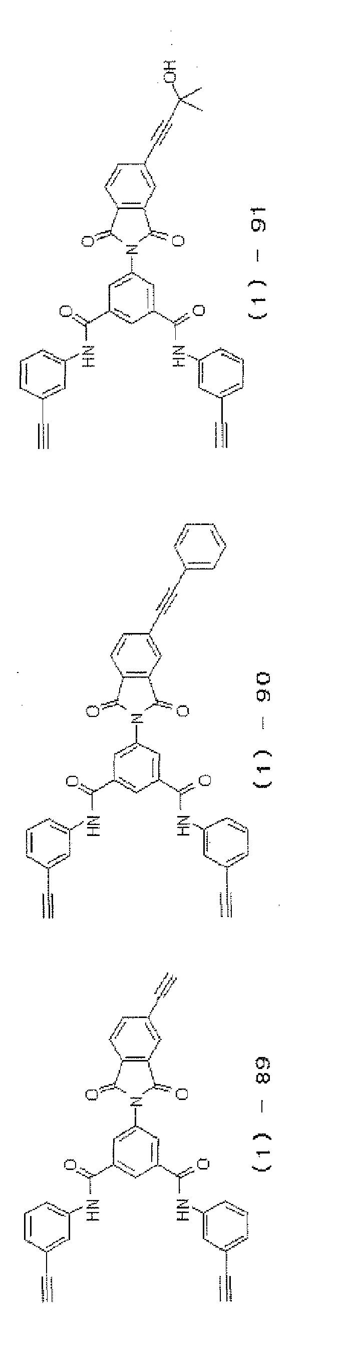

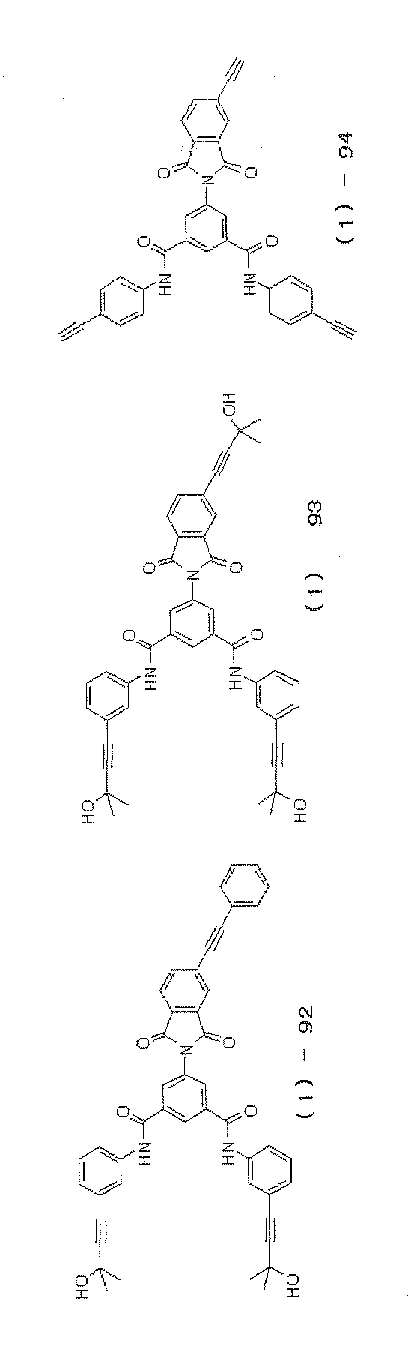

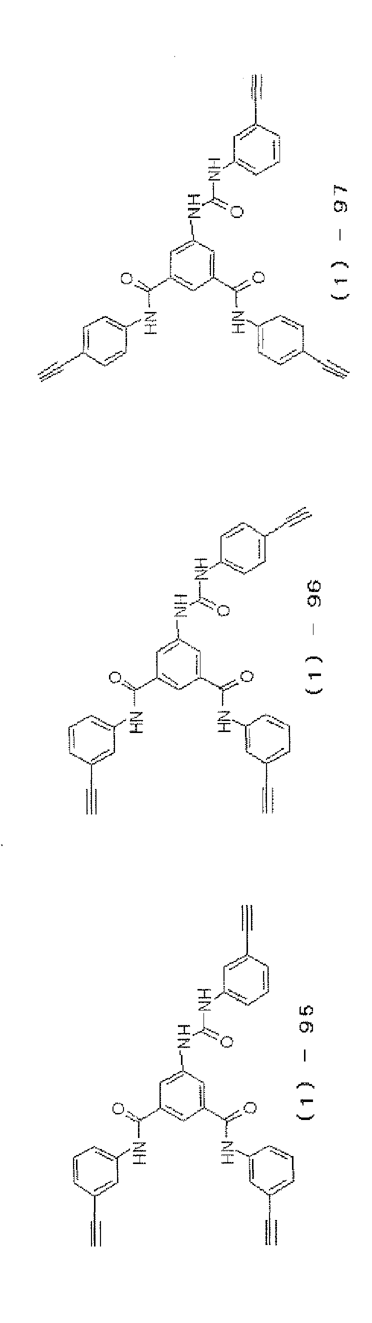

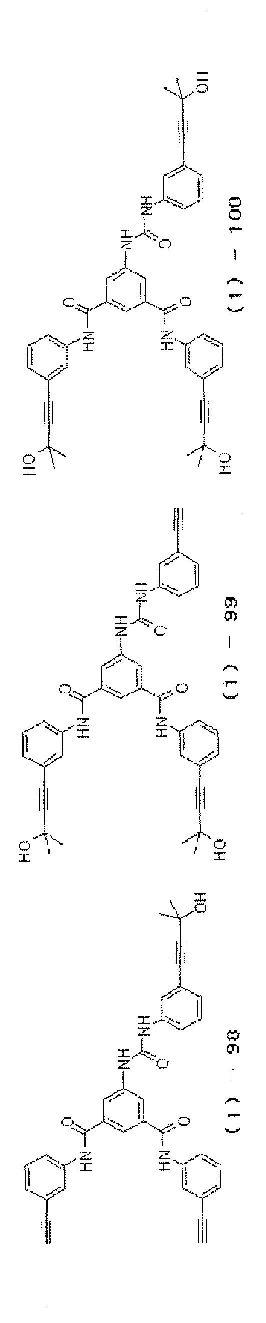

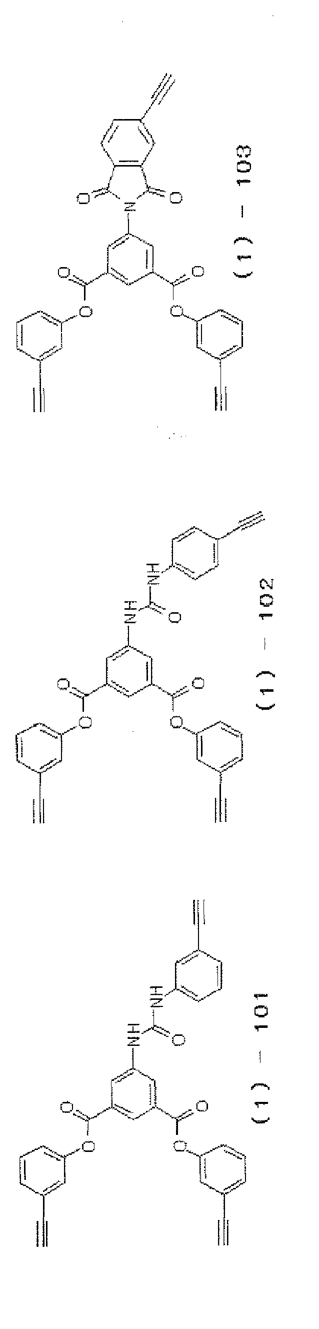

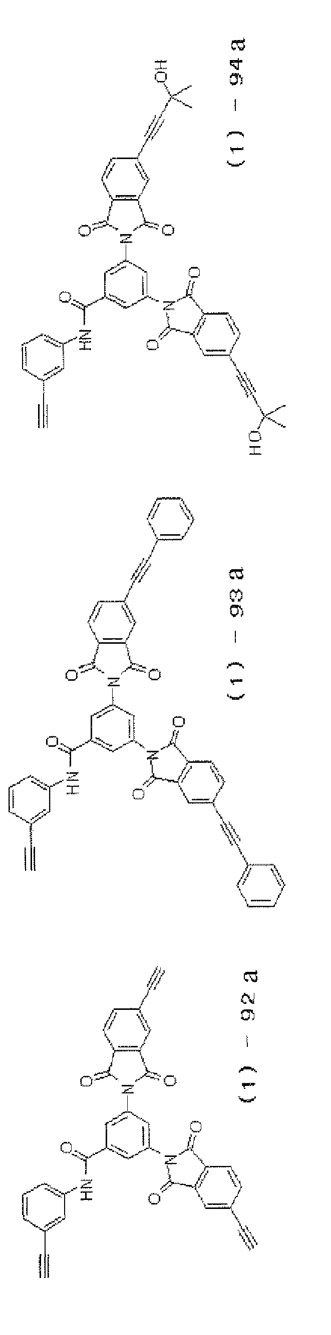

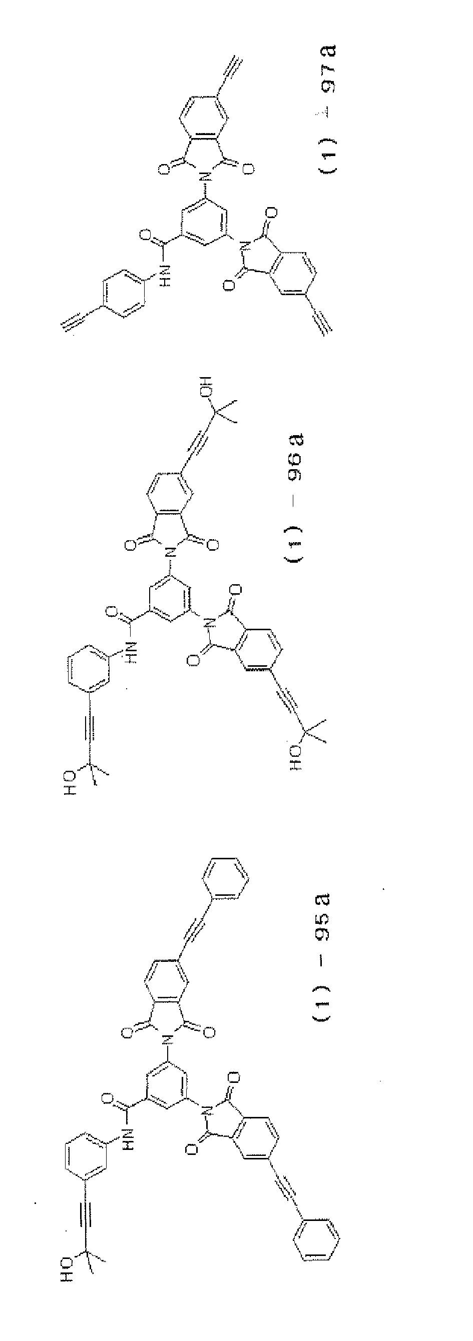

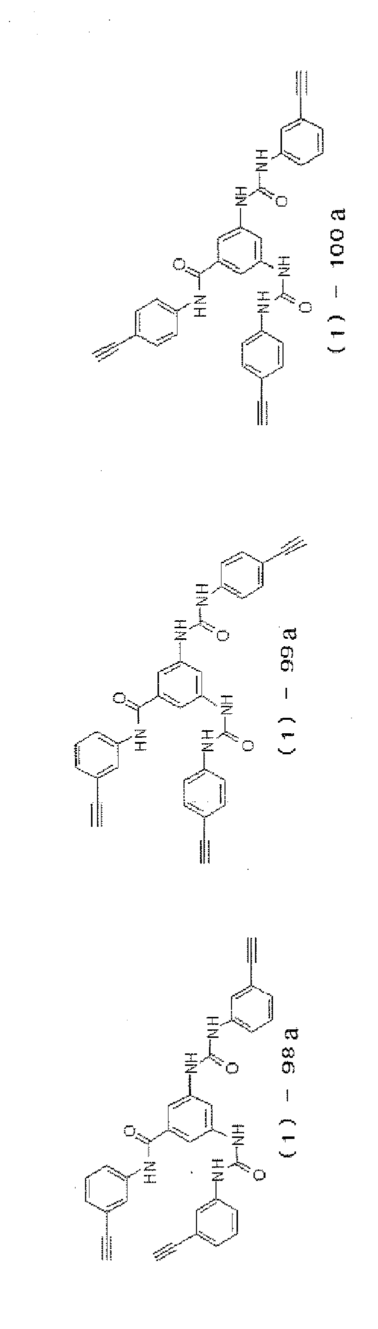

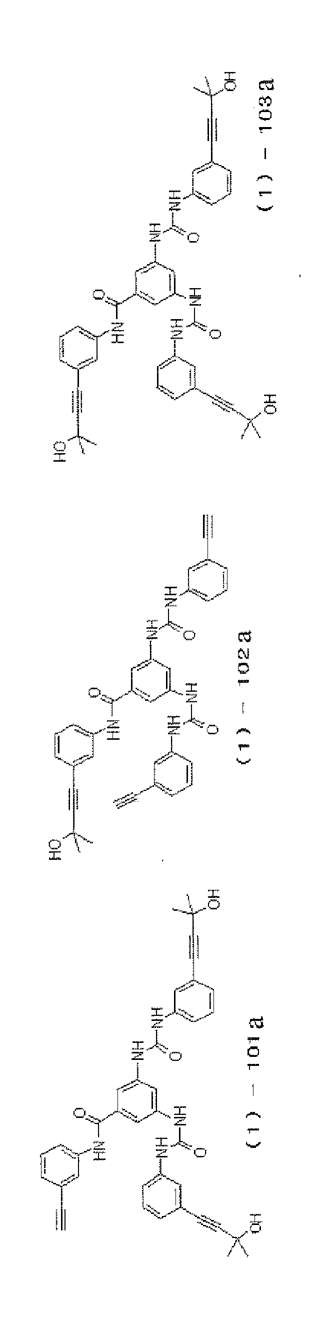

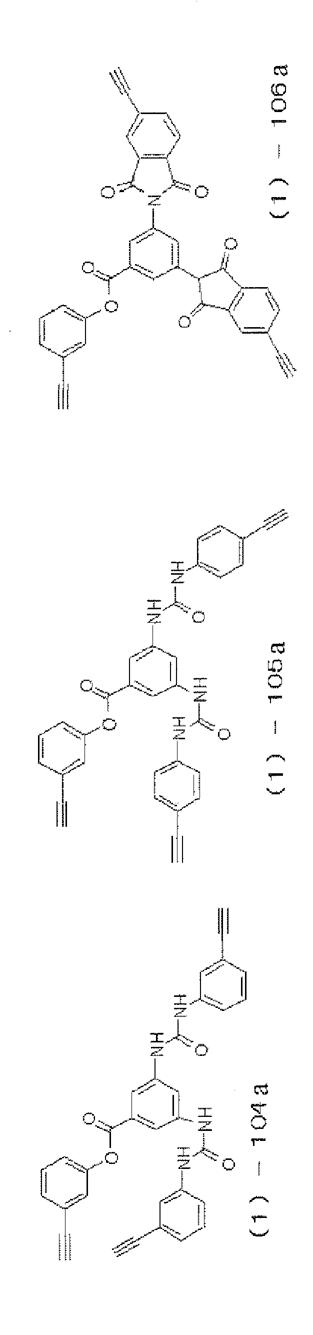

### <Explanations on polymer derived from acetylene compound>

As the polymer containing the acetylene compound of the invention as a constitutional unit, a polymer, in which the compound represented by Formula (1) is bonded as a part of a dicarboxylic acid compound or a part of the terminal end constitutional unit of a condensation polymer containing a condensation monomer represented by the following Formula (17) as a constitutional unit, ,is exemplified.

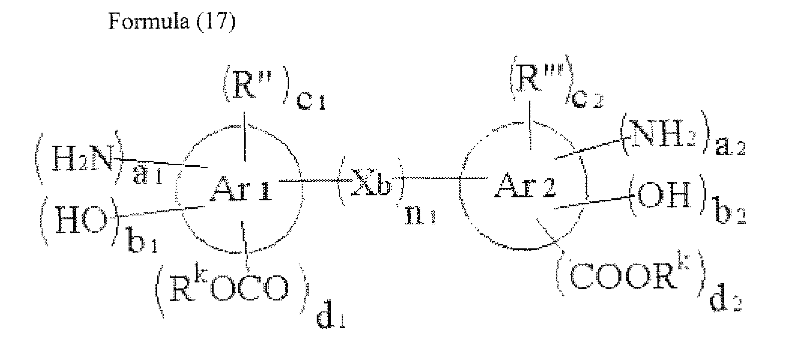

Here, R" and R"' each represent a group capable of substituting on an aryl group, and the encircled Ar1 and Ar2 each represent an aryl group or a heteroaryl group. Xb represents the following Formula (18).
R^{k} has the same definition as that of Q in Formula (1). a1, b1, c1, d1, and a2, b2, c2 and d2 each represent an integer from 0 to 5. However, all of them are not 0 simultaneously. n1 represents an integer from 0 or more, and when n1 is 0, the unit of the encircled Ar2 is not present. When c1, c2, d1 and d2 each are 2 or more, plurally present R^{k}(s), R" (s) and R"' (s), may be the same with one another, or may be different from one another, respectively.

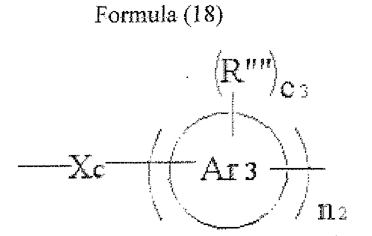

Here, R"" represent a group capable of substituting on the aryl ring, and the encircled Ar3 represents an aryl group or a heteroaryl group. Xc represents a single bond, or a divalent linking group such as -O-, -S-, -SO-, -SO₂-, an alkylene and alkenylene each having 1 to 10 carbon atoms, -NR(C=O)-, -NR(C=O)O-, -NR(C=O)NR'-, -(C=O)-, -(C=O)O-, -O(C=O)O-, -(C=O)S-, -NR(C=S)-, -NR(C=S)NR'- or -O(C=S)O-. R and R' each have the same definitions as those ofR and R' in Formula (1). c3 represents an integer from 0 to 4. n2 represents 0 or 1. When c3 is 2 or more, plurally present R"" (s) may be the same with one another, or may be different from one another.

Specifically, the encircled Ar1, the encircled Ar2 and the encircled Ar3 each are a benzene ring, a naphthalene ring, an anthracene ring, an indan ring, a benzimidazole ring, a benzopyrazole ring, a carbazole ring, an indolenine ring, a quinoline ring, a pyridine ring, a pyrimidine ring, an imidazole ring, a pyrazole ring, a furan ring, a thiophene ring, a pyrrole ring and the like. From the viewpoint of the availability of raw materials, the reactivity, or the like a benzene ring, a naphthalene ring and the like are desirable, and in particular, a benzene ring is desirable.

From the viewpoints of the availability of raw materials, the reactivity, and the like, Xc is preferably a single bond or -O. -S-, -SO₂-, an alkylene and alkenylene each having 1 to 6 carbon atoms, -NR(C=O)-, -NR(C=O)O-, -NR(C=O)NR', -(C=O)-, -(C=O)O- or -O(C=O)O-, more preferably a single bond or -O-, an alkylene having 1 to 6 carbon atoms, -NR(C=O)-, -NR(C=O)O-, -NR(C=O)NR'- or -(C=O)-, and particularly preferably a single bond, -O- or propylene.

Examples of R", R"' and R"" each include a halogen atom (-F, -Br, -Cl, -I), an alkyl group, an alkenyl group, an aryl group, an alkoxy group, an aryloxy group, a mercapto group, an alkylthio group, an arylthio group, an alkyldithio group, an aryldithio group, an N-alkylamino group, an N,N-diarylamino group, an N-alkyl-N-arylamino group, an acyloxy group, a carbamoyloxy group, an N-alkylcarbamoyloxy group, an N-arylcarbamoyloxy group, an N,N-dialkylcarbamoyloxy group, an N,N-diarylcarbamoyloxy group, an N-alkyl-N-arylcarbamoyloxy group, an alkylsulfoxy group, an arylsulfoxy group, an acylthio group, an acylamino group, an N-alkylacylamino group, an N-arylacylamino group, a ureido group, an N'-alkylureido group, an N',N'-dialkylureido group, an N'-arylureido group, an N',N'-diarylureido group, an N'-alkyl-N'-arylureido group, an N-alkylureido group, an N-arylureido group, an N'-alkyl-N-alkylureido group, an N'-alkyl-N-arylureido group, an N', N'-dialkyl-N-alkylureido group, an N',N'-dialkyl-N-arylureido group, an N'-aryl-N-alkylureido group, an N'-aryl-N-arylureido group, an N',N'-diaryl-N-alkylureido group, an N',N'-diaryl-N-arylureido group, an N'-alkyl-N'-aryl-N-alkylureido group, an N'-alkyl-N'-aryl-N-arylureido group, an alkoxycarbonylamino group, an aryloxycarbonylamino group, an N-alkyl-N-alkoxycarbonylamino group, an N-alkyl-N-aryloxycarbonylamino group, an N-aryl-N-alkoxycarbonylamino group, an N-aryl-N-aryloxycarbanylamino group, a formyl group, an acyl group, an acyloxy group, an alkoxycarbonyl group, an arylcarbonyl group, an arylcarbonyloxy group, an aryloxycarbonyl group, a carbamoyl group, an N-alkylearbamoyl group, an N,N-dialkylcarbamoyl group, an N-arylcarbamoyl group, an N,N-diarylearbamoyl group, an N-alkyl-N-arylcarbamoyl group, an alkylsulfinyl group, an arylsulfinyl group, an alkylsulfonyl group, an arylsulfonyl group, an alkoxysulfonyl group, an aryloxysulfonyl group, a sulfinamoyl group, an N-alkylsulfinamoyl group, an N,N-dialkylsulfinamoyl group, an N-arylsulfinamoyl group, an N, N-diarylsulfinamoyl group, an N-alkyl-N-arylsuifinamoyl group, a sulfamoyl group, a N-alk-ylsulfainoyl group, an N,N-dialkylsulfamoyl group, an N-arylsulfamoyl group, an N,N-diarylsulfamoyl group, an N-alkyl-N-arylsulfamoyl group, a dialkylphosphono group, a diarylphsphono group, an alkylarylphosphono group, a monoalkylphosphono group, a monoarylphosphono group, a dialkylphosphonoxy group, a diarylphosphonoxy group group, an alkylarylphosphonoxy group, a monoalkylphosphonoxy group, a monoarylphosphonoxy group, a morphorino group, a cyano group and a nitro group.

Specific examples of the alkyl group in these substituents include a methyl group, an ethyl group, a propyl group, a butyl group, a pentyl group, a hexyl group, a heptyl group, an octyl group, an isopropyl group, an isobutyl group, an s-butyl group, a t-butyl group, an isopentyl group, a neopentyl group, a 1-methylbutyl group, an isohexyl group, a 2-ethylhexyl group, a 2-methylhexyl group, a cyclopentyl group and the like. Specific examples of the aryl group include a phenyl group, a biphenyl group, a naphthyl group, a tolyl group, a xylyl group, a mesityl group, a cumenyl group, a chlorophenyl group, a bromophenyl group, a chloromethylphenyl group, a hydroxyphenyl group, a methoxyphenyl group, an ethoxyphenyl group, a phenoxyphenyl group, an acetoxyphenyl group, a benzoyloxyphenyl group, a methylthiophenyl group, a phenylthiophenyl group, a methylaminophenyl group, a dimethylaminophenyl group, an acetylaminophenyl group, a carboxyphenyl group, a methoxycarbonylphenyl group, an ethoxyphenylcarbonyl group, a phenoxycarbonylphenyl group, an N-phenylcarbamoylphenyl group, a phenyl group, a cyanophenyl group, a sulfophenyl group, a sulfonatophenyl group, a phosphonophenyl group, a phosphonatophenyl group and the like. Further, examples of the alkenyl group include a vinyl group, a 1-propenyl group, a 1-butenyl group, a cinnamyl group, a 2-chloro-1-ethenyl group and the like. As G1 in the acyl group (G1CO-), a hydrogen, the alkyl group and the aryl group in the above may be exemplified.

Preferable examples of these substituents include a halogen atom (- F. - Br, -Cl), an alkyl group, an aryl group, an alkenyl group, an alkoxy group, an aryloxy group, an alkylthio group, an arylthio group, an N,N-dialkylamino group, an acyloxy group, an N-alkylcarbamoyloxy group, an N-arylcarbamoyloxy group, an acylamino group, an acyl group, an alkoxycarbonyl group, an arylcarbonyl group, an arylcarbonyloxy group, an aryloxycarbonyl group, a carbamoyl group, an N-alkyleabamoyl group, an N,N-dialkylcarbamoyl group, an N-arylcarbamoyl group, an N-alkyl-N-arylcarbamoyl group, a sulfonato group, a sulfamoyl group, an N-alkylsulfamoyl group, an N,N-dialkylsulfamoyl group, an N-arylsulfamoyl group, an N-alkyl-N-arylsulfamoyl group and a cyano group.
More preferable examples of these substituents include a halogen atom (- F.- CI), an alkyl group (a methyl group, a trifluoromethyl group, an ethyl group, a trifluoroethyl group, a propyl group, a butyl group, an isopropyl group, a t-butyl group), an aryl group (a phenyl group, a tolyl group, a mesityl group, a cumenyl group, a chlorophenyl group, a methoxyphenyl group, an ethoxyphenyl group, an acetoxyphenyl group, a benzoyloxyphenyl group), an alkoxy group (a methoxy group, an ethoxy group, an isopropoxy group), an aryloxy group (a phenoxy group), an acyloxy group (an acetoxy group, a propionyloxy group), an acetyl group, an acetoxy group, a benzoyl group, a benzoyloxy group and an acylamino group (an acetylamino group).

c1, c2 and c3 each are preferably from 0 to 2, more preferably 0 or 1, and particularly preferably 0.
R and R' each are preferably a hydrogen atom; an alkyl group and an alkyl halide group each having 1 to 20 carbon atoms, and an aryl group having 6 to 20 carbon atoms, more preferably a hydrogen atom; an alkyl group and an alkyl halide group each having 1 to 8 carbon atoms and a phenyl group, and even more preferably a hydrogen atom, a methyl group, an ethyl group, a chloromethyl group and a fluoromethyl group.

Examples of polymers derived from the acetylene compound of the invention include polymers containing a compound represented by Formula (1), preferably a compound represented by Formula (2) as a part of a dicarboxylic acid compound or a monocarboxylic acid compound in the main chain or at the terminal end of a polymer of any of a condensate of a dicarboxylic acid compound and a tetraamino compound, a condensate of a dicarboxylic acid compound and a bis(orthohydroxyamino) compound, a condensate of a dicarboxylic acid compound and an diamino compound, a condensate of a dicarboxylic acid compound and a diol compound, and a condensate of a tetracarboxylic acid dianhydride, a dicarboxylic acid compound and a diamino compound. Examples of the dicarboxylic acids other than ones represented by Formula (2) also include dicarboxylic compounds in which in Formula (17), a1, b1, a2 and b2 each are 0, and d1 and d2 each are 1; a substituted or unsubstituted (hetero)aryldicarboxylic acid or aliphatic dicarboxylic acid; and dicarboxylic acid derivatives recited in Jikken Kagaku Koza (Courses in Experimental Chemistry) (Maruzen Co., Ltd.) and New Polymer Experimentology (Kyoritsu Shuppan Co., Ltd.).

Among them, compounds, in which in Formula (17), the encircled Ar1, the encircled Ar2 and the encircled Ar3 each are a benzene ring or a naphthalene ring; R", R"' and R"" each are a halogen atom (-F, -Cl), an alkyl group (a methyl group, a trifluoromethyl group, an ethyl group, a trifluoroethyl group, a propyl group, a butyl group, an isopropyl group, a t-butyl group, or the like), an aryl group (a phenyl group, a tolyl group, a mesityl group, a cumenyl group, a chlorophenyl group, a methoxyphenyl group, an ethoxyphenyl group, an acetoxyphenyl group, a benzoyloxyphenyl group, or the like), an alkoxy group (a methoxy group, an ethoxy group, an isopropoxy group, or the like), an aryloxy group (such as a phenoxy group), an acyloxy group (such as an acetoxy group, or a propionyloxy group), an acetyl group, a benzoyl group, a benzoyloxy group, or an acylamino group (such as an acetylamino group); Xc is a single bond, -O-, -S-, -SO₂-, an alkylene having 1 to 10 carbon atoms, -NR(C=O)-, -NR(C=O)O-, -NR(C=O)NR'-, -(C=O)-, or -(C=O)O-, (here, R and R' each are a hydrogen atom or a methyl group), c1, c2, and c3 each are 0 to 2, and n1 and n2 each are 0 or 1; or (hetero)aryldicarboxylic acids compound having I to 10 carbon atoms which may be substituted or unsubstituted (here, the substituents the same as those of R" above); and an aliphatic dicarboxylic acid compound having 1 to 10 carbon atoms which may be substituted or unsubstituted (here, the substituents are the same as those of R" above), are preferable; compounds represented in which in Formula (17), the encircled Ar1 and Ar2 each are a benzene ring; R" and R"' each are a halogen atom (- F, -Cl), an alkyl group (such as a methyl group, an ethyl group, an isopropyl group, or a t-butyl group), an alkoxy group (such as a methoxy group, or an ethoxy group), an aryloxy group (such as a phenoxy group), an acetoxy group, an acetyl group, or a benzoyloxy group; Xc is a single bond or -O-, and -S-. -SO₂-, an alkylene having 1 to 6 carbon atoms, -NR(C=O)-, -NR(C=O)O, -(C=O)- or -(C=O)O-, (here, R is a hydrogen atom or a methyl group); c1 and c2 each are 0 to 2; n1 is 0 or 1; and n2 is 0; or an aryldicarboxylic acid compound having 1 to 10 carbon atoms which may be substituted or unsubstituted (here, the substituents are the same as those of R" above); or an aliphatic dicarboxylic acid compound having 1 to 6 carbon atoms which may be substituted or unsubstituted (here, the substituents are the same as those of R" above), are more preferable; and, dicarboxylic acid derivative compounds in which in Formula (17), the encircled Ar1. and Ar2 each are a benzene ring; Xc is a single bond or -O-, -SO₂-, an alkylene having 1 to 4 carbon atoms, -NR(C=O)-, -NR(C=O)O- or -(C=O)-, in which R is a hydrogen atom or a methyl group; c1 and c2, each are 0 to 1; n1 is 0 or 1; and n2 is 0, or substituted or unsubstituted phthalic acid compounds (here, the substituents are the same as R" above), are particularly preferable.

Specific examples of the dicarboxylic acid compounds other than Formula (2) include, for example, 4,4'-diearboxybiphenyl, 2,2-bis(4-carboxyphenyl)propane, bis(4-carboxyphenyl) sulfone, 4,4'-dicarboxybenzophenone, 4,4'-dicarboxybiphenyl ether, 3,3'-dicarboxybiphenyl, 2,2-bis(3-carboxyphenyl)propane, bis(3-carboxyphenyl) sulfone, 3.3'-dicarbaxybenzophenone, 4,4'-dicarboxy-3,3'-dimethylbiphenyl ether, 4,4'-dicarboxy-3,3'-dimethylbiphenyl, 2,2-bis(4-carboxy-3-methylphenyl)propane, bis(4-carboxy-3-methylphenyl) sulfone, 4,4'-dicarboxy-3,3'-dimethylbenzophenone, 4,4'-dicarboxy-3,3'-dichlorobiphenyl, 2,2-bis(4-carboxy-3-chlorophenyl)propane, bis(4-carboxy-3-chlorophenyl) sulfone, 4,4'-dicarboxy-3,3'-dichlorobenzophenone, phthalic acid, isophthalic acid, terephthalic acid, 4-methyl phthalic acid, 4-methyl isophthalic acid, 2,5-dimethyl terephthalic acid and the like, but are not limited to these compounds. Further, esters (for example, methyl ester, ethyl ester, propyl ester, 2-hydroxyethyl ester, or the like) of these dicarboxylic acid compounds with lower alcohols may be used.

As the tetraamino compounds, compounds represented by Formula (17), wherein b1, d1, b2 and d2 each are 0; and a1 and a2 each are 2, or a substituted or unsubstituted (hetero)aryltetraamino compounds may be exemplified, and tetraamino compound derivatives recited in Jikken Kagaku Koza (Courses in Experimental Chemistry) (Maruzen Co., Ltd.) and New Polymer Experimentology (Kyoritsu Shuppan Co., Ltd.) may be exemplified.

Among them, tetraamino compounds in which in Formula (17), the encircled Ar1, the encircled Ar2 and the encircled Ar3 each are a benzene ring or a naphthalene ring; R", R"' and R"" each are a halogen atom (-F, -CI), an alkyl group (such as a methyl group, a trifluoroethyl group, an ethyl group, a trifluoroethyl group, a propyl group, a butyl group, an isopropyl group, a t-butyl group or the like), an aryl group (such as a phenyl group, a tolyl group, a mesityl group, a cumenyl group, a chlorophenyl group, a ethoxyphenyl group, an ethoxyphenyl group, an acetoxyphenyl group, a benzoyloxyphenyl group or the like), an alkoxy group (such as a methoxy group, an ethoxy group, an isopropoxy group or the like), an aryloxy group (such as a phenoxy group), an acyloxy group (such as an acetoxy group, a propionyloxy group or the like), an acetyl group, a benzoyl group, a benzoyloxy group, or an acylamino group (such as an acetylamino group, a propionylamino group or the like); Xc is a single bond, -O-, -S-, -SO₂- an alkylene having 1 to 10 carbon atoms, -NR(C=O)-, -NR(C=O)O-, -NR(C=O)NR'-, -(C=O)- or -(C=O)O-, (here, R and R' each are a hydrogen atom or a methyl group); c1, c2, and c3 each are 0 to 2; and n1 and n2 each are 0 or 1, or (hetero)aryltetraamino compound having 1. to 10 carbon atoms which may be substituted or unsubstituted (here, the substituents are the same as R" above), are preferable; tetraamino compounds in which in Formula (17), the encircled Ar1 and Ar2 each are a benzene ring; R" and R"' each are a halogen atom (- F,-Cl), an alkyl group (such as a methyl group, an ethyl group, an isopropyl group, a t-butyl group, or the like), an alkoxy group (such as a methoxy group, an ethoxy group, or the like), an aryloxy group (such as a phenoxy group, or the like), an acetoxy group, an acetyl group, a benzoyloxy group, or an acylamino group (such as an acetylamino group, a propionylamino group, or the like); Xc is a single bond, -O-, and -S-. -SO₂-, an alkylene having 1 to 6 carbon atoms, -NR(C=O)-, -NR(C=O)O-, -(C=O)- or -(C=O)O- (here R is a hydrogen atom or a methyl group); c1 and c2, each are 0 to 2; n1 is 0 or 1; and n2 is 0, or aryltetraamino compounds having 1 to 10 carbon atoms which may be substituted or unsubstituted (here, the substituents are the same as R" above), are more preferable; and, tetraamino compounds, in which in Formula (17), the encircled Ar1 and Ar2 each are a benzene ring; Xc is a single bond, -O- -SO₂-, an alkylene having 1 to 4 carbon atoms, -NR(C=O)-, -NR(C=O)O- or -(C=O)- (here, R is a hydrogen atom or a methyl group); c1 and c2, each are 0 to 1; n1 is 0 or 1; and n2 is 0, or tetraaminobenzene compounds having 1 to 10 carbon atoms which may be substituted or unsubstituted (here, the substituents are the same as R" above), are particularly preferable.

Examples of the tetraamino compound include, for example, 3,3',4,4'-tetraaminobipbenyl, 2,2-bis(3,4-diaminophenyl)propane, bis(3,4-diaminophenyl) sulfone, 3,3',4,4'-tetraaminobenzophenone, 3,3',4,4'-tetraaminobiphenyl ether, 3,3',4,4'-tetraamino-5,5'-dimethylbiphenyl ether, 3,3',4,4'-tetraamino-5,5'-dimethylbiphenyl, 2,2-bis(3,4-diamino-5-methylphenyl)propane, bis(3,4-diamino-5-methylphenyl) sulfone, 3,3',4,4'-tetraamino-5,5'-dimethylbenzophenone, 3,3',4,4'-tetraamino-5,5'-dichlorobiphenyl, 2,2-bis(3,4-diamino-5-chlorophenyl)propane, bis(3,4-diamino-5-chlorophenyl) sulfone, 3,3',4,4'-tetraamino-5,5-dichlorobenzophenone, 1,2, 4,5-tetraaminobenzene and the like, but are not limited to these compounds.

As the bis(orthohydroxyamino) compounds, compound represented by Formula (17), in which d1 and d2 each are 0; and a1, a2, b1 and b2 each are 1, or substituted or unsubstituted (hetero)arylbis(orthobydroxyamino) compounds may be exemplified, and bis(orthohydroxyamino) compound derivatives recited in Jikken Kagaku Koza (Courses in Experimental Chemistry) (Maruzen Co., Ltd.) and New Polymer Experimentology (Kyoritsu Shuppan Co., Ltd.) may also be exemplified.

Among them, bis(orthohydroxylamino) compounds in which in Formula (17), the encircled Ar1, the encircled Ar2 and the encircled Ar3 each are a benzene ring or a naphthalene ring; R", R'" and R"" each are a halogen atom (-F, -Cl), an alkyl group (such as a methyl group, a trifluoroethyl group, an methyl group, a trifluoroethyl group, a propyl group, a butyl group, an isopropyl group, a t-butyl group, or the like), an aryl group (such as a phenyl group, a tolyl group, a mesityl group, a cumenyl group, a chlorophenyl group, a methoxy phenyl group, an ethoxy phenyl group, an acetoxy phenyl group, a benzoyloxy phenyl group, or the like), an alkoxy group (such as a methoxy group, an ethoxy group, an isopropoxy group, or the like), an aryloxy group (such as a phenoxy group, or the like), an acyloxy group (such as an acetoxy group, a propionyloxy group, or the like), an acetyl group, a benzoyl group, a benzoyloxy group, or an acylamino group (such as an acetylamino group, a propionylamino group, or the like); Xc is a single bond, -O-, -S-, -SO₂-, an alkylene having 1 to 10 carbon atoms, -NR(C=O)-, -NR(C=O)O-, -NR(C=O)NR'-, -(C=O)-, or -(C=O)O- (here, R and R' each are a hydrogen atom or a methyl group); c1, c2, and c3 each are 0 to 2; and n1 and n2 each are 0 or 1; or azylbis(orthohydrpxylamino) compounds having 1 to 10 carbon atoms which may be substituted or unsubstituted (here, the substituents are the same as R" above), are preferable; bis(orthohydroxylmino) compounds, in which in Formula (17), the encircled Ar1 and Ar2 each are a benzene ring; R" and R"' each are a halogen atom (- F, -Cl), an alkyl group (such as a methyl group, an ethyl group, an isopropyl group, a t-butyl group, or the like), an alkoxy group (such as a methoxy group, an ethoxy group, or the like), an aryloxy group (such as a phenoxy group, or the like), an acetoxy group, an acetyl group, a benzoyloxy group, or an acylamino group (such as an acetylamino group, a propionylamino group, or the like); Xc is a single bond, -O-, -S-, -SO₂-, an alkylene having 1 to 6 carbon atoms, -NR(C=O)-, -NR(C=O)O-, -(C=O)- or -(C=O)O-, in which R is a hydrogen atom or a methyl group; c1 and c2 each are 0 to 2; n1 is 0 or 1; and n2 is 0, or bis(orthohydroxylamino) benzene compounds having 1 to 10 carbon atoms which may be substituted or unsubstituted (here, the substituents are the same as R" above) are more preferable; and, bis(orthohydroxylamino) compounds, in which in Formula (17), the encircled Ar1 and Ar2 each are a benzene ring; Xc is a single bond, -O-, -SO₂-, an alkylene having 1 to 4 carbon atoms, -NR(C=O)-, -NR(C=O)O- or -(C=O)-, (here, R is a hydrogen atom or a methyl group); c1 and c2, each are 0 to 1; n1 is 0 or 1; and n2 is 0, or bis(orthohydroxylamino) benzene compounds having 1 to 10 carbon atoms which may be substituted or unsubstituted (here, the substituents are the same as R" above) are particularly preferable.

Specific examples of the bis(orthohydroxyamino) compound include, for example, 3,3'-diamino-4,4'-dihydroxybiphenyl, 2,2-bis(3-amino 4-hydroxyphenyl)propane, bis(3-amino-4-hydroxyphenyl) sulfone, 3,3'-diamino-4,4'-dihydroxybenzophenone, 3,3'-diamino-4,4'-dihydroxybiphenyl ether, 3,3'-diamino-4,4'-dihydroxy-5,5'-dimethylbiphenyl ether, 3,3'-diamino-4,4'-dihydroxy-5,5'-dimethylbiphenyl, 2,2-bis(3-amino-4-hydroxy-5-methylphenyl)propane, bis(3-amino-4-hydroxy-5-methylphenyl) sulfone, 3,3'-diamino-4,4'-dihydroxy-5,5'-dimethylbenzophenone, 3,3'-diamino-4,4'-dihydroxy-5,5'-dichlorobiphenyl, 2,2-bis(3-amino-4-hydroxy-5-chlorophenyl)propane, bis(3-amino-4-hydroxy-5-chlorophenyl) sulfone, 3,3'-diamino-4,4'-dihydroxy-5,5'-dichlorobenzophenone, 1,4-diamino-2,5-dihydroxybenzene, and the like, but are not limited to these compounds.

As the diamine compounds, compounds in which, in Formula (17), b1, d1, b2 and d2 each are 0; and a1 and a2 each are 1, substituted or unsubstituted diamino(hetero)aryl compounds, or aliphatic diamine compounds may be exemplified, and diamine compound derivatives recited in Jikken Kagaku Koza (Courses in Experimental Chemistry) (Maruzen Co., Ltd.) and New Polymer Experimentology (Kyoritsu Shuppan Co., Ltd.) may also be exemplified.

Among them, compounds in which, in Formula (17), the encircled Ar1, the encircled Ar2 and the encircled Ar3 each are a benzene ring or a naphthalene ring; R", R"' and R"" each are a halogen atom (-F, -Cl), an alkyl group (a methyl group, a trifluoromethyl group, an ethyl group, a trifluoromethyl group, a propyl group, a butyl group, an isopropyl group, a t-butyl group), an aryl group (a phenyl group, a tolyl group, a mesityl group, a cumenyl group, a chlorophenyl group, a methoxyphenyl group, an ethoxyphenyl group, an acetoxyphenyl group, a benzoyloxyphenyl group), an alkoxy group (a methoxy group, an ethoxy group, an isopropoxy group), an aryloxy group (a phenoxy group), an acyloxy group (an acetoxy group, a propionyloxy group), an acetyl group, a benzoyl group, a benzoyloxy group, or an acylamino group (an acetylamino group, a propionylamino group); Xc is a single bond, -O-, -S-, -SO₂-, an alkylene having 1 to 10 carbon atoms, -NR(C=O)-, -NR(C=O)O-, -NR(C-O)NR'-, -(C=O)-, or -(C=O)O- (here, R and R' each are a hydrogen atom or a methyl group); c1, c2, and c3 each are 0 to 2; and n1 and n2 each are 0 or 1, or diamino(hetero)aryl compounds having 1 to 10 carbon atoms which may be substituted or unsubstituted (here, the substituent has the same definition as that in R" above), or aliphatic diamine compounds having 1 to 12 carbon atoms, are preferable; compounds in which, in Formula (17), the encircled Ar1 and Ar2 each are a benzene ring; R" and R"' each are a halogen atom (- F, -Cl), an alkyl group (a methyl group, an ethyl group, an isopropyl group, a t-butyl group), an alkoxy group (a methoxy group, an ethoxy group), an aryloxy group (a phenoxy group), an acetoxy group, an acetyl group, a benzoyloxy group, or an acylamino group (an acetylamino group, a propionylamino group); Xc is a single bond, -O-, -S-. -SO₂-, an alkylene having I to 6 carbon atoms, -NR(C=O)-, -NR(C=O)O-, -(C=O)- or -(C=O)O-, in which R is a hydrogen atom or a methyl group; c1 and c2, each are 0 or 1; n1 is 0 or 1; n2 is 0, or diamino(hetero)aryl compounds having 1 to 6 carbon atoms which may be substituted or unsubstituted (here, the substituent has the same definition as that in R" above), or aliphatic diamine compounds having 1 to 8 carbon atoms, are more preferable; and diamine compound derivatives in which, in Formula (17), the encircled Ar1 and Ar2 each are a benzene ring; Xc is a single bond, -O-, -SO₂-, an alkylene having 1 to 4 carbon atoms, -NR(C=O)-, -NR(C=O)O- or -(C=O)-, (here, R is a hydrogen atom or a methyl group); c1 and c2 each are 0 to 1; n1 is 0 or 1; and n2 is 0, substituted or unsbstituted diaminobenzene (here, the substituent has the same definition as that in R" above), or aliphatic diamine compounds having 1 to 6 carbon atoms are particularly preferable.

Examples of the diamine compound according to the invention are not particularly limited, but include the following diamine compounds.
p-phenylenediamine, m-phenylenediamine, o-phenylenediamine, 1,4-diamino-2-methylbenzene, 1,3-diamino-4-methyl-benzene, 1,3-diamino-4-chloro-benzene, 1,3-diamino-4-acetylamino-benzene, 1,3-bisaminoethyl-benzene, hexamethylenediamine, 3,3'-diaminobiphenyl, 4,4'-diamino-3.3'-dimethylbipheyl, 4,4'-diamino-3,3'-dichlorobiphenyl, 2,2'-difluoro-4,4'-diaminobiphenyl, 3,3'-difluoro-4,4'-diaminobiphenyl, 2,2'-difluoro-5.5'-diaminobiphenyl, 3,3'-difluoro-5,5'-diaminobiphenyl, 2,2'-dichloro-4,4'-diaminobiphenyl, 3,3'-dichloro-4,4'-diaminobiphenyl, 2,2'-dichloro-5,5'-diaminobiphenyl, 3,3'-dichloro-5,5'-diaminobiphenyl, 2,2-dibromo-4,4'-diaminobiphenyl, 3,3'-dibromo-4,4'-diaminobiphenyl, 2,2'-dibromo-5,5'-diaminobiphenyl, 3,3'-dibromo-5,5'-diaminobiphenyl, 2,2-bis(trifluoromethyl)-4,4'-diaminobiphenyl, 3,3'-bis(trifluoromethyl)-4,4'-diaminobiphenyl, 2,2'-bis(trifluoromethyl)-5,5'-diaminobiphenyl, 3,3'-bis(trifluoromethyl)-5,5'-diaminobiphenyl, 2,2'-bis(trichloromethyl)-4,4'-diaminobiphenyl, 3,3'-bis(trichloromethyl)-4,4'-diaminobiphenyl, 2,2'-bis(trichloromethyl)-5,5'-diaminobiphenyl, 3,3'-bis(trichloromethyl)-5,5'-diaminobiphenyl, 2,2'-bis(tribromomethyl)-4,4'-diaminobiphenyl, 3,3'-bis(tribromomethyl)-4,4'-diaminobiphenyl, 2,2'-bis(tribromomethyl)-5,5'-diaminobiphenyl, 3,3'-bis(tribromomethyl)- 5,5'-diaminobiphenyl, 3,3'-diaminodiphenyl ether, 3,4'-diaminodiphenyl ether, 4,4'-diaminodiphenyl ether, 4,4'-diamino-3,3'-dimethylbiphenyl ether,

3,3'-diaminodiphenyl sulfide, 3,4'-diaminodiphenyl sulfide, 4,4'-diaminodiphenyl sulfide, 3,3'-diaminodiphenyl sulfone, 3,4'-diaminodiphenyl sulfone, 4,4'-diaminodiphenyl sulfone, bis(4-amino-3-methylphenyl) sulfone, bis(4-amino-3-chlorophenyl) sulfone, bis(4-aminophenyl) sulfone, bis(3-aminophenyl) sulfone, bis(5-fluoro-4-aminophenyl) sulfone, bis(5-fluoro-3-aminophenyl) sulfone, bis(5-chloro-4-aminophenyl) sulfone, bis(5-chloro-3-aminophenyl) sulfone, bis(5-bromo-4-aminophenyl) sulfone, bis(5-bromo-3-aminophenyl) sulfone, bis(5-trifluoromethyl-4-aminopheny) sulfone, bis(5-trifluoromethyl-3-aminophenyl) sulfone, bis(5-trichloromethyl-4-aminophenyl) sulfone, bis(5-trichloromethyl-3-aminophenyl) sulfone, bis(5-tribromomethyl-4-aminophenyl) sulfone, bis(5-tribromomethyl-3-aminophenyl) sulfone, 3,3'-diaminobenzophenone, 4,4'-diaminobenzophenone, 3,4'-diaminobenzophenone, 4,4'-diamino-3,3'-dimethylbenzophenone, 4,4'-diamino-3,3'-dichlorobenzophenone, 3,3'-diaminodiphenylmethane, 4,4'-diaminodiphenylmethane, 3,4'-diaminodiphenylmethane, 2,2-di(3-aminophenyl)propane, 2,2-di(4-aminophenyl)propane, 2-(3-aminophenyl)-2-(4-aminophenyl)propane, 2,2-di(3-aminophenyl)-1,1,1,3,3,3-hexafluoropropane, 2,2-di(4-aminophenyl)-1,1,1,3,3,3-hexafluoropropane, 2-(3-aminophenyl)-2-(4-aminophenyl)-1,1,1,3,3,3-hexafluoropropane, 2,2-bis(4-amino-3-methylphenyl)propane, 2,2-bis(4-amino-3-chlorophenyl)propane,

1,1-di(3-aminophenyl)-1-phenylethane, 1,1-di(4-aminophetryl)-1-phenylethane, 1-(3-aminophenyl)-1-(4-aminophenyl)-1-phenylethane, 1,3-bis(3-aminophenoxy)benzene, 1,3-bis(4-aminophenoxy)benzene, 1,4-bis(3-aminophenoxy)benzene, 1,4-bis(4-aminophenoxy)benzene, 1,3-bis(3-aminobenzoyl)benzene, 1,3-bis(4-aminobenzoyl)benzene, 1,4-bis(3-aminobenzoyl)benzene, 1,4-bis(4-aminobenzolyl)benzene, 1,3-bis(3-amino-α,α-dimethylbenzyl)benzene, 1,3-bis(4-amino-α,α-dimethylbenzyl)benzene, 1,4-bis(3-amino-α,α-dimethylbenzyl)benzene, 1,4-bis(4-amino-α,α-dimethylbenzyl)benzene, 1,3-bis(3-amino-α,α-ditrifluoromethylbenzyl)benzene, 1,3-bis(4-amino-α,α-ditrifluoromethylbenzyl)benzene, 1,4-bis(3-amino-α,α-ditrifluoromethylbenzyl)benzene, 1,4-bis(4-amino-α,α-ditrifluoromethylbenzyl)benzene, 2,6-bis(3-aminophenoxy)benzonitrile, 2,6-bis(3-aminophenoxy)pyridine,

4,4'-bis(3-aminophenoxy)biphenyl, 4,4'-bis(4-aminophenoxy)biphenyl, bis[4-(3-aminophenoxy)phenyl] ketone, bis[4-(4-aminophenoxy)phenyl] ketone, bis[4-(3-aminophenoxy)phenyl] sulfide, bis[4-(4-aminophenoxy)phenyl] sulfide, bis[4-(4-aminophenoxy)phenyl] sulfone, bis[4-(3-aminophenoxy)phenyl] sulfone, bis[4-(5-fluoro-4-aminophenoxy)phenyl] sulfone, bis[4-(5-fluoro-3-aminophenoxy)phenyl] sulfone, bis[4-(5-chloro-4-aminophenoxy)phenyl] sulfone, bis[4-(5-chloro-3-aminophenoxy)phenyl] sulfone, bis[4-(5-bromo-4-aminophenoxy)phenyl] sulfone, bis[4-(5-bromo-3-aminophenoxy)phenyl] sulfone, bis[4-(5-trifluoromethyl-4-aminophenoxy)phenyl] sulfone, bis[4-(5-trifiuoromethyl-3-aminophenoxy)phenyl] sulfone, bis[4-(5-trichloromethyl-4-aminophenoxy)phenyl] sulfone, bis[4-(5-trichloromethyl-3-aminophenoxy)phenyl] sulfone, bis[4-(5-tribromomethyl-4-aminophenoxy)phenyl] sulfone, bis[4-(5-tribromomethyl-3-aminophenoxy)phenyl] sulfone, bis[4-(3-aminophenoxy)phenyl] ether, bis[4-(4-aminophenoxy)phenyl] ether, bis[4-(4-aminophenoxy)phenyl]methane, 2,2-bis[4-(3-aminophenoxy)phenyl]propane, 2,2-bis[4-(4-aminophenoxy)phenyl]propane, 2,2-bis[3-(3-aminophenoxy)phenyl]-1,1,1,3,3,3-hexafluoropropane, 2,2-bis[4-(4-aminophenoxy)phenyl]-1,1,1,3,3,3-hexafluoropropane,

1,3-bis[4-(3-aminophenoxy)benzoyl]benzene, 1,3-bis[4-(4-aminophenoxy)benzoyl]benzene, 1,4-bis[4-(3-aminophenoxy)benzoyl]benzene, 1,4-bis[4-(4-aminophenoxy)benzoyl]benzene, 1,3-bis[4-(3-aminophenoxy)-α,α-dimethylbenzyl]benzene, 1,3-bis[4-(4-aminophenoxy)-α,α-dimethylbenzyl]benzene, 1,4-bis[4-(3-aminophenoxy)-α,α-dimethylbenzyl]benzene, 1,4-bis[4-(4-aminophenoxy)-α,α-dimethylbenzyl]benzene,

4,4-bis[4-(4-aminophenoxy)benzoyl]diphenyl ether, 4,4'-bis[4-(4-amino-α,α-dimethylbenzyl)phenoxy]benzophenone, 4;4'-bis[4-(4-amino-α,α-dimethylbenzyl)phenoxy]diphenyl sulfone, 4,4'-bis[4-(4-aminophenoxy)phenoxy]diphenyl sulfone, 3,3'-diamino-4,4'-diphenoxybenzophenone, 3,3'-diamino-4,4'-dibiphenoxybenzophenone, 3,3'-diamino-4-phenoxybenzophenone, 3,3'-diamino-4-biphenoxybenzophenone,

6,6'-bis(3-aminophenoxy)3,3,3',3'-tetramethyl-1,1'-spirobiindane, 6,6'-bis(4-aminophenoxy)3,3,3',3'-tetramethyl-1,1'-spirobiindane, 1,3-bis(3-aminopropyl)tetramethyldisiloxane, 1,3-bis(4-aminobutyl)tetramethyldisiloxane, α,ω-bis(3-aminopropyl)polydimethylsiloxane, α,ω-bis(3-aminobutyl)polydimethylsiloxane, and diaminopolysiloxane.

These diamine compounds as illustrated above may be used alone or as a mixture. Further, the hydrogen atoms on the aromatic ring of these diamine compounds may be partly or totally substituted by a substituent selected from a fluorine atom, a methyl group, a methoxy group, a trifluoromethyl group, or a trifluoromethoxy group. In order to introduce a branched structure, part of the diamine compound may be changed to triamines or tetraamines. Specific examples of the triamines include pararosaniline.

Examples of the tetracarboxylic dianhydride that can be used in the polymer according to the invention is not particularly limited, but include the following compounds.
Pyromellitic dianhydride, 3-fluoropyromellitic dianhydride, 3-chloropyromellitic dianhydride, 3-bromopyromellitic dianhydride, 3-trifluoromethylpyromellitic dianhydride, 3-trichloromethylpyromellitic dianhydride, 3-tribromomethylpyromellitic dianhydride, 3,6-difluoropyromellitic dianhydride, 3,6-dichloropyromellitic dianhydride, 3,6-dibromopyromellitic dianhydride, 3,6-bistrifluoromethylpyromellitic dianhydride, 3,6-bistrichloromethylpyromellitic dianhydride, 3;6-bistribromomethylpyromellitic dianhydride, 2,2',3,3'-biphenyltetracarboxylic dianhydride, 3,3',4,4'-biphenyltetracarboxylic dianhydride, 2,2',3,3'-benzophenonetetracarboxylic dianhydride, 3,3',4,4'-benzophenonetetracarboxylic dianhydride, bis(2,3-dicarboxyphenyl) ether dianhydride, bis(3,4-dicarboxyphenyl) ether dianhydride, bis(2,3-dicarboxyphenyl) sulfide dianhydride, bis(3,4-dicarboxyphenyl) sulfide dianhydride, bis(2,3-dicarboxyphenyl) sulfone dianhydride, bis(3,4-dicarboxyphenyl) sulfone dianhydride, 2,2-bis(2,3-dicarboxyphenyl)propane dianhydride, 2,2-bis(3,4-dicarboxyphenyl)propane dianhydride, 2,2-(bis(2,3-dicarboxyphenyl)-1,1,1,3,3,3-hexafluoropropane dianhydride, 2,2-bis(3,4-dicarboxyphenyl)-1,1,1,3,3,3-hexafluoropropane dianhydride, 1,3-bis(3,4-dicarboxyphenoxy)benzene dianhydride, 1,3-bis(2,3-dicarboxyphenoxy)benzene dianhydride, 1,4-bis(2,3-dicarboxyphenoxy)benzene dianhydride, 1,4-bis(3,4-dicarboxyphenoxy)benzene dianhydride, 4,4'-bis(3,4-dicarboxyphenoxy)biphenyl dianhydride, 2,2-bis[(3,4-dicarboxyphenoxy)phenyl]propane dianhydride, 9,9-bis(3,4-dicarboxyphenyl)fluorene dianhydride, 9,9-bis[4-(3,4-dicarboxyphenoxy)phenyl]fluorene dianhydride, 4,4'-biphenylenebis(trimellitic acid anhydride monoester), p-phenylenebis(trimellitic acid anhydride monoester), p-methylphenylenebis(trimellitic acid anhydride monoester), p-(2,3-dimethylphenylene)bis(trimellitic acid monoester acid anhydride), 1,4-naphthalenebis(trimellitic acid monoester acid anhydride), 2,6-naphthalenebis(trimellitic acid anhydride monoester), 2,2-bis[4-(trimellitic acid anhydride monoester)phenyl]propane, 2,2-bis[4-(trimellitic acid anhydride monoester)phenyl]hexafluoropropane, 1,2,5,6-naphthalenetetracarboxylic dianhydride, 2,3,5,6-pyridinetetracarboxylic dianhydride, 3,4,9,10-perylenetetracarboxylic dianhydride, 1,3-bis(3,4-dicarbaxyphenyl)-1,1,3,3-tetramethyldisiloxane dianhydride, 1-(2,3-dicarboxyphenyl)-3-(3,4-dicarboxyphcnyl)-1,1,3,3-tetramethyldisiloxane dianhydride, 1,2,5,6-naphthalenetetracarboxylic dianhydride, 2,3,6,7-naphthalenetetracarboxylic dianhydride, 1,4,5,8-naphthalenetetracarboxylic dianhydride, ethyelnetetracarboxylic dianhydride, butanetetracarboxylic dianhydride, and cyclopentanetetracarboxylic dianhydride.

These tetracarboxylic dianhydrides as illustrated above may be used alone or as a mixture. Further, the hydrogen atoms on the aromatic ring of these tetracarboxylic dianhydrides may be partly or totally substituted by a substituent selected from a fluorine atom, a methyl group, a methoxy group, a trifluoromethyl group, or a trifluoromethoxy group.
In order to introduce a branched structure, part of the tetracarboxylic anhydride may be changed to hexacarboxylic trianhydrides or octacarboxylic tetraanhydrides.

As the diol compounds when diol compounds are used for the polymer of the invention, compound in which, in Formula (17), a1, d1, a2 and d2 each are 0; and b1 and b2 each are 1. or aliphatic diol compounds may be exemplified, and diol compound derivatives recited in Jikken Kagaku Koza (Courses in Experimental Chemistry) (Maruzen Co., Ltd.), New Polymer Experimentology (Kyoritsu Shuppan Co., Ltd.) or the like may also be exemplified.

Among them, compounds, in which, in Formula (17), the encircled Ar1, the encircled Ar2 and the encircled Ar3 each are a benzene ring or a naphthalene ring; R", R"' and R"" each are a halogen atom (-F, -Cl), an alkyl group (a methyl group, a trifluoromethyl group, an ethyl group, a trifluoroethyl group, a propyl group, a butyl group, an isopropyl group, a t-butyl group), an aryl group (a phenyl group, a tolyl group, a mesityl group, a cumenyl group, a chlorophenyl group, a methoxyphenyl group, an ethoxyphenyl group, an acetoxyphenyl group, a benzoyloxyphenyl group), an alkoxy group (a methoxy group, an ethoxy group, an isopropoxy group), an aryloxy group (a phenoxy group), an acyloxy group (an acetoxy group, a propionyloxy group), an acetyl group, a benzoyl group, a benzoyloxy group, or an acylamino group (an acetylamino group, a propionylamino group); Xc is a single bond, -O-, -S-, -SO₂-, an alkylene having 1 to 10 carbon atoms, -NR(C=O)-, -NR(C=O)O-, -NR(C=O)NR' -, -(C=O)- or -(C=O)O-, (here, R and R' each are a hydrogen atom or a methyl group),;c1, c2, and c3 each are 0 to 2; and n1 and n2 each are 0 or 1, or aliphatic diol compounds having 1 to 12 are preferable; compounds derivatives in which, in Formula (17), the encircled Ar1 and Ar2 each are a benzene ring; R" and R"' each are a halogen atom (-F, -Cl), an alkyl group (a methyl group, an ethyl group, an isopropyl group, a t-butyl group), an alkoxy group (a methoxy group, an ethoxy group), an aryloxy group (a phenoxy group), an acetoxy group, an acetyl group or a benzoyloxy group; Xc is a single bond, -O-, -S-, -SO₂-, an alkylene having 1 to 6 carbon atoms, -NR(C=O)-, -NR(C=O)O-, -(C=O)- or -(C=O)O-, (here, R is a hydrogen atom or a methyl group); c1 and c2, each are 0 to 1; n1 is 0 or 1; and n2 is 0, or aliphatic diol compounds having 1 to 8 carbon atoms are more preferable; and diol compound derivatives in which, in Formula (17), the encircled Ar1 and Ar2 each are a benzene ring; Xc is a. single bond, -O-, -SO₂-, an alkylene having 1 to 4 carbon atoms, -NR(C=O)-, -NR(C=O)O-, -(C=O)- or -(C=O)O-, (here, R is a hydrogen atom or a methyl group); c1 and c2, each are 0 to 1; n1 is 0 or 1, and n2 is 0, or aliphatic diol compounds having 1 to 6 carbon atoms are particularly preferable.

Examples of the diol compound according to the invention include, but not limited thereto, the following compounds.
4,4'-dihydroxybiphenyl, 2,2-bis(4-hydroxyphenyl)propane, 2,2-bis(4-hydroxyphenyl)-1-phenylpropane, bis(4-hydroxyphenyl) sulfone, 4,4'-dihydroxybenzophenone, 4,4'-dihydroxybiphenyl ether, 3,3'-dihydroxybiphenyl, 2,2-bis(3-hydroxyphenyl)propane, bis(3-hydroxyphenyl) sulfone, 3,3'-dihydroxybenzophenone, 4,4'-dihydroxy-3,3'-dimethylbiphenyl ether, 4,4'-dihydroxy-3,3'-dimethylbipbenyl, 2,2-bis(4-hydroxy-3-methylphenyl)propane; bis(4-hydroxy-3-methylphenyl) sulfone, 4,4'-dihydroxy-3,3'-dimethylbenzophenone, 4,4'-dihydroxy-3,3'-dichlorobiphenyl, 2,2-bis(4-hydroxy-3-chlorophenyl)propane, bis(4-amino-3-chlorophenyl) sulfone, 4,4'-diamino-3,3'-dichlorobenzophenone, 1,4-dihydroxybenzene, 1,3-dihydroxybenzene, 1,4-dihydroxy-2-methylbenzene, 1,3-dihydroxy-4-methyl-benzene, 1,3-dihydroxy-4-chloro-benzene, 1,3-dihydroxy-4-acetoxy-benzene, 1,3-bishydroxyethyl-benzene, ethylene glycol, diethylene glycol, propylene glycol, dipropylene glycol, butanediol, hexanediol, cyclohexanediol, 1,6-bishydroxymethylcyclohexane, and neopentyl glycol.

As the polymers derived from the acetylene compound of the invention, polybenzimidazole derivatives obtained by condensing the tetraamino compound and phthalic acid in which a hydrogen atom on the benzene ring is unsbstituted or optionally substituted, or the derivatives thereof, with the compound represented by Formula (2) may be exemplified. Among them, polybenzimidazole derivatives obtained by condensing 3,3',4.4'-tetraaminobiphenyl in which a hydrogen atom on the benzene ring is unsubstituted or optionally substituted, and phthalic acid in which a hydrogen atom on the benzene ring is unsbstituted or optionally substituted, or the derivative thereof with the compound represented by Formula (2), are preferred.

Further, as the polymers derived from the acetylene compound of the invention, polybezoxazole derivatives obtained by condensing the bis(orthohydroxyamino) compound and phthalic acid, in which a hydrogen atom on the benzene ring is unsbstituted or optionally substituted, or the derivative thereof with the compound represented by Formula (2) may be exemplified. Among them, polybenzoxazole derivatives obtained by condensing polybenzoxazole derivatives obtained by condensing 3,3'-diamino-4,4'-dihydroxybiphenyl, in which a hydrogen atom on the benzene ring is unsubstituted or optionally substituted, and phthalic acid, in which a hydrogen atom on the benzene ring is unsbstituted or optionally substituted, or the derivative thereof with the compound represented by Formula (2), are preferred.

Further, as the polymers derived from the acetylene compound of the invention, polyamide derivatives obtained by condensing the diamine compound and phthalic acid, in which a hydrogen atom on the benzene ring is unsbstituted or optionally substituted, and the derivative thereof with the compound represented by Formula (2) may be exemplified. Among them, polyamide derivatives obtained by condensing diaminobenzene, in which a hydrogen atom on the benzene ring is unsubstituted or optionally substituted, and phthalic acid, in which a hydrogen atom on the benzene ring is unsbstituted or optionally substituted or the derivatives thereof, with the compound represented by Formula (2), are preferred.

Furthermore, as the polymers derived from the acetylene compound of the invention, polyamideimide derivatives obtained by condensing the diamine compound and phthalic acid in which a hydrogen atom on the benzene ring is unsbstituted or optionally substituted or the derivative thereof, a tetracarboxylic acid dianhydride in which a hydrogen atom on the benzene ring is unsbstituted or optionally substituted or the derivative thereof, with the compound represented by Formula (2) may be exemplified. Among them, polyamideimide derivatives obtained by condensing diaminobenzene in which a hydrogen atom on the benzene ring is unsubstituted or optionally substituted, and phthalic acid, in which a hydrogen atom on the benzene ring is unsbstituted or optionally substituted or the derivative thereof, and tetracarboxylic acid dianhydride or the derivatives thereof, with the compound represented by Formula (2), are preferred.

Moreover, as the polymers derived from the acetylene compound of the invention, polyester derivatives obtained by condensing the diol compound and phthalic acid, in which a hydrogen atom on the benzene ring is unsubstituted or optionally substituted, or the derivative thereof, with the compound represented by Formula (2) may be exemplified. Among them, polyester derivatives obtained by condensing diols having a cyclic or non-cyclic hydrocarbon group which may be optionally substituted, and phthalic acid, in which a hydrogen atom on the benzene ring is unsbstituted or optionally substituted, or the derivative thereof with the compound represented by Formula (2), are preferred.

Among them, as the polymers derived from the acetylene compound of the invention, in particular, the polybenzimidazole derivatives and the polybenzoxazole derivatives are preferred. These may be a homopolymer, or may be a hybrid polymer compound formed by block copolymerizing at least two kind of monomers.

A hydrogen atom on the benzene ring of 3,3',4,4'-tetraaminobiphenyl, 3,3'-diamino-4,4'-dihydroxybiphenyl, diaminobenzene, phthalic acid or the derivatives thereof, in which a hydrogen atom on the benzene ring is unsubstituted or optionally substituted, which are the raw materials of the polybenzimidazole derivatives, the polybenzoxazole derivatives and the polyamide derivatives may be unsubstituted, or optionally substituted, but is particularly preferably unsubstituted.

The hydrocarbon groups of the diols having an optionally substituted cyclic or non-cyclic hydrocarbon group as raw materials for the polyester derivatives are preferably cyclic or non-cyclic saturated hydrocarbons, more preferably straight chaine saturated hydrocarbons, and even more preferably unsbstituted and have 2 to 6 carbon atoms.

As the method of preparing and deriving the polymer containing the acetylene compound of the invention as a constitutional unit, when an acetylene compound as recited in any of <1> to <12> above is allowed to react with a compound represented by Formula (17), a method, in which these compounds are converted into an intermediate having a high activity to the condensation reaction, and thereafter, are reacted, or an acetylene compound as recited in any of <1> to <12> is directly condensed with or added to the compound represented by Formula (17), may be exemplified.

Although the method of manufacturing the polymer of the invention is not specifically restricted, the polymer of the invention can be prepared by using the acetylene compound and the monomer or the mixture of the monomers.
For example, as the method of manufacturing a polyamide-based polymer according to the invention, known polymerization methods such as a method, in which an acid anhydride is dispersed in an organic solvent in which an amine compound such as a diamine or the like is dissolved, and the dispersion is stirred, so that the acid anhydride is completely dissolved, thereby polymerizing the amine compound, a method, in which after an acid anhydride is dissolved and/or dispersed in an organic solvent, polymerization is performed by using an amine compound, or a method, in which polymerization is performed by reacting a mixture of an acid anhydride and an amine compound in an organic solvent, may be used.
When water is generated in oxazole-cyclization, imidazole-cyclization, esterification, amidation, imidation or the like, it is preferable that the water be subjected to azeotropic distillation to be removed from the reaction system to accelerate the reaction, and, further, the reaction can easily proceed by using a dehydration agent such as an aliphatic acid anhydride such as acetic anhydride, or an aromatic acid anhydride.

Further, the reaction can also be completed rapidly by adding a polycondensation accelerator to the reaction system, if needed. As such a polycondensation accelerator catalyst, a basic polycondensation accelerator and an acidic polycondensation accelerator may be exemplified, and both accelerators may also be used together. Examples of the basic polycondensation accelerator include N,N-dimethylaniline, N,N-diethylaniline, pyridine, quinoline, isoquinoline, α-picoline, β-picoline, γ-picoline, 2,4-lutidine, triethylamine, tributylamine, tripentylamine, N-methylmorpholine, diazabicycloundecene, diazacyclononene and the like. Among them, from the viewpoints of the availability, reaction accelerating property and the like, diazabicycloundecene, diazacyclononene, methylpyridine, pyridine and trimethylamine are preferred, and pyridine and trimethyl amine are more preferred. Examples of the acidic polycondensation accelerator include benzoic acid and o-hydroxybenzoic acid, m-hydroxybenzoic acid, p-hydroxybenzoic acid, 2,4-dihydroxybenzoic acid, p-hydroxyphenyl acetic acid, 4-hydroxyphenyl propionic acid, phosphoric acid, p-phenol sulfonic acrid, p-toluenesulfonic acid, crotonic acid and the like.

The use amount of the polycondensation accelerator is from 1 to 50% by mole, and preferably from 5 to 35% by mole with respect to alcohol or an amine compound component, Since the reaction temperature can be set to a low temperature by the use of these polycondensation accelerators, the side reaction which is considered as a cause of coloration can be prevented, and the reaction time can also be greatly reduced, thereby becoming economical. It is preferable that the polycondensation temperature be 60°C or less, and further, it is preferable that the temperature be 40°C or less, from the viewpoints of effectively promoting the reaction and increasing the viscosity of the reaction system.

Examples of solvents to be used for manufacturing the polymer include, for example, ureas such as tetramethyl urea or N,N-dimethyl urea; sulfoxides or sulfones such as dimethyl sulfoxide, diphenyl sulfone or tetramethyl sulfone; amides such as N,N-dimethyl acetamide (DMAc), N,N-dimethyl formamide (DMF), N,N'-diethyl acetamide, N-methyl-2-pyrrolidone (NMP), γ-butyllactone or hexamethylphosphoric triamide; or aprotic solvents such as phophoryl amides; alkyl halides such as chloroform or methylene chloride; aromatic hydrocarbons such as benzene and toluene; phenols such as phenol or cresol; ethers such as dimethyl ether, diethyl ether or p-cresol methyl ether, and the like. In general, these solvents are used singly, but two or more kinds of solvents may be used in combination, as needed. Among these solvents, amides such as DMF, DMAc or NMP are preferably used.

Among these manufacturing methods, from the viewpoints of the reactivity and prevention of decomposition or reaction of the target ethynyl group, a method, in which these compound are reacted after these compounds are converted into an intermediate having a high activity to the condensation reaction, is preferred. For example, a carboxylic acid is beforehand converted into -COL (here, L is a monovalent heaving group and may be any group as long as the group can be replaced by a nitrogen atom or an oxygen atom by the reaction with an amino group or a hydroxyl group, is preferably a halogen atom (for example, fluorine, chlorine, bromine or iodine), a sulfonate group (for example, mesylate, tosylate or triflate), a methanesulfonyl group, an alkoxycarbonyl group, a diszonium group, a trialkyl ammonium group (for example, trimethyl ammonium), more preferably a halogen atom, a methanesulfonyl group, a sulfonate group and an alkoxycarbonyl group, and even more preferably a halogen atom or a methanesulfonyl group), and thereafter, COL is allowed to react with a compound having an amino group or a hydroxyl group, so that the reaction temperature can be set to a low temperature, and the condensation reaction time can be shorten, and this is preferred.

The polymer containing the acetylene compound of the invention as a constitutional unit can be suitably manufactured with reference to the methods recited in New Polymer Experimentology (edited by The Society of Polymer Science, Japan; published by Kyoritsu Shuppan Co., Ltd.), Volume 28 of Jikken Kagaku Koza (Courses in Experimental Chemistry) (edited by the Chemical Society of Japan; published by Maruzen Co., Ltd.), and the like.

Although the molecular weight of the polymer containing the acetylene compound of the invention as a constitutional unit is not specifically restricted, the weight average molecular weight is from 300 to 1,000,000, preferably from 500 to 200,000, and even more preferably from 1,000 to 50,000, from the viewpoints of handling property and curability. The polymer having a molecular weight of 10,000 may be referred to as an oligomer.
Hereinafter, specific examples of polymers derived from the acetylene compound are shown, but the invention is not limited to these examples.

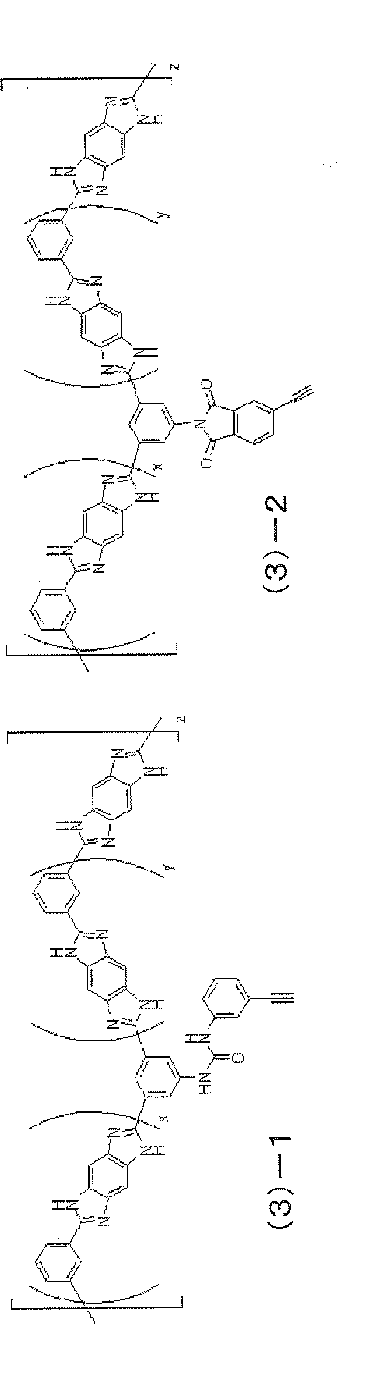

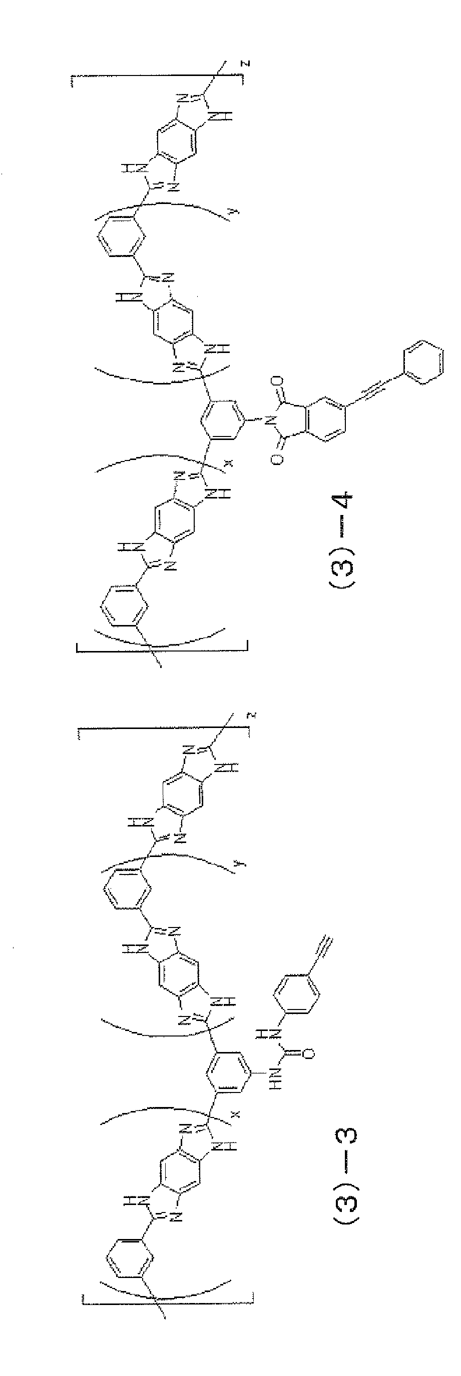

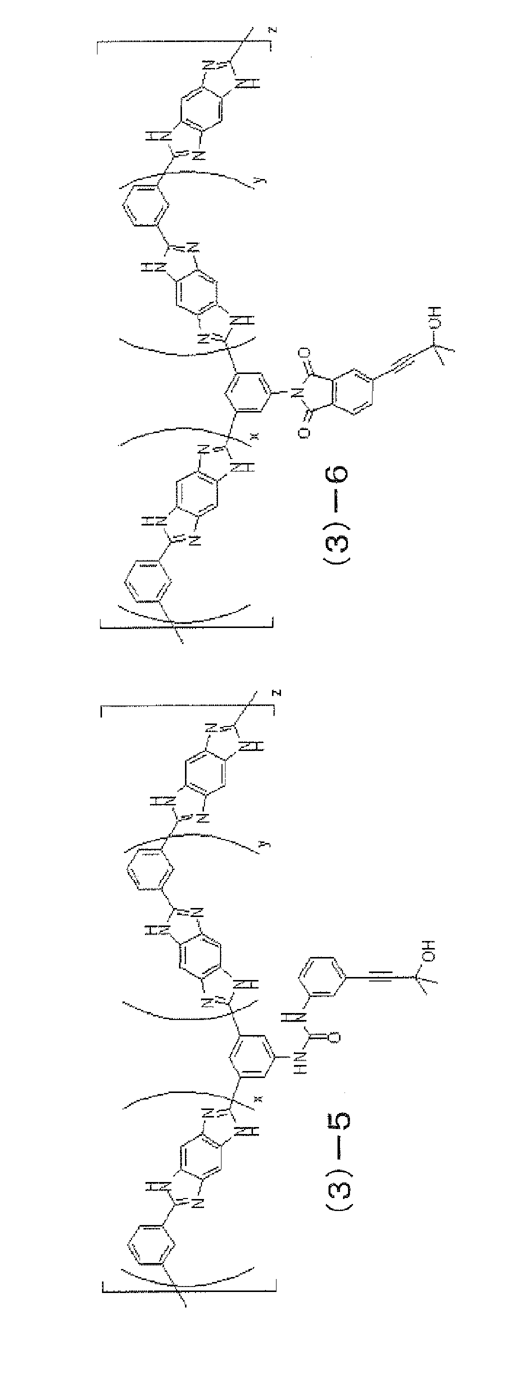

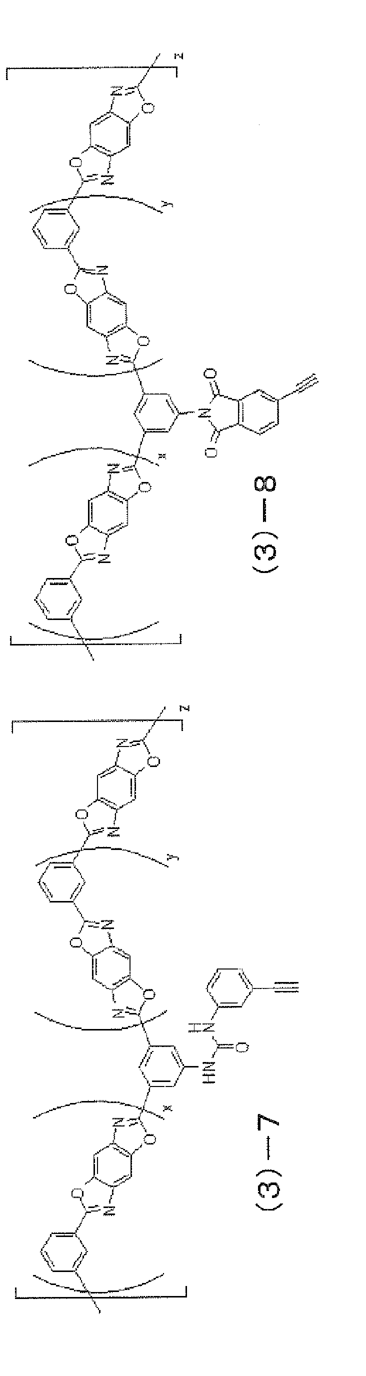

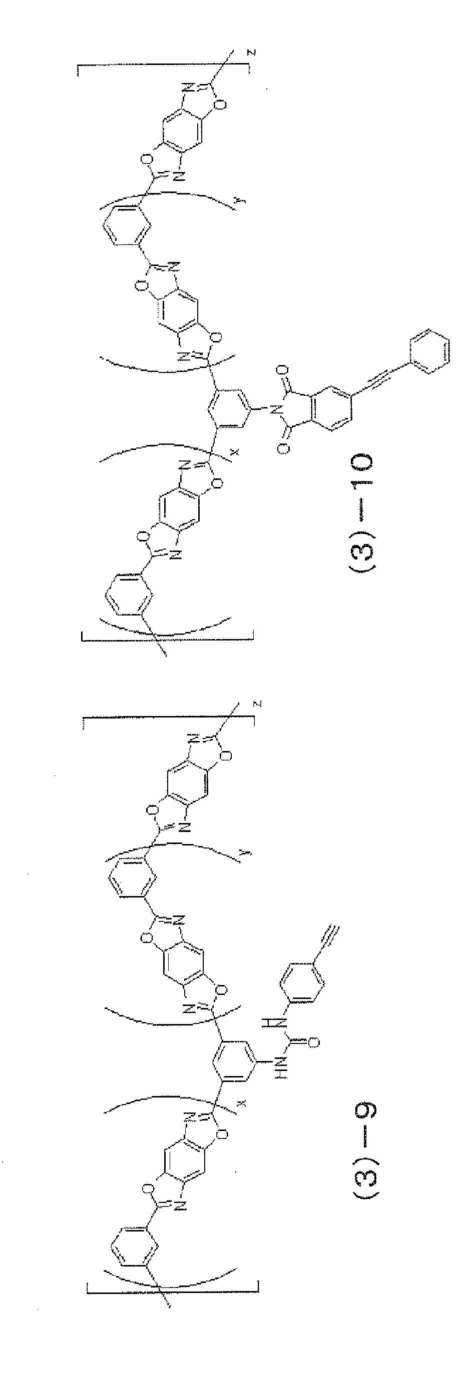

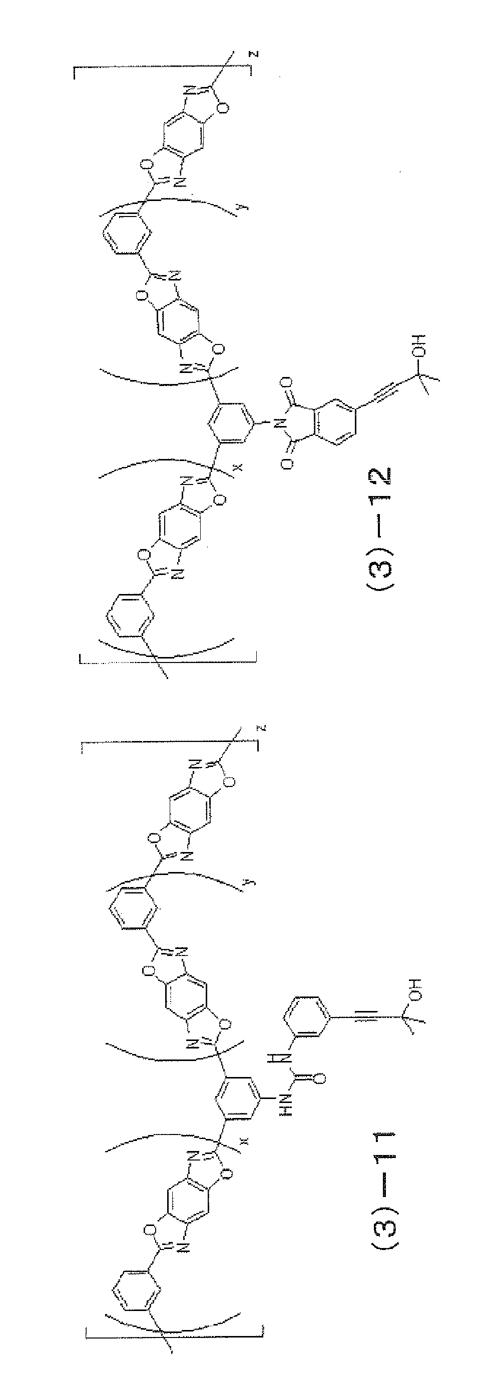

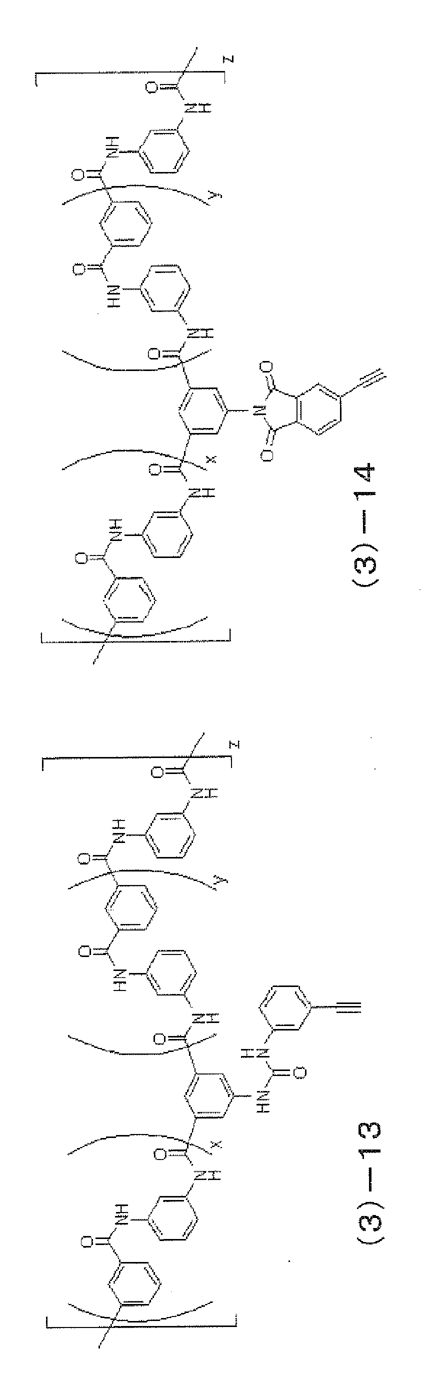

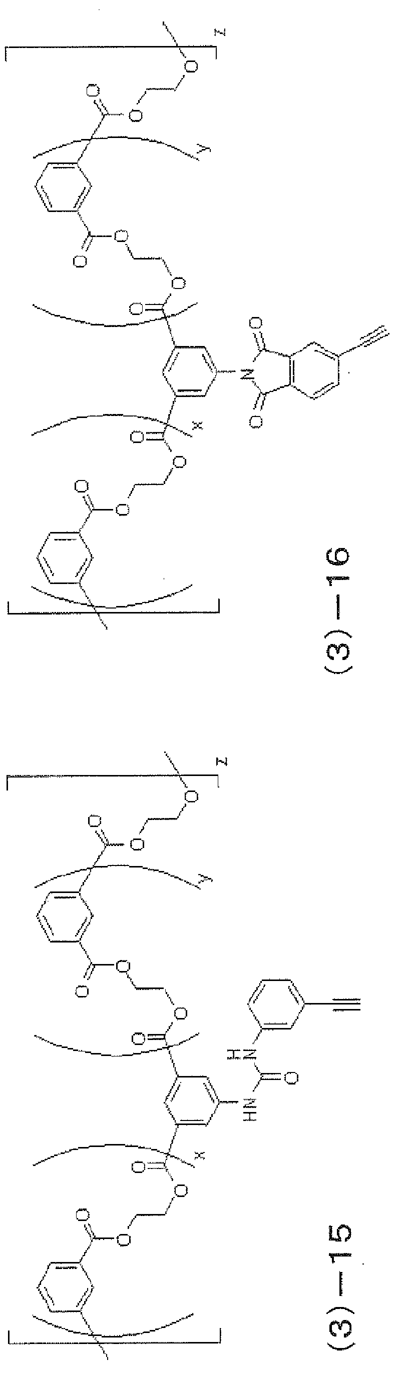

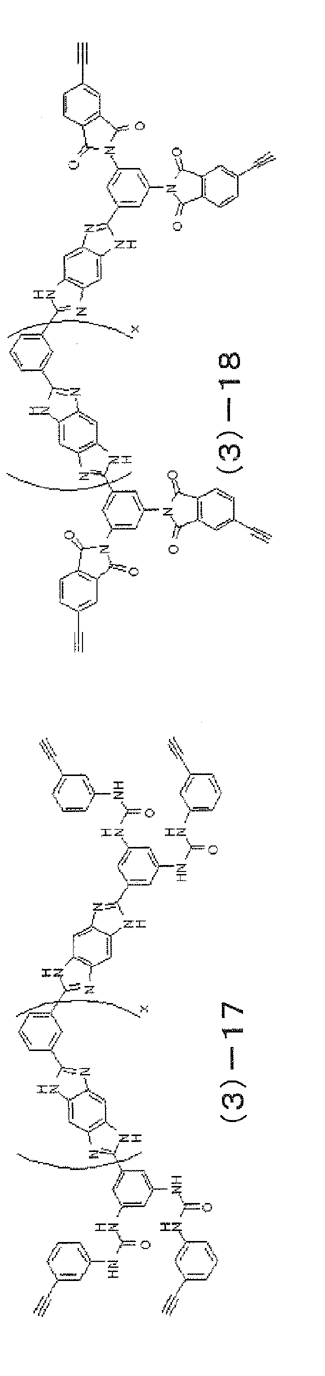

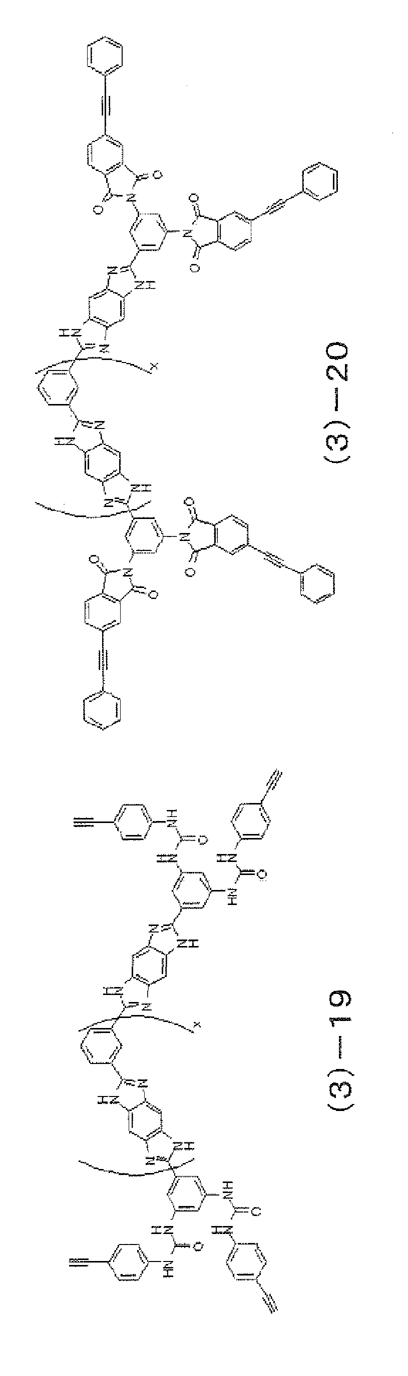

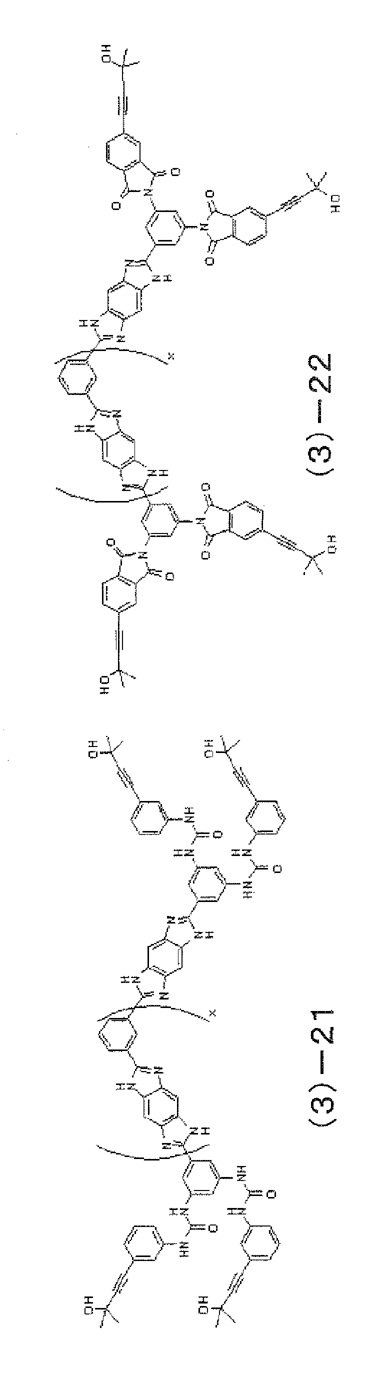

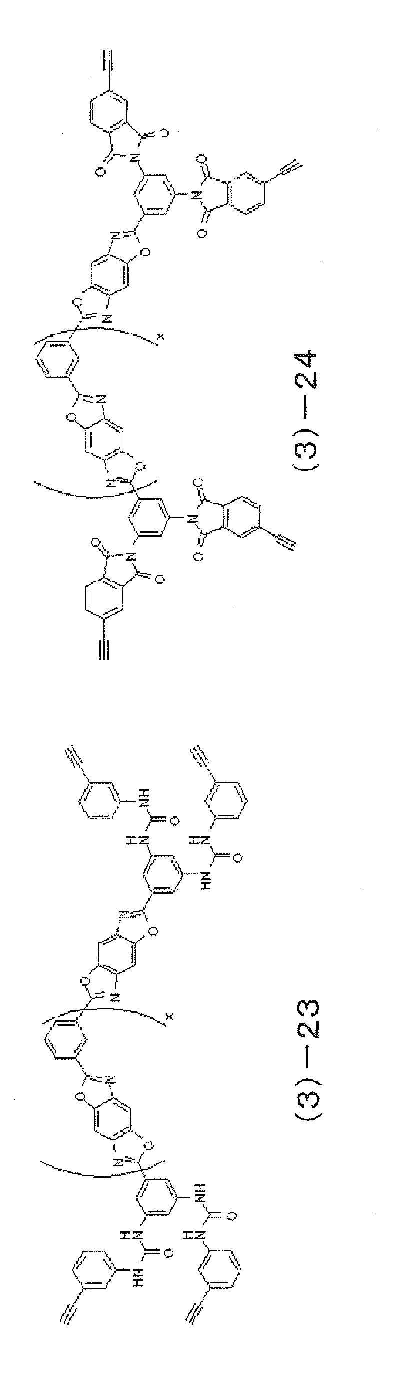

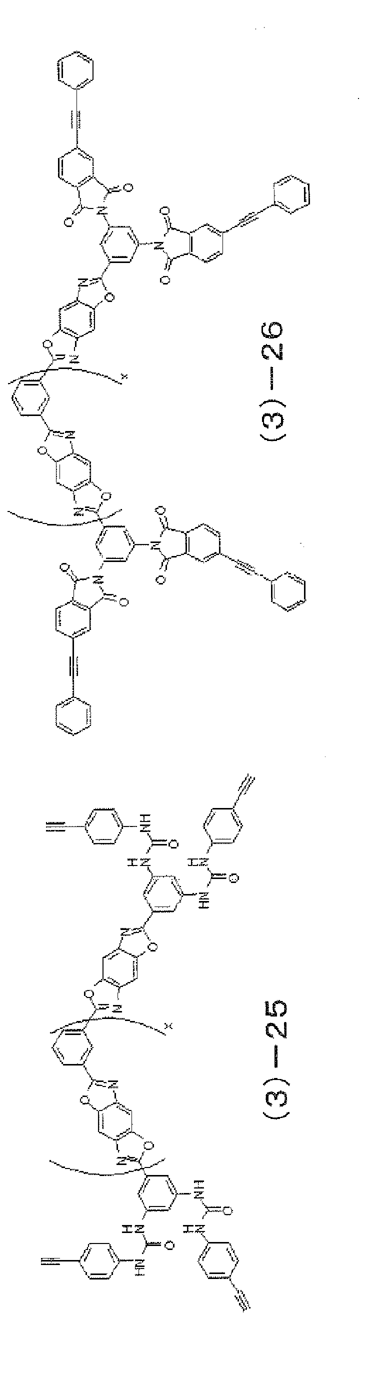

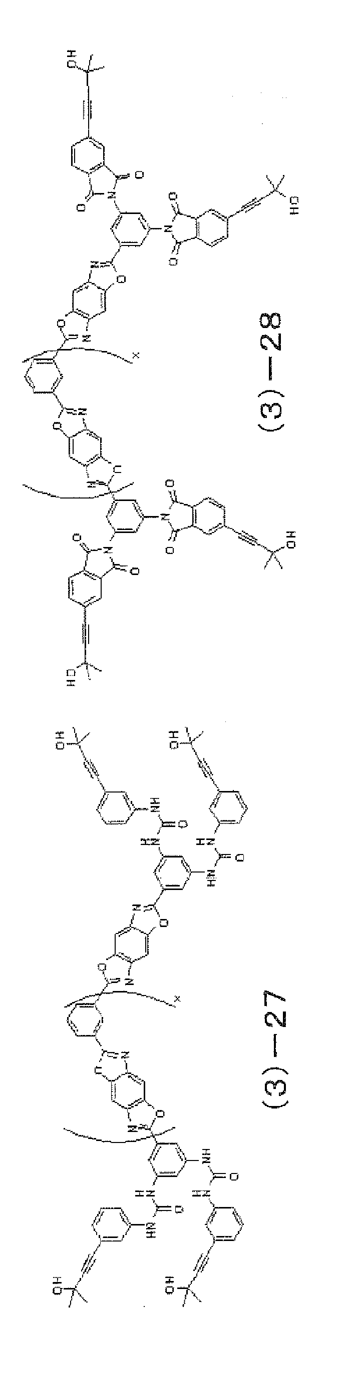

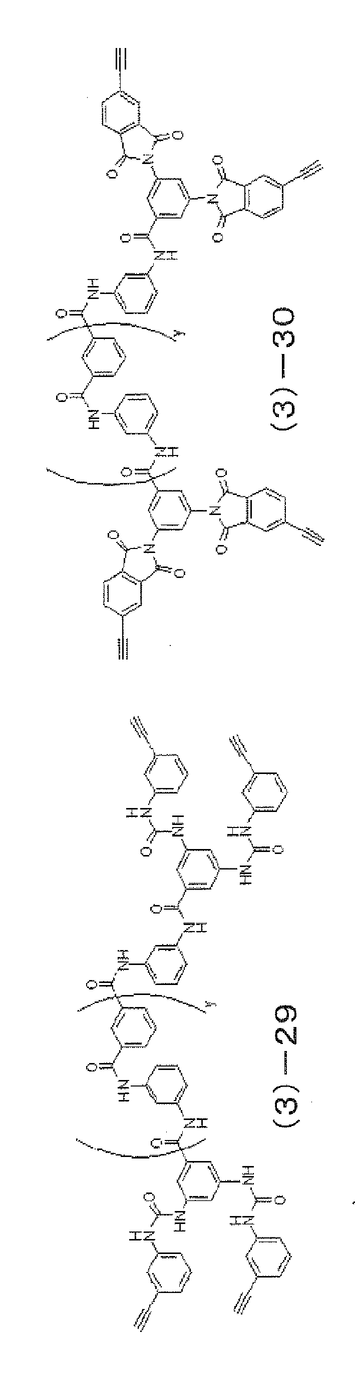

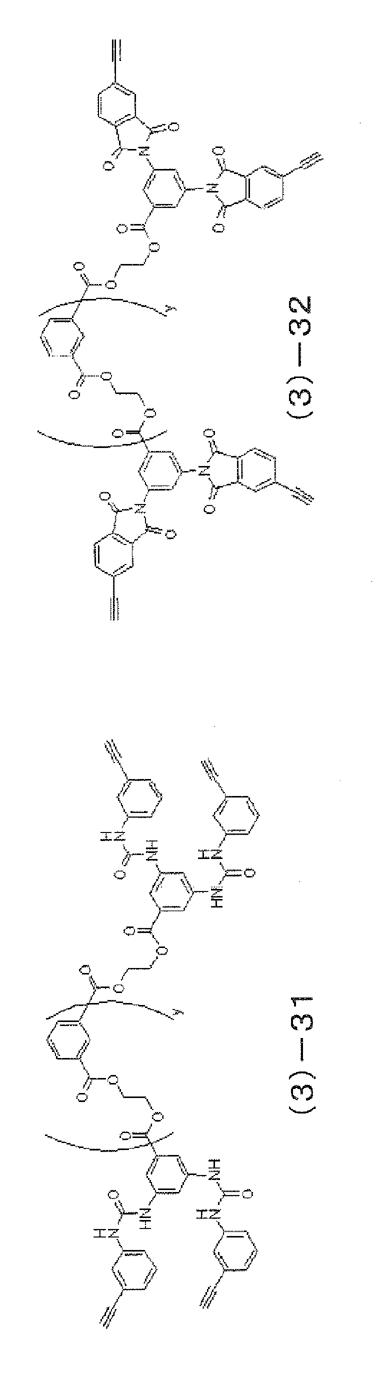

### <Explanation of manufacturing method of acetylene compound>

Next, the manufacturing method of the acetylene compound represented by Formula (1) is explained.

### Explanation of Formula (6)

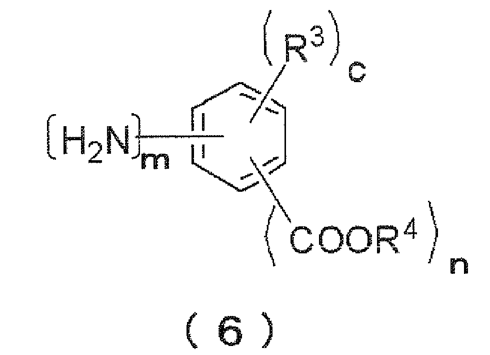

In Formula (6), the meanings of R³, c, m and n each are the same as those of R³, c, m and n in Formula (3), and preferable ranges thereof are also the same as those of R³, c, m and n in Formula (3). R⁴ represents a cyclic or non-cyclic hydrocarbon group or heterocyclic group which may be unsubstituted or optionally substituted. Examples of the unsbstituted hydrocarbon group include an alkyl group having 1 to 20 carbon atoms (for example, methyl, butyl, octyl, hexadecyl, cyclohexyl or the like), an aryl group having 1 to 20 carbon atoms (for example, phenyl, naphthyl, anthracene or the like), and examples of the optionally substituted hydrocarbon group include hydrocarbon groups substituted with a halogen atom (for example, a fluorine atom, a chlorine atom, a bromine atom or an iodine atom), a cyano group, a nitro group, an alkoxy group having 1 to 20 carbon atoms (for example, methoxy, butoxy, dodecyloxy, or the like), an aryl group such as phenyl or naphthyl, a hydroxyl group and the like. Examples of the heterocyclic group include, for example, pyridine, quinoline, pyrrole, furan, thiophene and the like. Among them, substituted or unsbstituted hydrocarbon groups are preferable, and unsubstituted hydrocarbon groups are more preferable. Specific examples of the compounds represented by Formula (6) include, for example, 4-aminophthalic acid, 5-aminoisophthalic acid, 4-aminoterephthalic acid, 4-amino-5-methylphthalic acid, 5-amino-2-methylisophthalic acid, 5-amino-2-chloroisophthalic acid, 5-amino-4,6-dimethyl-isophthalic acid, dimethyl 4-aminophthalate, dimethyl 5-amino isophthalate, dimethyl 4-aminoterephthalate, dimethyl 4-amino-5-methylphthalate, dimethyl 5-amino-2-methylisophthalate, dimethyl 5-amino-2-chloroisophthalate, dimethyl 5-amino-4,6-dimethylisophthalate, diethyl 5-aminoisophthalate, diphenyl 5-aminoisophthalate, dicyclohexyl 5-aminoisophthalate, diaminobenzoic acids (for example, 3,5-diaminobenzoic acid, 3,4-diaminobenzoic acid, 3,5-diaminobenzoic acid dihydrate, 3,4-diaminobenzoic acid dihydrate, 2-methyl-3,5-diaminobenzoic acid, 2,6-dimethyl-3,5-diaminobenzoic acid, 2-phenyl-3,5-diaminobenzoic acid, 4-fluoro-3,5-diaminobenzoic acid, 3,5-diaminobenzoic acid hydrochloride, 3,4-diamino benzoic acid hydrochloride, 2-methyl-3,5-diaminobenzoic acid methanesulfonate, 2,6-dimethyl-3,5-diaminobenzoic acid oxalate, and 2-phenyl-3,5-diaminobenzoic acid sulfate, but are not limited to these compounds. Among them, from the viewpoints of the availability, of raw materials and the reactivity, 5-aminoisophthalic acid, dimethyl 5-amino-isophthalate, diethyl 5-aminoisophthalate, diphenyl 5-aminoisophthalate, dicyclohexyl 5-aminoisophthalate, 3,5-diaminobenzoic acid and 3,4-diaminobenzoic acid are desirable, and dimethyl 5-aminoisophthalate, diethyl 5-aminoisophthalate, 5-aminoisophthalic acid and 3,5-diaminobenzoic acid are particularly desirable.

### Explanation of Formula (7)

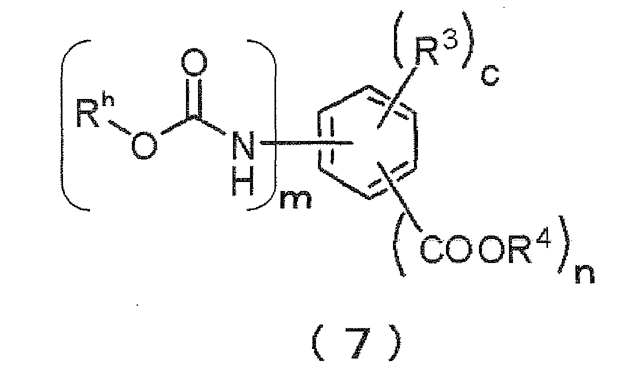

In Formula (7), the meanings of R³ c, m and n each are the same as those of R³ c, m and n in Formula (3), and the preferable ranges are also the same as those of R³ c, m and n in Formula (3). R^{h} has the same definition as that of R in Formula (1). R^{h} represents a cyclic or non-cyclic hydrocarbon group which may be optionally substituted, represents preferably a cyclic or non-cyclic hydrocarbon group having 1 to 10 carbon atoms which may be optionally substituted, and represents more preferably a cyclic or non-cyclic hydrocarbon group having 6 to 10 carbon atoms which may be optionally substituted, and represents particularly preferably a cyclohexyl group, a phenyl group or a tolyl group.

Specific examples of the compound represented by Formula (7) include, for example, 4-phenoxycarbonyl aminophthalic acid, 5- phenoxycarbonyl aminoisophthalic acid, 4-phenoxycarbonyl aminoterephthalic acid, 4-phenoxycarbonylamino-5-methylphthalic acid, 5-phenoxycarbonylamino-2-methylisophthalic acid, 5-phenoxycarbonylamino-2-chloroisophthalic acid, 5-phenoxycarbonylamino-4,6-dimethyl-isophthalic acid, dimethyl 4-tolyloxycarbonylamino phthalate, dimethyl 5-cyclohexyloxycarbonylamino isophthalate, dimethyl 5-tolyloxycarbonylaminoisophthalate, dimethyl 5-phenoxycarbonylaminoisophthalate, dimethyl 5-p-chlorophenoxycarbonylaiminoisophthalate, dimethyl 4-phenoxycarbonylaminoterephthalate, dimethyl 4-phenoxycarbonylamino-5-methylphthalate, dimethyl 5-phenoxycarbonylamino-2-methylisophthalate, dimethyl 5-phenoxycarbo-nylamino-2-chloroisophthalate, dimethyl 5-phenoxycarbonylamino-4,6-dimethylisophthalate, diethyl 5-phenoxycarbonylaminoisophthalate, diphenyl 5-phenoxycarbonylaminoisophthalate, dicyclohexyl 5-cyclohexylaxycarbonylaminoisophthalate, dicyclohexyl 5-phenoxycarbonylaminoisophthalate, diaminobenzoic acids (for example, 3,5-bis(phenoxycarbonylamino)benzoic acid, 3,4-bis(phenoxycarbonylamino)benzoic acid, methyl 3,5-bis(phenoxycarbonylamino) benzoate, methyl 3,4-bis(phenoxycarbonylamino)benzoate, 2-methyl-3,5-bis(phenoxycarbonylamino)benzoic acid, 2,6-dimethyl-3,5-bis(phenoxycarbonylamino)benzoic acid, 2-phenyl-3,5-bis(phenoxycarbonylamino)benzoic acid, 4-fluoro-3,5-bis(phenoxycarbonylamino)benzoic acid, 3,5-bis(tolyloxycarbonylamino)benzoic acid, methyl 3,5-bis(tolyloxycarbonylamino)benzoate, 3,5-bis(cyclohexyloxycarbonylamino) benzoic acid, ethyl 3,5-bis(cyclohexyloxycarbonylamino)benzoate, 3,5-bis(p-chlorophenoxycarbonylamino)benzoic acid, 3,4-bis(cyclohexyloxycarbonylamino)benzoic acid, and the like, but are not limited to these compounds.

In view of the availability of raw materials, the reactivity and the like, 5-phenoxycarbonylaminoisophthalic acid, dimethyl 5-phenoxycarbonylaminoisophthalate, diethyl 5-phenoxycarbonylaminoisophthalate, diphenyl 5-phenoxycarbonylaminoisophthalate, dicyclohexyl 5-phenoxycarbonylaminoisophthalate, 3,5-bis(phenoxycarbonylamino)benzoic acid, methyl 3,5-bis(phenoxycarbonylamino)benzoate, methyl 3,4-bis(phenoxycarbonylamino)benzoate, methyl 3,5-bis(tolyloxycarbonylamino)benzoate, are preferable, and dimethyl 5-phenoxycarbonylaminoisophthalate, diethyl 5-phenoxycarbonylaminoisophthalate, methyl 3,5-bis(phenoxycarbonylamino)benzoate and methyl 3,4-bis(phenoxycarbonylamino)benzoate are particularly preferable.

Explanation of Formula (8)

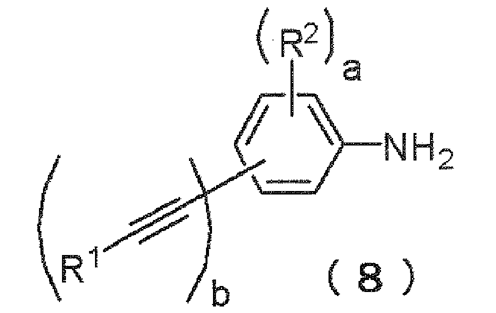

In Formula (8), the meanings of R¹, R², a and b each are the same as those of R¹, R², a and b in Formula (3), and the preferable ranges are also the same as those of R¹, R², a and b in Formula (3). Specific examples of compounds represented by Formula (8) include, for example, m-ethynylaniline, p-ethynylaniline, o-ethynylaniline, 2,3-diethynylaniline, 3,4-diethynylaniline, 3,5-diethynylaniline, 3,6-diethynylaniline, m-propynylaniline, m-butynylaniline, m-hexynylaniline, m-dodecylethynylaniline, m-t-butylethynyianiline, m-cyclohexylethynylaniline, m-phenylethynylaniline, m-3-pyridylethynylaniline, m-2-pyridylethynylaniline, m-naphthylethynylaniline, m-quinolinylethynylaniline, m-2-hydroxypropyl-2-ethynylaniline, m-trimethylsilylethynylaniline, m-ethynyltoluidine, p-ethynyltoluidine, o-ethynyl-p-chloroaniline, 2,3-diethynyl-5-methylaniline, 3,4-diethynyltoluidine, 3,5-diethynyltoluidine, 4-chloro-3,6-diethynyianiline, m-propynyltoluidine, m-butynyltoluidine, m-hexynyltoluidine, 3-dodecylethynyl-5-methoxyaniline, 3-t-butylethynyl-5-chloroaniline, 3-cyclohexylethynyl-5-chloroaniline, 3-phenylethynyltoluidine, m-2-hydroxypropyl-2-ethynyltoluidine, m-trimethylsilyl ethynyltoluidine and the like.

In view of the availability of raw materials, the reactivity and the like, m-ethynylaniline, p-ethynylaniline, o-ethynylaniline, 2,3-diethynylaniline, 3,4-diethynylaniline, 3,5-diethynylaniline, 3,6-diethynylaniline, m-propynylaniline, m-hexynylaniline, m-t-butylethynylaniline, m-cyclohexylethynylaniline, m-phenylethynylaniline, m-3-pyridylethynylaniline, m-trimethylsilylethynylaniline, m-ethynyltoluidine and the like are preferable, and m-ethynylaniline, p-ethynylaniline, 3,4-diethynylaniline, 3,5-diethynylaniline, m-propynylaniline and m-cyclohexyl ethynylaniline are particularly preferable.

Explanation of Formula (9)

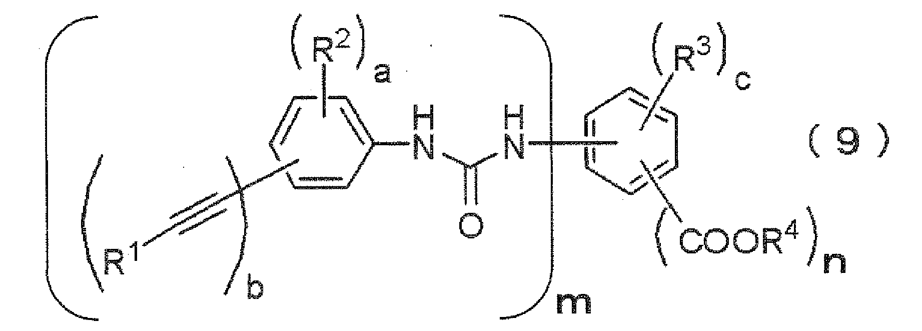

The compounds represented by Formula (9) are the reaction products resulting from the compound represented by Formula (7) and the compound represented by Formula (8), and in Formula (9), the meanings of R¹, R², R³, a, b, c, m and n each are the same as those of R¹, R², R³, a, b, c, m and n in Formula (3), and the preferable ranges are also the same as those of R¹, R², R³, a, b, c, m and n in Formula (3). R is the same as that of R in Formula (1), and the desirable range is also the same as that of R in Formula (1).

Explanation of Formula (10)

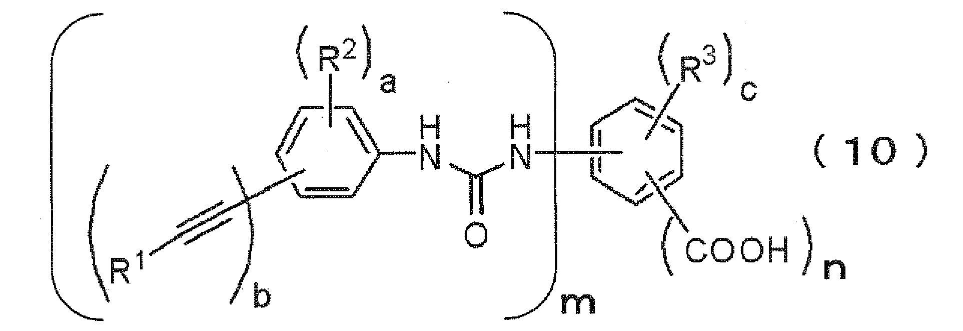

In Formula (10), the meanings of R¹, R², R³, a; b, c, m and n each are the same as those of R¹, R², R³, a, b, c, m and n in Formula (3), and the preferable range thereof are also the same as those of R¹, R², R³, a, b, c, m and n in Formula (3).

Explanation of Formula (11)

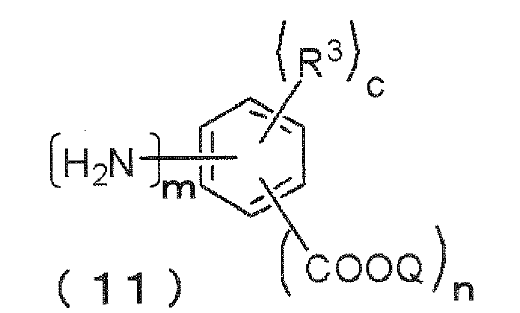

In Formula (11), the meanings of R³, Q, c, m and n each are the same as those of R³, Q, c, m and n in Formula (4), and the preferable range thereof are also the same as those of R³, Q, c, m and n in Formula (4). Specific examples of the compounds represented by Formula (11) include, for example, 4-aminophthalic acid, 5-aminoisophthalic acid, 4-aminoterephthalic acid, 4-amino-5-methylphthalic acid, 5-amino-2-methylisophthalic acid, 5-amino-2-chloroisophthalic acid, 5-amino-4,6-dimethyl-isophthalic acid, sodium 4-aminophthalate, sodium 5-aminoisophthalate, sodium 4-aminoterephthalate, sodium 4-amino-5-methylphthalate, sodium 5-amino-2-methylisophthalate, sodium 5-amino-2-chloroisophthalate, sodium 5-amino-4,6-dimethyl-isophthalate, potassium 4-aminophthalate, potassium 5-aminoisophthalate, potassium 4-aminoterephthalate, potassium 4-amino-5-methylphthalate, triethylammonium 4-aminophthalate, triethylammonium 5-aminoisophthalate, triethylammonium 4-aminoterephthalate, triethylammonium 4-amino-5-methylphthalate, triethylammonium 5-amino-2-methylisophthalate, tributylammonium 4-aminophthalate, tributylammonium 5-aminoisophthalate, tributylammonium 4-aminoterephthalate, dimethyl 4-aminophthalate, dimethyl 5-aminoisophthalate, dimethyl 4-aminoterephthalate, dimethyl 4-amino-5-methylphthalate, dimethyl 5-amino-2-methylisophthalate, dimethyl 5-amino-2-chloroisophthalate, dimethyl 5-amino-4,6-dimethylisophthalate, diethyl 5-aminoisophthalate, diphenyl 5-aminoisophthalate, dicyclohexyl 5-aminoisophthalate, diaminobenzoic acids (for example, 3,5-diaminobenzoic acid, 3,4-diaminobenzoic acid, 3,5-diaminobenzoic acid dihydrate, 3,4-diaminobenzoic acid dihydrate, 2-methyl-3,5-diaminobenzoic acid, 2,6-dimethyl-3,5-diaminobenzoic acid, 2-phenyl-3,5-diaminobenzoic acid, 4-fiuoro-3,5-diaminobenzoic acid, methyl 3,5-diaminobenzoate, ethyl 3,5-diaminobenzoate, sodium 3,5-diaminobenzoate, potassium 3,5-diaminobenzoate, triethylammonium 3,5-diaminobenzoate, sodium 3,4-diaminobenzoate, potassium 3,4-diaminobenzoate, triethylammonium 3,4-diaminobenzoate, potassium 2-methyl-3,5-diaminobenzoate, potassium 2,6-dimethyl-3,5-diaminobenzoate, triethylammonium 2,6-dimethyl-3,5-diaminobenzoate, potassium 2-phenyl-3.5-diaininobenzoate, triethylammonium 2-phenyl-3,5-diaminobenzoate, and the like, but are not limited to these compounds.

In view of the availability of raw materials, the reactivity and the like, 4-aminophthalic acid, 5-aminoisophthalic acid, 4-aminoterephthalic acid, 4-amino-5-methylphthalic acid, 5-amino-2-methylisophthalic acid, potassium 4-aminophthalate, potassium 5-aminoisophthalate, potassium 4-aminoterephthalate, triethylammonium 4-aminophthalate, triethylammonium 5-aminoisophthalate, triethylammonium 4-aminoterephthalate, triethylammonium 4-amino-5-methylphthalate, tributylammonium 4-aminophthalate, tributylammonium 5-aminoisophthalate, tributylammonium 4-aminoterephthalate, dimethyl 5-aminoisophthalate and the like are preferable, and 5-aminoisophthalic acid, triethylammonium 4-aminophthalate, triethylammonium 5-aminoisophthalate, tributylammonium 4-aminophthalate, tributylammonium 5-aminoisophthalate, dimethyl 5-aminoisophthalate, 3,5-diaminobenzoic acid, methyl 3,5-diaminobenzoate, triethylammonium 3,5-diaminobenzoate and triethylammonium 3,4-diammobenzoate are particularly preferable.

Explanation of Formula (12)

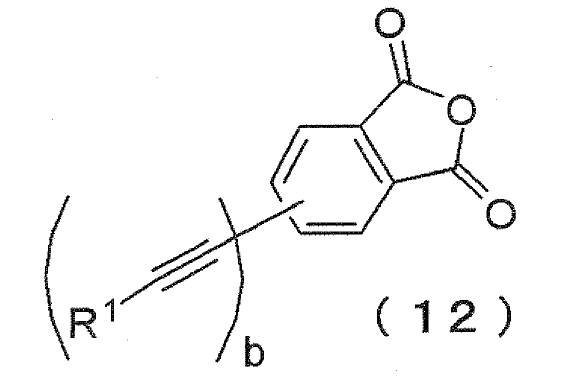

In Formula (12), the meanings of R¹ and b each are the same as those of R¹ and b in Formula (4), and the preferable ranges thereof are also the same as those of R¹ and b in Formula (4). Specific examples of the compound represented by Formula (12) include, for example, 4-ethynylphthalic anhydride, 3,4-diethynylphthalic anhydride, 3-propynylphthalic anhydride, 3-butynylphthalic anhydride, 3-hexynylphthalic anhydride, 3-dodecylethynylphthalic anhydride, 3-t-butylethynylphthalic anhydride, 3-cyclohexylethynylphthalic anhydride, 3-phenylethynylphthalic anhydride, 3-pyridylethynylphthalic anhydride, 3-naphthylethynylphthalic anhydride, 3-quinolinylethynylphthalic anhydride, 3-(2-hydroxypropyl-2-ethynyl)phthalic anhydride, 3-trimethylsilylethynylphthalic anhydride, 3-ethynyl-4-methylphthalic anhydride, 3-ethynyl-4-chlorophthalic anhydride, 3-ethynyl-4-methoxyphthalic anhydride and the like.

In view of the availability of raw materials, the reactivity and the like, 4-ethynylphthalic anhydride, 3-propynylphthalic anhydride, 3-butynylphthalic anhydride, 3-hexynylphthalic anhydride, 3-t-butylethynylphthalic anhydride, 3-cyclohexylethynylphthalic anhydride, 3-phenylethynylphthalic anhydride, 3-(2-hydroxypropyl-2-ethynyl)phthalic anhydride, and the like are preferable, and 4-ethynylphthalic anhydride, 3-phenylethynylphthalic anhydride and 3-(2-hydroxypropyl-2-ethynyl)phthalic anhydride are particularly preferable.

Explanation of Formula (13)

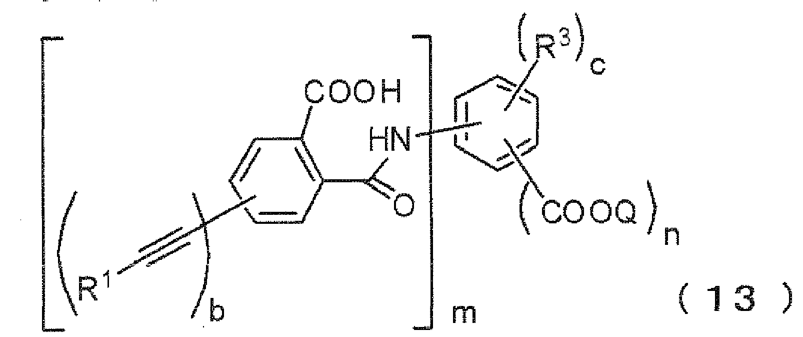

The compounds represented by Formula (13) are the reaction products resulting from the compound represented by Formula (11) and the compound represented by Formula (12), and in Formula (13), the meanings of R¹, R³, b, c, m and n each are the same as those of R¹, R³, b, c, m and n each are the same in Formula (4), and the preferable ranges are also the same as those of R¹, R³, b, c, m and n each are the same in Formula (4).

Next, the reaction method is explained.
The use amount of the acetylene compounds represented by Formula (8) or (12) relative to the amino group equivalent of the carboxylic acids represented by Formula (6) or Formula (11), or derivatives thereof is preferably in the range of from 1.0 to 10 times by mole, more preferably from 1.0 to 2.0 times by mole, and even more preferably from 1.0 to 1.2 times by mole. It is not desirable that the amount be less than 1.0 time by mole, because the yield is reduced resulting from inevitable formation of unreacted compounds represented by Formula (6) or Formula (11). It is not desirable that the amount exceed 20 times by mole, because the production cost is raised due to excessive amount of raw material to be used, even though the reaction is not seriously hindered.

The solvent which can be used for the reaction is not specifically restricted as long as the solvent does not cause any problems on process operations, does not hinder the progress of the reaction, and does not adversely affect the reaction due to decomposition of the solvent. For example, an amide-based solvent (for example, N,N-dimethylformamide, N,N-dimethylacetamide, 1-methyl-2-pyrrolidone), a sulfone-based solvent (for example, sulfolane), a sulfoxide-based solvent (for example, dimethylsulfoxide), a ureido-based solvent (for example, tetramethylurea), an ether-based solvent (for example, dioxane and cyclopentyl methyl ether), a ketone-based solvent (for example, acetone, cyclohexanone), a hydrocarbon-based solvent (for example, toluene, xylene, n-decane), a halogen-based solvent (for example, tetrachloroethane, chlorobenzene), a pyridine-based solvent (for example, pyridine, γ-picoline, 2,6-lutidine), an ester-based solvent (for example, ethyl acetate, butyl acetate), and a nitrile-based solvent (for example, acetonitrile) may be used alone or in combination. Among them, the amide-based solvent, the ether-based solvent, the pyridine-based solvent and the nitrile-based solvent are preferable, and the amide-based solvent and the ether-based solvent are even more preferable. These solvents may be used alone, or two or more kinds of the solvents may be mixed, and used.

The reaction temperature is preferably in the range of from 0°C to 150°C, more preferably from 20°C to 100°C, and even more preferably from 20°C to 80°C. Although the reaction time varies with the amount of preparations and the reaction temperature, the reaction time is preferably in the range of from 0.5 to 12 hours, and more preferably from 2 to 6 hours. It is desirable to form an inert atmosphere with a stream of nitrogen, argon or the like at the time of the reaction.

As a method of isolating the acetylene compound of the invention from a reaction mixture, for example, a separation purifying method by chromatography, crystallization or recrystallization after extraction with an organic solvent, may be used. The acetylene compound of the invention is desirably isolated by crystallization. When the acetylene compound deposits by cooling the solvent extracted with an organic solvent, the acetylene compound can be isolated by a usual solid-liquid separation. Alternatively, the acetylene compound can also be crystallized from a suitable solvent system, and isolated by a solid-liquid separation.

Examples of the organic solvent for extracting the acetylene compound include ether-based solvents such as diethyl ether, diisopropyl ether, methyl-t-butyl ether, methoxy benzene or the like, ester-based solvents such as ethyl acetate, n-butyl acetate or the like, aliphatic hydrocarbon solvents such as hexane, heptane, cyclohexane or the like, and aromatic hydrocarbon solvents such as toluene, xylene or the like, but from the viewpoint of the mass-produceability in an industrial scale and availability, ester-based solvents, aliphatic hydrocarbon solvents and aromatic hydrocarbon solvents are preferable, and ester-based solvents are even more preferable.

As an organic solvent for crystallizing the acetylene compound, for example, the mixed system of the organic solvent explained in the above, and other organic solvents is exemplified. Examples of the other organic solvents to be mixed include ether-based solvents such as diethyl ether, diisopropyl ether, methyl-t-butyl ether, methoxy benzene or the like, nitrile-based solvents such as acetonitrile or the like, aliphatic hydrocarbons such as hexane, heptane, cyclohexane or the like, aromatic hydrocarbon solvents such as toluene, xylene or the like, and alcohol-based solvents such as 2-propanol, t-butanol or the like, but ester-based solvents, aromatic hydrocarbon solvents, nitrile-based solvents, and water are desirable.

In particular, a manufacturing method, in which the amino group of the amino carboxylic acid ester represented by Formula (6) is converted into a carbamic acid ester represented by Formula (7) with the use of a halogenated carbonate, the carbamic acid ester is allowed to react with the amino acetylene compound represented by Formula (8), so that an ester compound having an ethynyl group substituted by R¹ represented by Formula (9) is synthesized, the ester compound is alkali-hydrolyzed, and the hydrolysate is neutralized with an acid to obtain a carboxylic acid compound represented by Formula (10), can continuously be performed, without taking out the intermediates from a series of these reactions.

As alkali agents used for the alkali hydrolysis, inorganic or organic alkali agents usually used in organic synthetic reactions can be used. Examples of inorganic alkali agents include, for example, hydroxides of alkali metals such as lithium, sodium, potassium or the like, alkali earth metals such as calcium, barium or the like, and the salts of weak acids such as carbonic acid, bicarbonic acid, silicic acid, oxalic acid, acetic acid or the like. Examples of organic alkali agents include, for example, aqueous ammonia, ethylamine, diethylamine, dimethyl ethanolamine, tetramethyl ammonium hydroxide, tetraethyl ammonium hydroxide, choline, pyrrole, piperidine, 1, 5-diazabicyclo-[4, 3, 0]-5-nonene, 1,8-diazabicyclo-[5, 4, 0]-7-undecene, and the like, but are not limited to these compounds. Among them, sodium hydroxide, potassium hydroxide, tetraethyl ammonium hydroxide, 1,5-diazabicyclo-[4, 3, 0]-5-nonene, 1,8-diazabicyclo-[5, 4, 0]-7-undecene and the like are desirable.

These alkali agents may be used suitably singly or in combination of two or more kinds thereof in the reaction, as needed, in an amount of 0.1 to 100 times by equivalent, preferably 0.5 to 30 times by equivalent and more preferably 0.8 to 10 times by equivalent with respect to the diester compound represented by Formula (8).

In general, the reaction product is preferably neutralized using an acid after the alkali hydrolysis reaction. As the acid used, inorganic or organic acids which are usually used in organic synthetic reactions can be used. For example, as inorganic acids such as hydrochloric acid, bromic acid, fluoric acid, nitric acid, sulfuric acid, phosphoric acid, perchloric acid, chromic acid, tetrafluoroboric acid, hexafluorophosphoric acid, carbonic acid or the like may be exemplified, and for example, as organic acids such as methanesulfonic acid, trifluoromethanesulfonic acid, nonafluorobutanesulfonic acid, p-toluenesulfonic acid, acetic acid, formic acid, or the like may be exemplified, but are not limited to these acids. These may be used singly, or two or more kind thereof may be used in combination. These acids alkali agents may be used in the neutralizing reaction, in an amount of 0.1 to 100 times by equivalent, preferably 0.5 to 50 times by equivalent and more preferably 0.8 to 20 times by equivalent with respect to the salt of hydrolysates of the ester compound represented by Formula (9).

Further, an aminocarboxylic acid derivative represented by Formula (11) is allowed to react with an acetylene compound having an ethynyl group substituted with R¹ shown in Formula (12) to convert into an amic acid compound represented by Formula (13), and further the amic acid is cyclized to form continuously a product represented by Formula (4), without taking out the amic acid represented by Formula (13).

The compound, in which X in Formula (1) or (2) is represented by -NH(C=O)O- as shown the following Formula (19), is a compound which is a reaction product of the compound represented by Formula (7) and a compound represented by the following Formula (20), and R¹, R², R³, a, b and c each have the same definitions as those of R¹, R², R³, a, b and c in Formula (3), and the preferable ranges thereof are also the same as those of R¹. R², R³, a, b and c in Formula (3). R⁴has the same definition as that of R⁴ in Formula (6), and the preferable range thereof is also the same as that of R⁴ in Formula (6).

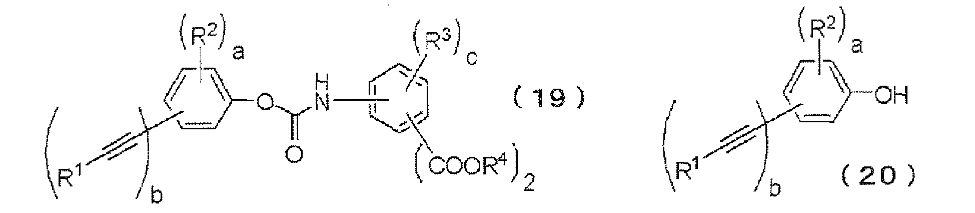

In the compound represented by Formula (20), R¹, R², a and b each have the same definitions as those of R¹, R², a and b in Formula (3).

The reaction method is the same as the formation method of the compound represented by Formula (9), except that the compound represented by Formula (20) is used in place of the compound represented by Formula (8).

The compound, in which X in Formula (1) or (2) is represented by -NH(C=O)O- as shown in the following Formula (21), is a compound which is a reaction product of a compound represented by Formula (6) and a compound represented by the following Formula (22), and R¹, R², R³, a, b and c each have the same definitions as those of R¹, R², R³, a, b and c in Formula (3), and the preferable ranges thereof are also the same as those of R¹, R², R³, a, b and c in Formula (3). R⁴ has the same definition as that of R⁴ in Formula (6), and the preferable range thereof is also the same as that of R⁴ in Formula (6).

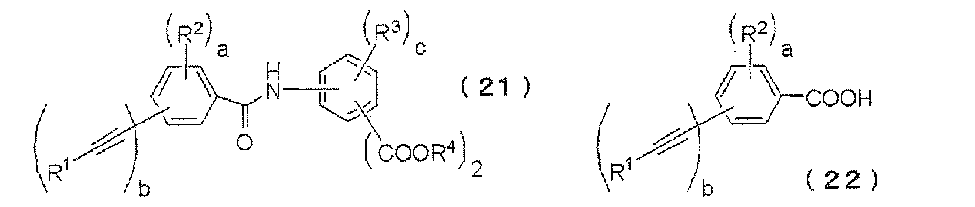

In the compound represented by Formula (22), R¹, R², a and b each have the same definitions as those of R¹, R², a and b in Formula (3).

When the reaction is carried out, an appropriate dehydrating agent may be added to the reaction system, if needed, and the reaction may be carried out by converting the hydroxyl group in the carboxyl group of Formula (22) into a leaving group having a higher activity by using a suitable activating agent.
Specific examples of the dehydrating agent include inorganic acids such as hydrochloric acid, sulfuric acid, phosphoric acid or the like, organic acids such as methanesulfonic acid, p-toluenesulfonic acid or the like, acidic ion exchange resins such as AMBERLITE, AMBERLYSTE or the like, and condensing agents such as dicyclocarbodiimide or the like. In view of the reactivity, and the low possibility of causing a side reaction, dicyclocarbodiimide is desirable.

As the of types of a more highly active leaving group, chlorine, bromine, acid anhydride, a sulfonyl derivative and the like are exemplified, and from the viewpoints of the reactivity and the availability of raw materials, chlorine, a sulfonyl derivative and the like are desirable. When the leaving group is chlorine, as the type of the activating agent, thionyl chloride, oxalyl chloride, posphorus trichloride, phosphorus pentachloride, N-chlorosuccinimide and the like are exemplified. When the leaving group is bromine, phosphorus tribromide, N-bromosuccinimide and the like are exemplified. When the leaving group is an acid anhydride, acetyl chloride, pivaloyl chloride, acetic anhydride and the like are exemplified. When the leaving group is a sulfonyl derivative, methanesulfonyl chloride, p-toluenesulfonyl chloride and the like are exemplified.
The method of reaction other than the above matters is similar to that of the compound shown in Formula (9).

Other embodiments of the invention include an acetylene compound recited in at least one of <1> to <14> above, and/or a composition containing the polymer recited in at least one of <15> to <18> above. The composition is preferably a composition containing the polymer recited in at least one of <15> to <18> above, and the kind of other additives and the addition amount of the additives may be suitably selected and added to the composition used as a final product or the intermediate product thereof according to the needs in industrial segments in accordance with use, purpose and the like for functional materials such as liquid crystal materials, non-linear optical materials, electronic materials (for example, a semiconductor protective film, a substrate for flexible printed wiring circuit and the like), materials for adhesives, materials for sliding agent, additives for photographic use and materials for gas separation membrane, and raw materials for medical and agricultural intermediates in the industrial segments.

### <Other additives>

Examples of the other additives include polymerizable compounds, resins, crosslinkable resins, solvents, polymerization initiators, colorants, polymerization inhibitors, fillers, silane coupling agents and release agents.

Exemplary polymerizable compounds include an addition-polymerizable compound having at least one ethylenically unsaturated double bond, which can be selected from compounds having at least one ethylenically unsaturated bond, preferably two or more. These compounds are widely known in this industrial field, and can be used in the invention without any particular limitation. These compounds have a chemical structure of a monomer, a prepolymer (a dimer, a trimer or an oligomer), a mixture thereof, a copolymer thereof, or the like. Examples of the monomer or the copolymer thereof include unsaturated carboxylic acids (such as acrylic acid, methacrylic acid, itaconic acid, crotonic acid, isocrotonic acid and maleic acid), esters thereof, and amides thereof. Esters of an unsaturated carboxylic acid and an aliphatic polyhydric alcohol compound, and amides of an unsaturated carboxylic acid and an aliphatic polyhydric alcohol compound are preferably used. Further, an addition-reaction product obtained from an unsaturated carboxylic acid ester or an amide having a nucleophilic substituent such as a hydroxy group, an amino group or a mercapto group and a monofunctional or polyfunctional isocyanate or epoxy compound; and a dehydration-condensation-reaction product obtained from an unsaturated carboxylic acid ester or an amide and a monofunctional or polyfunctional carboxylic acid, are also suitably used. Moreover, an addition-reaction product obtained from an unsaturated carboxylic acid ester or an amide having an electrophilic substituent such as an isocyanate group or an epoxy group and a monofunctional or polyfunctional alcohol, amine or thiol; and a substitution-reaction product obtained from an unsaturated carboxylic acid ester or an amide having a leaving substituent such as a halogen group or a tosyloxy group and a monofunctional or polyfunctional alcohol, amine or thiol are also suitable. Further examples include compounds in which the aforementioned unsaturated carboxylic acid is substituted with an unsaturated phosphonic acid, styrene, vinyl ether or the like.

Further, for example, the aliphatic alcohol-based esters described in Japanese Patent No. 46-27926, Japanese Patent No. 51-47334 and JP-A No. 57-196231; the compounds having an aromatic skeleton described in JP-ANos. 59-5240, 59-5241 and 2-226149; the compounds having an amino group described in JP-A No. 1-165613 are also preferably used. Moreover, the aforementioned ester monomers may be used as a mixture.

Specific examples of the monomer of an amide formed from an aliphatic polyvalent amine compound and an unsaturated carboxylic acid include methylenebis-acrylamide, methylenebis-methacrylamide, 1,6-hexamethylenebis-acrylamide, 1,6-hexamethylenebis-methacrylamide, diethylenetriamine trisacrylamide, xylylene bisacrylamide, and xylylene bismethacryalmide. Other examples of the preferred amide-based monomers include those having a cyclohexylene structure as described in Japanese Patent No. 54-21726.
Urethane-based addition-polymerizable compounds, which are produced via addition reaction of an isocyanate and a hydroxy group, are also suitable. Specific examples thereof include vinylurethane compounds having two or more polymerizable vinyl groups in one molecule as described in JP-A No. 2004-252201, which are obtained by adding a vinyl monomer having a hydroxy group to a polyisocyanate compound having two or more isocyanato groups in one molecule as described in Japanese Patent No. 48-41708.

Further, urethane acrylates as described in, JP-A No. 51-37193, Japanese Patent No. 2-32293 and Japanese Patent No. 2-16765; urethane compounds having an ethyleneoxide-based skeleton as described in Japanese Patent No. 58-49860, Japanese Patent No. 56-17654, Japanese Patent No. 62-39417 and Japanese Patent No. 62-39418; and addidtion-polymerizable compounds having an amino structure or a sulfide structure in its molecule as described in JP-A No. 63-277653, JP-A No. 63-260909 and JP-A No. 1-105238 can also be mentioned.

Further examples include polyfunctional acrylates or methacrylates such as polyester acrylates and epoxy acrylates obtained by reacting an epoxy resin with (meth)acrylic acid, as described in JP-A No. 48-64183, Japanese Patent No. 49-43191 and Japanese Patent No. 52-30490. Further, specific unsaturated compounds as described in Japanese Patent No. 46-43946, Japanese Patent No. 1-40337 and Japanese Patent No. 1-40336, and vinylphosphonic acid-based compounds as described in JP-A No. 2-25493 can also be mentioned. In certain cases, a structure having a perfluoroalkyl group as described in JP-A No. 61-22048 is suitably used. Further, photo-curable monomers or oligomers as described on pages 300 to 308 of Jounal of the Adhesion Society of Japan, Vol. 20, No. 7 (1984) are also applicable. In addition, polymerizable compounds as described in JP-A No. 2004-252201, JP-ANo. 2007-138105 and JP-A No. 2007-177177 are also applicable.

Radically polymerizable or crosslinkable monomers, oligomers and polymers which are available as commercial products or known in the industry that can be used in the invention include those described in "Crosslinking Agent Handbook" (1981), edited by Shinzo Yamashita, published by Taiseisha Ltd.; "UV-EB Curing Handbook -Raw Materials-" (1985) edited by Seishi Kato, published by KobunshiKankokai; "Applications and Markets for UV-EB curing techniques" (1989), edited by RadTech Japan, published by CMC Publishing Co., LTd., p. 79; and "Polyester Resin Handbook" (1988), authored by Eiichiro Takiyama, published by the Nikkan Kogyo Shinbun, Ltd.

As the polymerization compound, photo-curable polymerizable compound materials used for a photopolymerizable composition described in the following documents are also suitably used in the invention: JP-A No. 7-159983, JP-A No. 7-31399, JP-A No. 8-224982, JP-A No. 10-863 and JP-A No. 9-134011.

Further examples include aromatic vinyl compounds such as styrene, vinyl toluene and α-methylstyrene; vinylesters such as vinyl acetate, vinyl propionate and vinyl versatate; allylesters such as allyl acetate; halogen-containing monomers such as vinylidene chloride and vinyl chloride; vinyl cyanides such as (meth)acrylonitrile; and high-boiling olefins.

Exemplary resins that may be optionally added include alkyd-based resin, polyester-based resin, polyether-based resin, polyurethane-based resin, vinyl-based resin, acrylic-based resin, rubber-based resin, polyolefin-based resin, polyurea-based resin, melamine resin, epoxy resin, nylon resin, polyimide resin, polystyrene-based resin, polyacetal resin, polybutyral resin, polyketal resin, novolac resin, resol resin, silicone resin, cellulose-inodified resin, and waxes.

A crosslinking agent may be added to the composition according to the invention in order to adjust the curability or the curing rate thereof. The crosslinking agent is not particularly limited as long as it can cure a film by crosslinking reaction, and those that form a crosslinked structure by heat, light, UV rays, electron beams or the like are applicable. Exemplary crosslinking agents include polyisocyanate, polyimide derivatives, epoxy resin, melamine compounds or guanamine compounds that are substituted by a methylol group and at least one selected from an alkoxymethyl group or an acyloxymethyl group, glycoluril compounds or urea compounds, phenol compounds that are substituted by a methylol group and at least one selected from an alkoxymethyl group or an acyloxymethyl group, naphthol compounds, and hydroxyanthracene compounds. Among these, a polyfunctional epoxy resin is particularly preferred.

The epoxy resin is not particularly limited as long as it has an epoxy group and an ability of crosslinking, and examples thereof include divalent glycidyl group-containing low-molecular compounds such as bisphenol-A-diglycidyl ether, ethylene glycol diglycidyl ether, butanediol diglycidyl ether, hexanediol diglycidyl ether, dihydroxybiphenyl diglycidyl ether, phthalic diglycidyl ether, and N,N-diglycidyl aniline; trivalent glycidyl group-containing low-molecular compounds such as trimethylolpropane triglycidyl ether, trimethylolphenol triglycidyl ether and TrisP-PA triglycidyl ether; tetravalent glydidyl group-containing low-molecular compounds such as pentaerythritol tetraglycidyl ether and tetramethylol bisphenol-A-tetraglycidyl ether; and glycidyl group-containing high-molecular compounds such as polyglycidyl (meth)acrylate and a 1,2-epoxy-4-(2-oxyranyl)cyclohexane-adduct of 2,2-bis(hydroxymethyl)-1-butanol.

Commercially available products include bisphenol A-type epoxy resins such as EPICOAT 828 EL and EPICOAT 1004 (manufactured by Japan Epoxy Resins Co., Ltd.) and EPICOAT 806 and EPICOAT 4004 (manufactured by Japan Epoxy Resins Co., Ltd.); bisphenol F-type epoxy resins such as EPICLON 830 CRP (manufactured by DIC Corporation); bisphenol S-type epoxy resins such as EPICLON EXA 1514 (manufactured by DIC Corporation); 2,2'-diallyl bisphenol A-type epoxy resins such as RE-810 NM (manufactured by Nippon Kayaku Co., Ltd.); hydrogenated bisphenol-type epoxy resins such as EPICLON EXA 7015 (manufactured by DIC Corporation); propylene oxide-added bisphenol A-type epoxy resins such as EP-44005 (manufactured by ADEKA Corporation); resolcinol-type epoxy resins such as EX-201 (manufactured by Nagase ChemTeX Corporation); biphenyl-type epoxy resins such as EPICOAT YX-4000H (manufactured by Japan Epoxy Resins Co.. Ltd.); sulfide-type epoxy resins such as YSLV-50TE (manufactured by Tohto Kasei Co., Ltd.); ether-type epoxy resins such as YSLV-80DE (manufactured by Tohto Kasei Co., Ltd.); dicyclopentadiene-type epoxy resins such as EP-4088S (manufactured by ADEKA Corporation); naphthalene-type epoxy resins such as EPICLON HP 4032 and EPICLON EXA-4700 (manufactured by DIC Corporation); phenol novolac-type epoxy resins such as EPICLON N-770 (manufactured by DIC Corpporation); orthocresol novolac-type epoxy resins such as EPICLON N-670-EXP-S (manufactured by DIC Corporation); dicyclopentadiene novolac-type epoxy resins such as EPICLON HP 7200 (manufactured by DIC Corporation); biphenyl novolac-type epoxy resins such as NC-3000P (manufactured by Nippon Kayaku Co., Ltd.); naphthalene phenol novolac-type epoxy resins such as ESN-165S (manufactured by Tohto Kasei Co., Ltd.); glycidylamine-type epoxy resins such as EPICOAT 630 (manufactured by Japan Epoxy Resins Co., Ltd.), EPICLON 430 (manufactured by DIC Corporation) and TETRAD-X (manufactured by Mitsubishi Gas Chemical Company, Inc.); alkylpolyol-type epoxy resins such as ZX-1542 (manufactured by Tohto Kasei Co., Ltd.), EPICLON 726 (manufacrued by DIC Corporation), EPOLITE 80 MFA (manufactured by Kyoeisha Chemical Co., Ltd.) and DENACOL EX-611 (manufactured by Nagase ChemTeX Corporation); rubber modified-type epoxy resins such as YR-450 and YR-207 (manufactured by Tohto Kasei Co., Ltd.) and EPOLEAD PB (manufactured by Daicel Chemical Industries, Ltd.); glycidylester compounds such as DENACOL EX-147 (manufactured by Nagase ChemTeX Corporation); bisphenol A-type episulfide resins such as EPICOAT YL-7000 (manufactured by Japan Epoxy Resins Co., Ltd.); and other products such as YDC-1312, YSLV-80XY and YSLV-90CR (manufactured by Tohto Kasei Co., Ltd.), XAC 4151 (manufactured by Asahi Kasei Corporation), EPICOAT 1031 and EPICOAT 1032 (manufactured by Japan Epoxy Resins Co., Ltd.), EXA-7120 (manufacrued by DIC Corporation), and TEPIC (manufactured by Nissan Chemical Industries, Ltd.)

The amount of the epoxy resin to be formulated is not particularly limited, and may be adjusted as appropriate according to the type or the amount of the aforementioned (meth)acrylate, such as an epoxy (meth)acrylate.

### <Thermal-curing agent>

A thermal-curing agent may be included in the composition according to the invention in order to further promote the thermal curing of the epoxy resin or the like. The thermal-curing agent is used to cause reaction of unsaturated bonds, epoxy groups or the like in the curable resin by heating, thereby allowing the same to crosslink. Therefore, the thermal-curing agent plays a role of improving adhesion or moisture resistance of the cured product after being cured. The thermal-curing agent is not particularly limited, but when the composition according to the invention is cured at a relatively low temperature of, for example, from 100 to 150°C, the thermal-curing agent preferably includes an amine group and/or a thiol group that has superior low-temperature reactivity.

Examples of the thermal-curing agent having an amine group and/or a thiol group include organic acid dihydrazide compounds such as 1,3-bis[hydrazinocarbonoethyl-5-isopropylhydantoin] and adipic acid dihydrazide; dicyanamide, guanidine derivatives, 1-cyanoethyl-2-phenylimidazole, N-[2-(2-methyl-1-imidazolyl)ethyl)urea, 2,4-diamino-6-[2'-methylimidazolyl-(1')]-ethy]-s-triazine, N,N'-bis(2-methyl-1-imidazolylethyl)urea, N,N'-(2-methyl-1-imidazolylethyl)-adipamide, 2-phenyl-4-methyl-5-hydroxymethylimidazole, 2-imidazoline-2-thiol, 2-2'-thiodiethanethiol, and addition products of various amines and an epoxy resin. These thermo-curing agents may be used alone or in combination of two or more kinds.

A solvent may be added to the composition according to the invention. The solvent is not particularly limited as long as it does not hamper the progress of reaction during curing the composition or the like, or does not adversely affect the preservation storability of the composition. Exemplary solvents include amide-based solvents (such as N,N-dimethylformamide, N,N-dimethylacetamide and 1-methyl-2-pyrrolidone), sulfone-based solvents (such as sulfolane), sulfoxide-based solvents (such as dimethylsulfoxide), ureido-based solvents (such as tetramethylurea), ether-based solvents (dioxane and cyclopenthyl methyl ether), ketone-based solvents (such as acetone and cyclohexanone), hydrocarbon-based solvents (such as toluene, xylene and n-decane), halogen-based solvents (such as tetrachloroethane and chlorobenzene), pyridine-based solvents (such as pyridine, γ-picoline and 2,6-lutidine), ester-based solvents (such as ethyl acetate and butyl acetate) and nitrile-based solvents (such as acetonitrile), and these solvents may be used alone or in combination. Among these, amide-based solvents, sulfone-based solvent, sulfoxide-based solvents, ureido-based solvents, ether-based solvents, halogen-based solvents, pyridine-based solvents and nitrile-based solvents are preferred; amide-based solvents, ether-based solvents, halogen-based solvents and nitrile-based solvents are more preferred; and amide-based solvents and nitrile-based solvents are further preferred. These solvents may be used alone or in combination of two or more kinds. The amount of the solvent to be added to the composition according to the invention may be selected according to applications or necessary performances in the applied area, but is preferably from 0 to 90% by mass, more preferably from 0 to 80% by mass, further preferably from 0 to 70% by mass, with respect to the total amount of the composition. Further, use of a solvent may not be preferred in some cases.

The composition according to the invention may include a polymerization initiator such as a photo-polymerization initiator or a thermal-polymeirzation initiator, in order to promote polymerization of a polymerizable compound or reaction of a crosslinking agent.
Exemplary photo-polymerization initiators include photo-initiators described in JP-A No. 2004-252201; peroxides described in the U.S. Patent No. 4,950,581; aromatic sulfoniums, phosphoniums or iodiniums described in the U.S. Patent No. 4,950,581; cyclopentadienyl-arene-metal complex salts, oxime sulfonic acid esters described in European Patent No. 780,729; and pyridinium and (iso)quinolinium salts described in European Patent Nos. 497,531 and 441,232. Further examples include halomethyltriazines described in G. Buhr, R. Dammel and C. Lindley, Polym. Mater. Sci. Eng. 61, 269 (1989) and European Patent No. 022788; halomethyloxazole photoinitiators described in the U.S. Patent Nos. 4,371,606 and 4,371,607; 1,2-disulfones described in E.A.Bartmann, Synthesis 5,490 (1993); hexaarylbisimidazole and a hexaarylbisimidazole/co-initiator system (such as 2-mercaptobenzthiazole and ferrocenium compounds), or titanocenes (such as a mixture of o-chlorohexaphenyl-bisimidazole and bis(cyclopentadienyl)-bis(2,6-difluoro-3-pyrylphenyl)titanium). A photosensitizer may be used in combination, and examples thereof include amines such as triethanolamine and 4-dimethylaminobenzoic acid ethyl ester, benzophenone and derivatives thereof, thioxanthone and derivatives thereof, anthraquinone and derivatives thereof, and coumarin derivatives.

Exemplary thermo-polymerization initiators include azo compounds such as 2,2'-azobis(4-methoxy-2,4-dimethylvaleronitrile), triazene, diazosulfide and pentaazadiene; and organic peroxides such as hydroperoxide, peroxycarbonate and tert-butylhydroperoxide. Organic peroxides that do not produce air bubbles are preferably used as the thermo-polymerization initiator. Widely used organic peroxides are applicable, and examples thereof inlcude peroxydicarbonate, peroxyester, peroxyketal, ketoneperoxide and hydroperoxide. These organic peroxides may be used alone or in combination of two or more kinds, and may be used by diluting with a solvent or adsorbing to a powder material. The amount of the polymerization initiator is preferably from 0.01 to 10% by mass with respect to the total amount of the composition. When the above proportion is less than 0.01 % by mass, the degree of curing during heating may not be sufficient; and when exceeds 10% by mass, the curing reaction may be adversely affected.

Further, for the purpose of suppressing undesired reaction during storage, known additives such as polymerization inhibitors, chain transfer agents, UV absorbers or stabilizers may be added to the composition according to the invention. Exemplary polymerization inhibitors include hydroquinone, hydroquinone derivatives, p-methoxyphenol, and sterically-hindered phenols such as 2,6-di-tert-butyl-p-cresol. In order to improve the stability during dark storage, copper compounds (such as copper naphthenate, copper stearate and copper octanoate), phosphorous compounds (such as triphenyl phosphine, tributyl phosphine, triethyl phosphite, triphenyl phosphite and tribenzyl phosphite), quartenary ammoniuom compounds (such as tetramethylammonium chloride and trimethylbenzyl ammonium chloride) or hydroxyamine derivatives (such as N-diethylhydroxyamine) may be added. Exemplary chain transfer agents include mercaptan, amine and benzothiazole.

Further, a small amount of light stabilizer may be added, and examples thereof include UV absorbers (such as hydroxyphenylbenzotriazole, hydroxyphenylbenzophenone, oxalamide or hydroxyphenyl-s-triazine-type). These compounds may be used alone or in combination of two or more kinds, under the presence or absence of a sterically-hindered amine. Exemplary UV absorbers or light stabilizers include 2-(2'-hydroxyphenyl)benzotriazole, 2-hydroxybenzophenone, substituted or unsubstituted benzoates, acrylates, sterically-hindered amines, oxalamides, 2-(2-hydroxyphenyl)-1,3,5-triazine, phosphite esters, and phosphonate esters.

The composition according to the invention may include other components such as a known silane coupling agent, a fludity modifier or an adhesion promotor, in order to improve the adhesion of the composition. Exemplary silane coupling agents include vinyltrimethoxysilane, vinyltriethoxysilane, vinyltris(2-methoxyethoxy)silane, vinyltrichlorosilane (KA-1003, manufactured by Shin-Etsu Chemical Co., Ltd.), 2-(3,4epoxycyclohexyl)ethyltrimethoxysilane (KBM-303, manufactured by Shin-Etsu Chemical Co., Ltd.), p-styryltrimethoxysilane (KBM-1403, manufactured by Shin-Etsu Chemical Co., Ltd.), 3-methacryloxypropylmethyldimethoxysilane (KBM-502, manufactured by Shin-Etsu Chemical Co., Ltd.), 3-acryloxypropyltrimethoxysilane (KBM-5103, manufactured by Shin-Etsu Chemical Co., Ltd.), 3-aminopropyltriethoxysilane, 3-aminopropyltrimethoxysilane (KBM-903, manufactured by Shin-Etsu Chemical Co., Ltd.), N-(2-aminoethyl)-3-aminopropylmethyldimethoxysilane, N-(2-aminoethyl)-3-aminopropyltrimethoxysilane, 3-glycidoxypropyltrimethoxysilane, 3-glycidoxypropylmethyldimethoxysilane, 3-chloropropylmethyldimethoxysilane, 3-chloropropyltrimethoxysilane, and 3-mercaptopropyltrimethoxysilane.

### <Filler>

The composition according to the invention may include a filler in order to adjust the viscosity or preservation stability thereof, or the rigidity, viscoelasticity, bulk density or expansion coefficient of the cured product. The filler is not particularly limited, and examples thereof include inorganic fillers such as silica, diatom earth, alumina, zinc oxide, iron oxide, magnesium oxide, tin oxide, titanium oxide, alumina, magnesium hydroxide, aluminum hydroxide, carcium carbonate, magnesium carbonate, barium sulfate, magnesium sulfate, plaster, calcium silicate, aluminum silicate, zirconium silicate, potassium titanate, kaolin, talc, glass beads, sericite, activated clay, bentonite, aluminum nitride, silicon nitride, glass microparticles described in the U.S. Patent No. 5,013,768, or micronized glass fibers; and known organic fillers such as polymethyl(meth)acrylate, polystyrene, copolymers of these polymers and a monomer than can be copolymerized with these polymers, polyester microparticles, polyurethane microparticles, and rubber microparticles.

### <Colorant>

The composition according to the invention may include a colorant such as a dye or a pigment in view of texture, appearance or design. Known dyes including those commercially obtainable or those described in "Dye Handbook", edited by the Society of Synthetic Organic Chemistry, Japan, (1970), or the like, can be used in the invention. Specific examples of the dye include triarylmethane-based dyes, carbonium-based dyes, anthraquinone-based dyes, naphthoquinone-based dyes, quinone-imine-based dyes, azomethine-based dyes, azo-based dyes, metal complex salt azo-based dyes, benzilidene-based dyes, oxonol-based dyes, cyanine-based dyes, phenothiazine-based dyes, xanthene-based dyes, phthalocyanine-based dyes, benzopyrane-based dyes, indigo-based dyes, methine-based dyes, azine-based dyes, oxazine-based dyes, thiazine-based dyes, anthrapyridone-based dyes, squarylium-based dyes, pyrylium salt-based dyes, and metal thiolate-based complexes.
Preferred dyes include the cyanine dyes described in JP-A No. 58-125246, JP-A No. 59-84356, JP-A No. 59-202829 and JP-A No. 60-78787; the methine dyes described in JP-A No. 58-173696, JP-A No. 58-181690 and JP-A No. 58-194595; the naphthoquinone dyes described in JP-A No. 58-112793, JP-A No. 58-224793, JP-A No. 59-48187, JP-A No. 59-73996, JP-A No. 60-52940 and JP-A No. 60-63744; the squarylium dyes described in JP-A No. 58-112792; and the cyanine dyes described in British Patent No. 434,875.

Further examples of the dye include the near-infrared-absorbing sensitizers described in the U.S. Patent No. 5,156,938; the substitued arylbenzo(thio)pyrylium salts described in the U.S. Patent No. 3,881,924; the trimethinethiapyrylium salts described in JP-ANo. 57-142645 (the U.S. Patent No. 4,327,169); the pyrylium compounds described in JP-A Nos. 58-181051, 58-220143, 59-41363, 59-84248, 59-84249, 59-146063 and 59-146061; the cyanine dyes described in JP-A No. 59-216146; the pentamethinethiopyrylium salts described in the U.S. Patent No. 4,283,475; and the pyrylium compounds described in Japanese Patent Nos. 5-13514 and 5-19702.
Futher preferred examples of dye include the near-infrared-absorbing dyes described in the U.S. Patent No. 4,756,993.
It is also possile to use the dyes disclosed in JP-A No. 64-90403, JP-A No. 64-91102, JP-A No. 1-94301, JP-A No. 6-11614, Japanese Patent No. 2592207, the U.S. Patent No. 4,808,501, the U.S. Patent No. 5,667,920, the U.S. Patent No. 5,059,500, JP-A No. 5-333207, JP-A No. 6-35183, JP-A No. 6-51115, JP-A No. 6-194828 and JP-A No. 2004-252201.
Since many dyes may cause decrease in sensitivity of the polymerization system, a pigment is particularly preferably used as the colorant.

Examples of the pigment that may be optionally added in the invention include commercially available pigments and pigments described in the Color Index (C.I.) Handbook; "Saishin Ganryou Binran (Newest Pigment Handbook)", published by the Japan Society of Color Material, 1977; "Saishin Ganryou Ouyou Gjutsu (Newest Pigment Application Technique)", published by CMC Publishing Co., Ltd., 1986; "Insatsu Ink Gijutsu (Printing Ink Technique)", published by CMC Publishing Co., Ltd., 1984; JP-A No. 2004-252201; JP-A No. 2007-138105; and JP-A No. 2007-177177.

Exemplary pigments that can be used in the invention include black pigments, yellow pigments, orange pigments, brown pigments, red pigments, purple pigments, blue pigments, green pigments, fluorescent pigments, metal powder pigments, and polymer-bonded colorants. Specific examples thereof include insoluble azo pigments, azo lake pigments, condensed azo pigments, chelate azo pigments, phthalocyanine pigments, anthraquinone pigments, perylene and perinone-based pigments, thioindigo-based pigments, quinacridone-based pigments, dioxazine-based pigments, isoindolinone-based pigments, quinophthalone-based pigments, colored lake pigments, azine pigments, nitroso pigments, nitro pigments, natural pigments, phosphorescent pigments, inorganic pigments, and carbon black. Among these pigments, carbon black is preferred.

Surface treatment of the pigment may be conducted or may not. Methods of the surface treatment include a method of coating the surface of the pigment with resin or wax, a method of attaching a surfactant to the surface of the pigment, and a method of binding a reactive substance (such as a silane coupling agent, an epoxy compound or a polyisocyanate) to the surface of the pigment. The above surface treatment methoes are described in "Kinzoku Sekken no Seishitu to Ouyou (Properties and Applications of Metal Soap)", publizhed by Saiwai Shobo; "Insatsu Ink Gijutsu (Printing Ink Technique)", published by CMC Publishing Co., Ltd., 1984; and "Saishin Ganryou Ouyou Gjutsu (Newest Pigment Application Technique)", published by CMC Publishing Co., Ltd., 1986.

The particle diameter of the pigment is preferably in a range of from 0.01 µm to 10 µm, more preferably from 0.05 µm to 1 µm, particularly preferably from 0.1 µm to 1 µm. When the particle diameter of the pigment is 0.01 µm or greater, stability of materials dispersed in the coating liquid for forming an image recording layer can be improved; and when the particle diameter of the pigment is 10 µm or less, uniformity of the image recording layer can be improved.

Dispersing of the pigment may be carried out by known dispersing techniques used for the production of inks and toners. Exemples of the dispersing machine include a sand mill, an attritor, a pearl mill, a super mill, a ball mill, an impller, a disperser, a KD mill, a colloid mill, a dynatron, a three-roll mill, and a pressure kneader. Details of these machines are described in "Saishin Ganryou Ouyou Gjutsu (Newest Pigment Application Technique)", published by CMC Publishing Co., Ltd., 1986.

In addition, known additives may be added to the composition according to the invention, as necessary. Examples of the additives that can be used include surfactants, matting agents, promotors and co-initiaors such as thiol, thioether, disulfide, phosphonium salts, phosphine oxide and phosphine described in European Patent No. 438,123, British Patent No. 2,180,358 and JP-A No. 6-68309, autoxidizers, optical brightners, moisturerizers, smoothing aids, dispersants, coagulation inhibitors, defoaming agents, leveling agents, ion-trap agents, ion-exchange agents, plasticizers such as dioctylphthaltate, didodecylphthaltate, triethylene glycol dicaprylate, dimethyl glycol phthalate, tricresyl phosphate, dioctyl adipate, dibutyl sebacate and triacetylglycerin, and other additives.

The additives as mentioned above are preferably selected in accordance with the intended purpose of the composition or the characteristics required in the intended porpose. The additives as mentioned above are commonly used in the present technique, and therefore these additives are preferably added in an ordinary amount with respect to each intended purpose.

A further embodiment of the invention is a cured product produced by curing a composition including the acetylene compound according to at least one of <1> to <14> and/or the polymer according to at least one of <15> to <19>; or a cured product produced by curing the polymer according to <15> to <19>, Methods of obtaining the cured product include a method of heating and drying the composition of the invention as mentioned above and/or at least one of the polymer according to <15> to <19> or a solution thereof; and a method of melting and solidifying a powder of the composition according to the invention. However, the invention is not limited thereto.

The obtained polymer according to the invention has an acetylene group as a structural component, and by allowing this acetylene group to further polymerize, a cured product having even more improved mechanical properties and heat resistance can be obtained (here, a product produced by the reaction of acetylene in the polymer is referred to as the "cured product"). The method of reaction of the acetylene group is not particularly limited, but it is possible to progress a reaction among acetylene groups (polymerization reaction) by applying heat, light or radiation. By causing the polymerization reaction of acetylene groups, the obtained product (cured product) has a branched or three-dimensional structure, thereby making it possible to obtain a shaped product having excellent tensile modulus of elasticity or heat resistance (glass transition temperature).

The composition according to the invention has superior preservation stability without occurrence of polymerization during preservation. When energy such as heat, light, UV rays or electron beams is imparted, polymerization of a polymerizable compound in the composition is efficiently initiated in a short time, the effectiveness of which depending on the nature of the crosslinking group or polymerizable group, or a crosslinking group incorporated in a side chain, main chain or terminal of the polymer chain or the compound according to the invention crosslinks and forms a cured resin product. As a result, the thus obtained cured product is insoluble with respect to an organic solvent and exhibits improved solvent resistance, chemical resistance, heat resistance, mechanical strength or the like. Therefore, the composition according to the invention can be formed into various kinds of shaped products through various kinds of shaping methods, as a matrix resin of various kinds being soluble in an organic solvent. Moreover, the compositon is highly versatile and by curing the same by crosslinking after the shaping, the resulting product exhibits excellent solvent resistance, chemical resistance or mechanical strength. Therefore, the cured product can be used as an excellent resin material, and is suitably used for a mechanical member or an electrical-resistance body. Accordingly, the composition according to the invention is suitably used also for printing inks (such as inks for screen printing, off-set printing and flexographic printing); clear finishing agents (such as white or color finishing agents applied to wood or metal); powder coatings (in particular, coating materials applied to paper, wood, metal or plastics); marking materials for architecture, objects or roads; materials for photoduplication, holographic recording, image recording or production of print precursors; sun light-curable coating materials for producing masks used in screen printing; filler compositions for dental use; adhesives; pressure-sensitive ahesives; resins for forming a laminate; etching resists in the form of a liquid or a film; soldering resists; electroplating resists; permanent resists; dielectrics used for print curcuit boards or electic circuits, which are constructable by a photolithograpy; materials for various kinds of displays; materials for producing color filters (such as the color filters described in the U.S. Patent No. 5,853,446, European Patent No. 863,534, JP-A Nos. 9-244230, 10-62980 and 8-171863, the U.S. Patient No. 5,840,465, European Patent No. 855,731, JP-A No. 5-571576 and JP-A No. 5-67405); materials for producing optical switches, optical grids (interference grids) and optical circuits; materials for producing three-dimentional objects by mass-curing (UV-curing using a clear molding) or stereolithography (such as those described in the U.S. Patent No. 4,575,330); composite materials (such as styrene-based polyester at least including glass fiber and/or other fiber in combination with other aiding agents); materials for producing other compositions that forms a thick layer; resists used for coating or sealing electronic members or intergrated curcuits; and optical lens (such as coating agents for contact lens and Fresnel lens). Further, the composition according to the invention is suitably used for producing medical instruments, aiding devices and dental implants. Moreover, the composition according to the invention is suitably used for producing a gel having thermotropic properties, such as those described in German Patent No. 19,700,064 and European Patent No. 678,534.

By curing the composition by crosslinking the same after shaping, the composition can exhibit extremely high solvent resistance, chemical resistance and mechanical strength. Threfore, the compositon can be used as excellent resin materials. In particular, the composition is particularly suitable for electrical resistance materials or moisture-proof coating materials, such as lubricants described in JP-A No. 2006-225481, JP-A No. 2006-176548, JP-A No. 2006-169398, JP-A No. 2005-194370 and JP-A No. 2005-036158. For example, the composition can be used also as a binder resin for carbon resistance materials, or a moisture-proof coating material for semiconductor materials. The composition can be used as a resistance material for a variable resistor by forming a resistance paste by mixing with carbon, and then sintering the same.

In the invention, the composition is preferably heated to form a crosslinked structure. When energy is applied by heating, the heating can be carried out with an oven or a hot plate having a heater, or by means of photothermal conversion using infrared rays or visible light. The curing is preferably carried out by heating the polymer according to the invention, and the heating temperature is preferably from 50 to 500°C, more preferably from 150 to 450°C, further preferably from 200 to 400°C. The time for curing varies according to the temperature, but is typically from 0.1 seconds to 24 hours, preferably from 10 minutes to 10 hours, more preferably from 30 minutes to 5 hours. When the curing is carried out under the conditions within the above ranges, a cured product having excellent mechanical properties and heat resistance can be obtained.

When energy is applied by exposing the composition to light, the means for light irradiation can be selected from light sources that emit light of from ultraviolet rays to visible rays, such as mercury lams of from low pressure to super-high pressure, metal halide lamps, and Xe lamps.
Further, as a shape of the cured products obtained by the method, a film, a pellet, a fibrous product, other various kinds of molded products and the like are exemplified, but are not limited to these products.
Disclosures of Japanese Patent Application No. 2007-256372 filed on September 28, 2007, Japanese Patent Application No. 2007-256501, filed on September 28, 2007, and Japanese Patent Application No. 2008-244134 filed on September 24, 2008, as a whole, are cited and incorporated herein.

### EXAMPLES

Next, the invention is explained based on Examples in detail, but the invention is not limited to Examples.

### <Measurement device and measurement conditions>

For the purpose of evaluating characteristics of the obtained compounds, various spectra of 1H-NMR and MS were measured. The measurement conditions of respective characteristics are as follows.
Nuclear magnetic resonance spectrum analysis (1H-NMR): 1H-NMR was measured at the resonant frequency of 400 MHz using AV400M manufactured by BRUKER Corporation. As a measurement solvent, deuterated dimethyl sulfoxide (DMSO-d6) was used.
Mass analysis (MS): Mass spectrum was measured by the ESI method using API QSTAR Pulsar i manufactured by Applied Biosystems Inc.

### <Synthesis of acetylene compound >

### [Example 1]

Exemplary Compound (1)-64 was synthesized according to the following Formula:

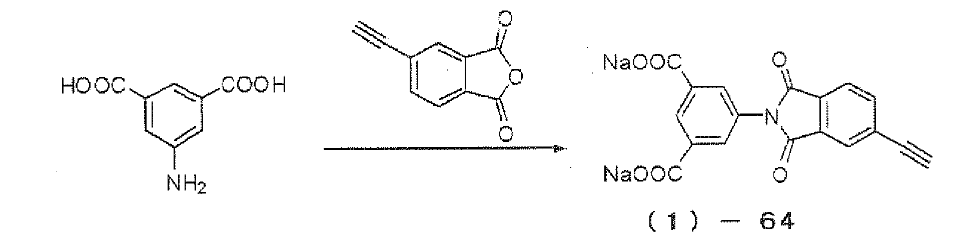

To a 3000 mL three-necked flask, were introduced 63 g of 5-aminoisophthalic acid and 600 mL of N-inethyl-2-pyrrolidone, and the mixture was stirred until the solute was completely dissolved. To a 500 mL conical flask, were introduced 60 g of 5-ethynyi isobenzofuran-1,3-dione and 600 mL of N-methyl-2-pyrrolidone, and the mixture was stirred until the solute was completely dissolved, thereby forming a dropping liquid 1. The dropping liquid 1 was added dropwise using a dropping funnel over 15 minutes to the mixture in the 3000 mL three-necked flask, with stirring continuously, and the resultant mixture was heated to 40°C and was stirred continuously for 4 hours.

After the flask was cooled to room temperature, a liquid in which 2.8 g of pyridine was dissolved in 20 mL of N-methyl-2-pyrrolidone was added to the 3000 mL three-necked flask, subsequently, a mixed liquid of 153 g of acetic anhydride and 150 g of N-methyl-2-pyrrolidone was added dropwise using a dropping funnel over 15 minutes to the 3000 mL three-necked flask, and the resultant mixture was stirred continuously for 4 hours, and was allowed to stand overnight after stopping the stirring.

The flask was ice-cooled at the time of restarting the stirring, an aqueous solution, in which 252 g of sodium hydrogen carbonate was dissolved in 1600 mL of ion exchanged water, was added dropwise into the flask over 2 hours, and 83 g of the aimed Exemplary Compound (1)-64 was obtained by being precipitated and filtered as a sodium salt (yield; 63%).
MS:M+= 379.23.

### [Example 2]

Exemplary Compound (1)-63 was synthesized according to the following Formula:

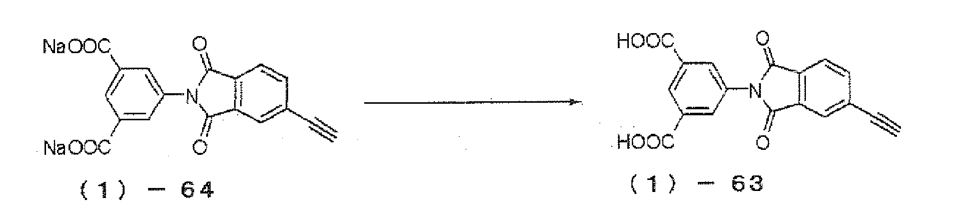

Exemplary Compound (1)-64 was neutralized in an aqueous hydrochloric acid solution prepared from 175 mL of concentrated hydrochloric acid and 1000 mL of ion exchanged water, thereby obtaining crude crystals of Exemplary Compound (1)-63. The crude crystals were washed with 1000 mL of ion exchanged water, and were filtrated off. The filtered product was introduced into a 3000 mL three-necked flask, and was dissolved in 1800 mL of N-methyl-2-pyrrolidone. To the solution was added dropwise a mixed liquid of 210 mL of acetonitrile and 490 mL of ion exchanged water using a dropping funnel over one hour, thereby precipitating Exemplary Compound (1)-63. The crystals were washed with 500 mL of acetonitrile and filtrated off, thereby obtaining 45 g of the aimed Exemplary Compound (1)-63 (yield; 61%).

MS:M+=335.04
1H-NMR (400 MHz, DMSO-d6). δ=12.79 (s, 2H), δ= 8.85 (s, 2H), δ= 8.34 (s, 1H), δ= 8.29 (s, 1H), δ= 8.10 (d, 1H), δ= 7.85 (d, 1H), δ= 4.65 (s, 1H).

### [Example 3]

Exemplary Compound (1)-67 was synthesized according to the following Formula:

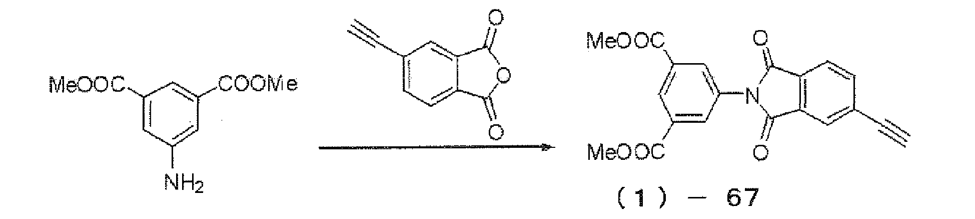

To a 2000 mL three-necked flask, were introduced 63 g of dimethyl 5-ammoisophthalate and 200 mL of N-methyl-2-pyrrolidone, and the mixture was stirred until the solute was completely dissolved. To a 500 mL conical flask, were introduced 52 g of 5-ethynyl isobenzofuran-1,3-dtone and 400 mL of N-methyl-2-pyrrolidone, and the mixture was stirred until the solute was completely dissolved, to form a dropping liquid 2. The dropping liquid 2 was added dropwise using a dropping funnel over 15 minutes to the mixture in the 2000 mL three-necked flask, with stirring continuously, and the resultant mixture was heated to 40°C and was stirred continuously for 4 hours.

After the flask was cooled to room temperature, a liquid, in which 2.4 g of pyridine was dissolved in 20 mL, was added to the 2000 mL three-necked flask, subsequently, a mixed liquid of 101 g of acetic anhydride and 100 g of N-methyl-2-pyrrolidone was added dropwise using a dropping funnel over 15 minutes to the 2000 mL three-necked flask, and the resultant mixture was stirred continuously for 4 hours. The precipitation of Exemplary Compound (1)-67 was observed with the progress of reaction. After terminating the reaction, the crystals of Exemplary Compound (1)-67 was washed with 500 mL of acetonitrile and filtered off, thereby obtaining 27 g of the aimed Exemplary Compound (1)-67 (yield; 25%).

MS:M+=363.07
1H-NMR (400 MHz, DMSO-d6): δ=8.85 (s, 2H), δ= 8.34 (s, 1H), δ= 8.29 (s, 1H), δ**=** 8.10 (d, 1H), δ= 7.85 (d, 1H), δ= 4.64 (s, 1H), δ= 3.88 (s, 6H).

### [Example 4]

Exemplary Compound (1)-5 was synthesized according to the following Formula:

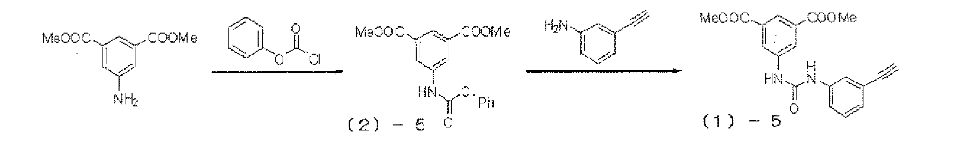

To a 1000 mL three-necked flask, were introduced 52 g of dimethyl 5-aminoisophthalate, 600 mL of tetrahydrofuran and 21 g of pyridine, and the mixture was stirred for 15 minutes. Phenyl chlorocarbonate (41 g) diluted with 100 mL of tetrahydrofuran was added dropwise using a dropping funnel over 10 minutes to the mixture in the 1000 mL three-necked flask, and the stirring was continued at room temperature for 3 hours.

Pyridine hydrochloride which was side produced during the reaction was filtered off, and the filtrate containing Exemplary Compound (2)-5 was transferred to a 2000 mL three-necked flask. The pyridine hydrochloride was extracted and washed using 800 mL of ethyl acetate and 400 mL of ion exchanged water, and a product formed by concentrating and drying the ethyl acetate phase in vacuo was dissolved in 100 mL of tetrahydrofuran, and the solution was transferred to the 2000 mL three-necked flask to mix the filtrate.

3-ethynylaniline (30 g) diluted with 100 mL of tetrahydrofuran was added dropwise using a dropping funnel over 10 minutes. Subsequently, 76 g of trimethylamine diluted with 100 mL of tetrahydrofuran was added dropwise using a dropping funnel, and the mixture was heated and refluxed for 4 hours.

The mixture was cooled to room temperature, and was dropped into a 3000 mL three-necked flask, in which 2000 ml of ethyl acetate was placed, over 30 minutes, and the obtained crystals were filtrated, thereby obtaining 73.5 g of the aimed Exemplary Compound (1)-5 (yield; 83%).

MS:M+=352.11
1H-NMR (400 MHz, DMSO-d6): δ=9.21 (s, 1H), δ= 8.86 (s, 1H), δ= 8.42 (s, 2H), δ= 8.02 (s, 1H), δ= 7.81 (s, 1H), δ= 7.62 (d, 1H), δ= 7.29 (t, 1H), δ= 7.14 (d, 1H), = 4.35 (s, 6H), δ**=** 4.17 (s, 1H).

### [Example 5]

Exemplary Compound (1)-2 was synthesized according to the following Formula:

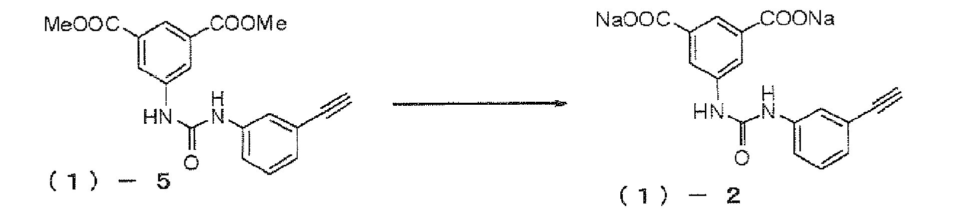

To a 1000 mL three-necked flask, were introduced 73.5 g of Exemplary Compound (1)-5 and 400 mL of tetrahydrofuran, and the mixture was stirred for 15 minute. An aqueous sodium hydroxide solution prepared from 25 g of sodium hydroxide and 200 ml of ion exchanged water was added dropwise to the mixture using a dropping funnel over 10 minutes, and the resultant solution was heated to 40°C with stirring continuously for 2 hours.

The reaction liquid was transferred to a 1000 mL three-necked flask, in which 1000 mL of acetonitrile was placed, and the obtained crystals were filtered, and 67.4 g of the aimed Exemplary Compound (1)-2 (yield : 88%).
MS:M+=368.25

### [Example 6]

Exemplary Compound (1)-1 was synthesized according to the following Formula:

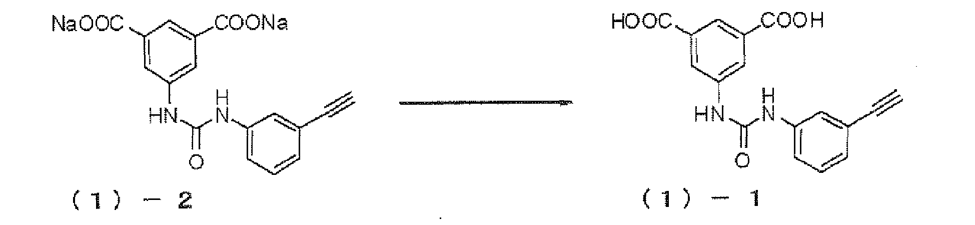

Exemplary Compound (1)-2 (67.4 g) was transferred to a 1000 mL three-necked flask, in which 400 mL of tetrahydrofuran was placed, and was stirred for 15 minutes. The flask was ice-cooled to an internal temperature of 10°C or less, 160 mL of 3M hydrochloric acid was added dropwise to the mixture using a dropping funnel over 10 minutes, and the reaction liquid was stirred for 30 minutes while maintaining the ice-cool.

The reaction liquid was transferred to a 5000 mL separatory funnel and the ethyl acetate phase was extracted by using 2000 mL of ethyl acetate and 800 mL of ion exchanged water, and using a rotary evaporator, a product formed by concentrating and drying the extract in vacuo was dissolved in 200 mL of tetrahydrofuran, and the resultant solution was added dropwise into a 2000 mL three-necked flask, in which 800 mL of acetonitrile was placed, over 30 minutes, and the obtained crystals were filtered, thereby obtaining 42.7 g of the aimed Exemplified Compound (1)-1 (yield: 72%).

MS:M+=327.07
1H-NMR (400 MHz, DMSO-d6): δ=13.21 (s, 2H), δ= 9.21 (s, 1H), δ= 8.86 (s, 1H), δ= 8.42 (s, 2H), δ= 8.02 (s, 1H), δ= 7.81 (s, 1H), δ= 7.62 (d, 1H), δ= 7.29 (t, 1H), δ=7.1.4 (d, 1H), δ= 4.18 (s, 1H).

### [Example 7]

Exemplary Compound (1)-1 was synthesized by a one-continuous method according to the following Formula:

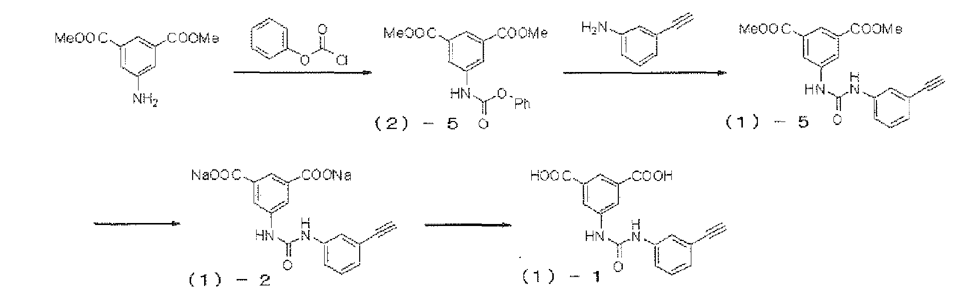

To a 2000 mL three-necked flask, were introduced 52 g of dimethyl 5-aminoisophthalate, 600 mL of tetrahydrofuran and 21 g of pyridine, and the mixture was stirred for 15 minutes. Phenyl chlorocarbonate (41 g) diluted with 100 mL of tetrahydrofuran was added dropwise by using a dropping funnel over 10 minutes with stirring to the mixture in the 2000 mL three-necked flask, and with stirring continuously at room temperature for 3 hours.

Subsequently, 30 g of 3-ethynylaniline was added dropwise by using a dropping funnel over 10 minutes. Subsequently, 76 g of triethylamine diluted with 100 mL of tetrahydrofuran was added dropwise by using a dropping funnel over 10 minutes, and the resultant reaction liquid was heated and refluxed for 4 hours.

After returning to room temperature, an aqueous sodium hydroxide solution prepared from 25 g of sodium hydroxide and 200 mL of ion exchanged water was added dropwise by using a dropping funnel over 10 minutes, and the mixture was heated to 40°C with stirring continuously for 2 hours. The flask was ice-cooled to an internal temperature of 10°C or less, 160 mL of 3M hydrochloric acid was added dropwise by using a dropping funnel over 10 minutes, and the mixture was stirred for 30 minutes while maintaining the ice-cool.

The reaction liquid was transferred to a 5000 mL separatory funnel and the ethyl acetate phase was extracted by using 800 mL of ion exchanged water, and using a rotary evaporator, a product formed by concentrating and drying the extract in vacuo was dissolved in 200 mL of tetrahydrofuran, and the solution was added dropwise into a 2000 mL three-necked flask, in which 800 mL of acetonitrile was placed, over 30 minutes, and the obtained crystals were filtered, thereby obtaining 49.2 g of the aimed Exemplified Compound (1)-1 (yield: 61%).

MS:M+=324.07
1H-NMR (400 MHz, DMSO-d6): δ=13.21 (s, 2H), δ= 9.21 (s, 1H), δ= 8.86 (s, 1H), δ= 8.42 (s, 2H), δ= 8.02 (s, 1H), δ= 7.81 (s, 1H), δ= 7.62 (d, 1H), δ= 7.29 (t, 1H), δ= 7.14 (d, 1H), δ= 4.18 (s,1H).

### [Example 8]

Exemplary Compound (1)-12 was synthesized by a one-continuous method according to the following Formula:

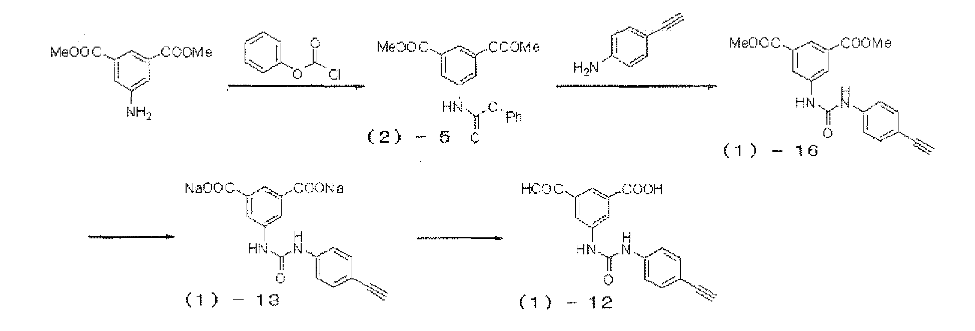

The method was the same as that in Example 7. except that 4-ethynylaniline was used in place of 3-ethynylaniline. The yield was 52.4 g. (yield: 65%).

MS:M+=324.07
1H-NMR (400 MHz, DMSO-d6): δ=13.21 (s, 2H), δ= 9.21 (s, 1H), δ= 8.86 (s, 1H), δ= 8.42 (s, 2H), δ= 8.02 (s, 1H), δ= 7.61 (d, 2H), δ= 7.40 (d, 2H), δ= 4.20 (s, 1H).

### [Example 9]

Exemplary compound (1)-16 was synthesized according to the following Formula:

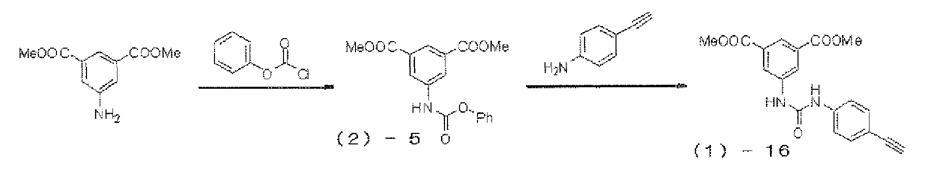

The method was the same as that in Example 7, except that 4-ethynylaniline was used in place of 3-ethynylaniline. The yield was 70.8 g. (yield: 80%).

MS:M+=352.11
1H-NMR (400 MHz, DMSO-d6): δ=9.21 (s, 1H), δ= 8.86 (s, 1H), δ= 8.42 (s, 2H), δ= 8.02 (s, 1H), δ= 7.61 (d, 2H), δ= 7.40 (d, 2H), δ= 4.35 (s, 6H), δ= 4.19 (s, 1H).

### [Example 10]

Exemplary compound (1)-95 was synthesized according to the following Formula:

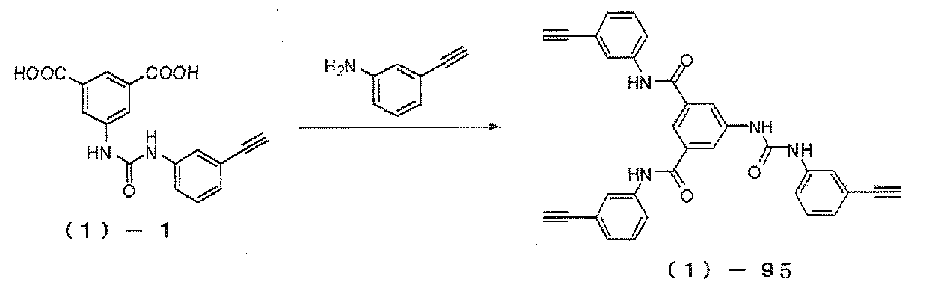

To a three-necked flask, were introduced 12 g of polyphosphoric acid and 12g of 3-ethynylaniline, and subsequently was introduced 32 g of (1)-1 synthesized according to the method of Example 6, and the mixture was heated to 200°C and was stirred continuously for 12 hours. The obtained reaction liquid was introduced into a beaker, in which 500 mL of ion exchanged water was placed, and after the solid of the aimed product thus obtained was washed with an aqueous sodium hydrogencarbonate solution, and subsequently with ion exchanged water and methanol, the solid was filtered off, thereby obtaining 34 g of the aimed Exemplary Compound (1)-95 (yield: 65%).

### [Example 1a]

Exemplary Compound (1)-67a was synthesized according to the following Formula:

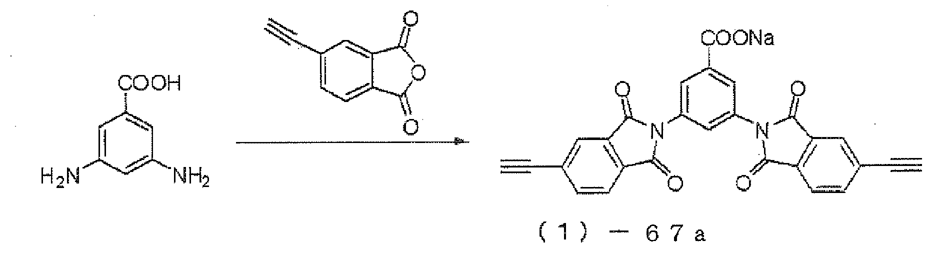

To a 5000 mL three-necked flask, were introduced 61 g of 3,5-diaminobenzoic acid and 400 mL of N-methyl-2-pyrrolidone, and the mixture was stirred until the solute was completely dissolved. To a 500 mL conical flask, were introduced 120 g of 5-ethynyl isobenzofuran-1,3-dione and 700 mL of N-methyl-2-pyrrolidone, and the mixture was stirred until the solute was completely dissolved, thereby forming a dropping liquid 1. The dropping liquid 1 was added dropwise by using a dropping funnel over 20 minutes to the mixture in the 5000 mL three-necked flask, with stirring continuously, and the resultant mixture was heated to 40°C, with stirring continuously for 4 hours.
After the flask was cooled to room temperature, a liquid, in which 5.8 g of pyridine was dissolved in 20 mL of N-methyl-2-pyrrolidone, was added to the 5000 mL three-necked flask, subsequently, a mixed liquid of 310 g of acetic anhydride and 300 g of N-inethyl-2-pyrrolidone was added dropwise by using a dropping funnel over 20 minutes to the 5000 mL three-necked flask, and the resultant mixture was stirred continuously for 4 hours, and was allowed to stand overnight after stopping the stirring.
The flask was ice-cooled at the time when stirring was restarted, an aqueous solution, in which 510 g of sodium hydrogencarbonate was dissolved in 3200 mL of ion exchanged water, was added dropwise into the flask over 2 hours, and by precipitating and filtrating as a sodium salt, 131 g of the aimed Exemplary Compound (1)-67 was obtained (yield; 68%).
MS:M+ = 482.38.

### [Example 2a]

Exemplary compound (1)-66a was synthesized according to the following Formula:

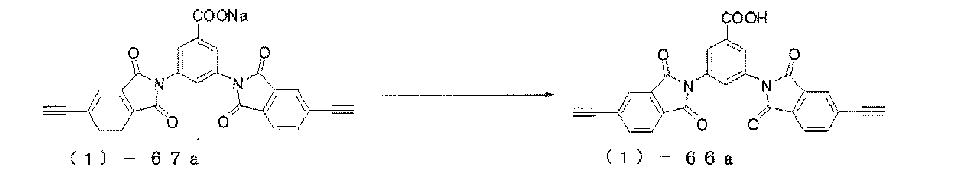

Exemplary Compound (1)-67a (131 g) was neutralized in an aqueous hydrochloric acid solution prepared from 350 mL of concentrated hydrochloric acid and 1000 mL of ion exchanged water, and crude crystals of Exemplary Compound (1)-66a was obtained. The crude crystals were washed with 1000 mL of ion exchanged water, and were filtrated off. After the filtered product was introduced into a 3000 mL three-necked flask, and was dissolved in 1800 mL of N-methyl-2-pyrrondonc, a mixed liquid of 210 mL of acetonitrile and 490 mL of ion exchanged water was added dropwise by using a dropping funnel to the solution over one hour, thereby precipitating Exemplary Compound (1)-66a. The crystals were washed with 500 mL of acetonitrile and filtrated off, thereby obtaining 80 g of the aimed Exemplary Compound (1)-66a (yield: 64%).

MS:M+=460.39
1H-NMR (400 MHz, DMSO-d6): δ=12.79 (s, 1H), δ= 8.36 (s, 1H), δ= 8.29 (s, 2H), δ= 8.25 (s, 2H), δ=8.10 (d, 2H), δ= 7.85 (d, 2H), δ= 4.65 (s. 2H).

### [Example 3a]

Exemplary Compound (1)-70a was synthesized according to the following Formula::

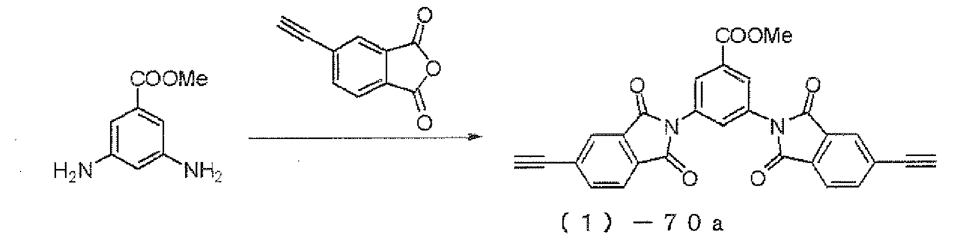

To a 5000 mL three-necked flask, were introduced 66 g of methyl 3,5-diaminobenzoate and 400 mL of N-methyl-2-pyrrolidone, and the mixture was stirred until the solute was completely dissolved. To a 500 mL conical flask, were introduced 120 g of 5-ethynyl isobenzofuran-1,3-dione and 700 mL of N-methyl-2-pyrrolidone, and the mixture was stirred until the solute was completely dissolved, thereby forming a dropping liquid 1. The dropping liquid 1 was added dropwise by using a dropping funnel over 20 minutes to the mixture in the 5000 mL three-necked flask, with stirring continuously, and the resultant mixture was heated to 40°C with stirring continuously for 4 hours. After the flask was cooled to room temperature, a liquid in which 5.8 g of pyridine was dissolved in 20 mL of N-methyl-2-pyrrolidone was added to the 5000 mL three-necked flask, subsequently, a mixed liquid of 310 g of acetic anhydride and 300 mL of N-methyl-2-pyrrolidone was added dropwise by using a dropping funnel over 20 minutes to the 5000 mL three-necked flask, and the resultant mixture was stirred continuously for 4 hours. The precipitation of Exemplary Compound (1)-70a was observed with the progress of the reaction.
After terminating the reaction, the crystals of Exemplary Compound (1)-70a was washed with 500 mL of acetonitrile and filtered off, and 93 g of the aimed Exemplary Compound (1)-70a was obtained (yield: 49%).

MS:M+=474.42
1H-NMR (400 MHz, DMSO-d6): δ= 8.36 (s, 1H), δ= 8.29 (s, 2H), δ= 8.25 (s, 2H), δ= 8.10 (d, 2H), δ= 7.85 (d, 2H), δ= 4.65 (s, 2H), δ= 3.88 (s, 3H).

### [Example 4a]

Exemplary compound (1)-5a was synthesized according to the following Formula::

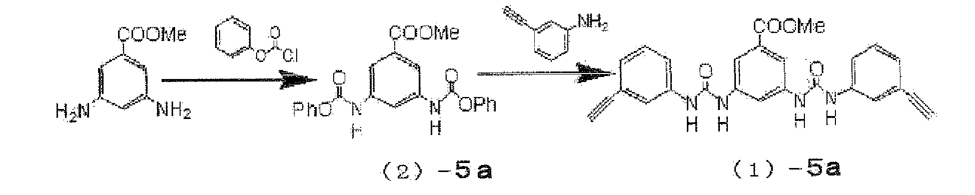

To a 2000 mL three-necked flask, were introduced 66 g of methyl 3,5-diaminobenzoate and 600 mL of N-methyl-2-pyrrolidone, and stirred for 15 minutes. To the 2000 mL three-necked flask, was added dropwise 82 g of phenyl chlorocarbonate by using a dropping funnel over 10 minutes, and the mixture was stirred at room temperature for 30 minutes.
Subsequently, 60 g of 3-ethynylaniline was added dropwise by using a dropping funnel over 10 minutes. Subsequently, 153 g of triethylamine was added dropwise by using a dropping funnel over 10 minutes, and the resultant mixture was heated and refluxed for 4 hours
The reaction liquid was transferred to a 5000 mL separatory funnel and the ethyl acetate phase was extracted by using 2500 mL of ethyl acetate and 800 mL of ion exchanged water, and using a rotary evaporator, a product formed by concentrating and drying the extract in vacuo was dissolved in 200 mL of tetrahydrofuran, and 150 mL of ethyl acetate was added thereto, and further 100 mL of hexane was added dropwise thereto over 30 minutes, and the obtained crystals were filtered, thereby obtaining 114 g of the aimed Exemplified Compound (1)-5a (yield: 63%).

MS:M+=452.46
1H-NMR (400 MHz, DMSO-d6): δ=9.07 (s, 2H), δ= 8.81 (s, 2H), δ= 8.23 (s, 1H), δ= 7.95 (s, 2H), δ= 7.81 (s, 2H), δ= 7.62 (d, 2H), δ= 7.29 (t, 2H), δ= 7.14 (d, 1H), δ= 4.36 (s, 3H), δ= 4.15 (s, 2H).

### [Example 5a]

Exemplary compound (1)-1a was synthesized based on the following formula:

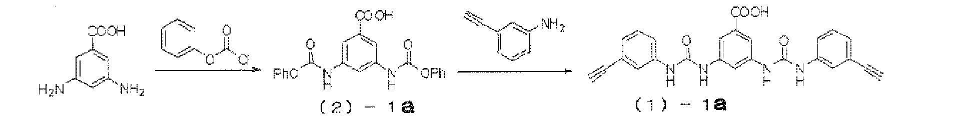

To a 2000 mL three-necked flask, were introduced 61 g of 5-diaxninomethylbenzoic acid and 600 mL of N-methyl-2-pyrrolidone, and stirred for 15 minutes. To the 2000 mL three-necked flask, was added dropwise 143 g of phenyl chlorocarbonate by using a dropping funnel over 10 minutes, and the mixture was stirred at room temperature for 30 minutes.
The reaction liquid was transferred to a 5000 mL separatory funnel and the ethyl acetate phase was extracted by using 2500 mL of the ethyl acetate and 800 mL of ion exchanged water, and using a rotary evaporator, a product formed by concentrating and drying the extract in vacuo was dissolved in 600 mL N-methyl-2-pyrrolidone, and the resultant solution was transferred to a 2000 mL three-necked flask.
Subsequently, 60 g of 3-ethynylaniline was added dropwise by using a dropping funnel over 10 minutes. Subsequently, 153 g of triethylamine was added dropwise by using a dropping funnel over 10 minutes, and the resultant mixture was heated and refluxed for 4 hours.
The reaction liquid was transferred to a 5000 mL separatory funnel and the ethyl acetate phase was extracted by using 2500 mL of the ethyl acetate and 800 mL of ion exchanged water, and using a rotary evaporator, a product formed by concentrating and drying the extract in vacuo was dissolved in 200 mL of tetrahydrofuran, and 150 mL of ethyl acetate was added thereto, and further 100 mL of hexane was further added dropwise thereto, and the obtained crystals were filtered, thereby obtaining 81 g of the aimed Exemplified Compound (1)-la (yield: 46%).

MS:M+=438.43
1H-NMR (400 MHz, DMSO-d6): δ=12.79 (s, 1H), δ= 9.07 (s, 2H), δ= 8.81 (s, 2H), δ= 8.31 (s, 1H), δ= 8.02 (s, 2H), δ= 7.81 (s, 2H), δ= 7.62 (d, 2H), δ= 7.29 (t, 2H), δ= 7.14 (d, 1H), δ= 4.16 (s, 2H).

### [Example 6a]

Exemplary compound (1)-2a was synthesized according to the following Formula:

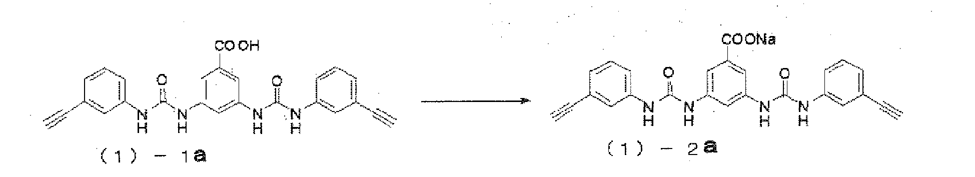

Exemplary compound (1)-1a (81 g) was dissolved in 500 mL of N-methyl-2-pyrrolidone, and to the solution was added dropwise an aqueous sodium hydroxide solution prepared from 25 g of sodium hydroxide and 200 ml of ion exchanged water by using a dropping funnel over 10 minutes, and the resultant solution was continuously stirred for 2 hours.
The reaction liquid was transferred to a 2000 mL three-necked flask, in which 1000 mL of acetonitrile was placed, and the obtained crystals were filtered, and 60.3 g of the aimed Exemplary Compound (1)-2a (yield: 71%).
MS:M+=460.42

### [Example 7a]

Exemplary compound (1)-1 6a was synthesized according to the following Formula:

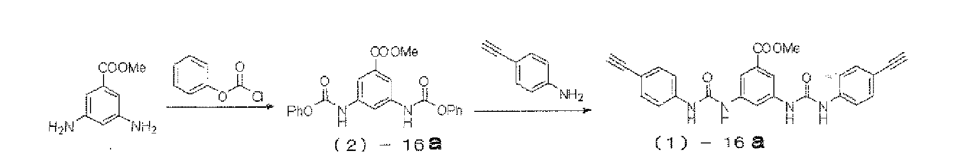

The method was the same as that in Example 4a, except that 4-ethynylaniline was used in place of 3-ethynylaniline. The yield was 119 g (yield: 66%).
MS:M+=452.46
1H-NMR (400 MHz, DMSO-d6): δ=9.07 (s, 2H), δ= 8.81 (s, 2H), δ= 8.23 (s, 1H), δ= 7.95 (s, 2H), δ= 7.61 (d, 4H), δ= 7.40 (d, 4H), δ= 4.36 (s, 3H), δ**=** 4.15 (s, 2H).

### [Example 8a]

Exemplary compound (1)-12a was synthesized by a one-continuous method according to the following Formula::

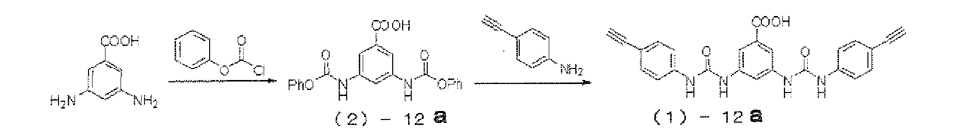

The method was the same as that in Example 5a, except that 4-ethynylaniline was used in place of 3-ethynylaniline. The yield was 73.7 g (yield: 42%).
MS:M+=438.43
1H-NMR (400 MHz, DMSO-d6): δ=13.21 (s, 1H), δ= 9.07 (s, 2H), δ= 8.81 (s, 2H), δ= 8.31 (s, 1H), δ= 8.02 (s, 2H), δ= 7.61 (d, 4H), δ= 7.40 (d, 4H), δ= 4.15 (s, 2H).

### [Example 9a]

Exemplary Compound (1)-98a was synthesized according to the following Formula:

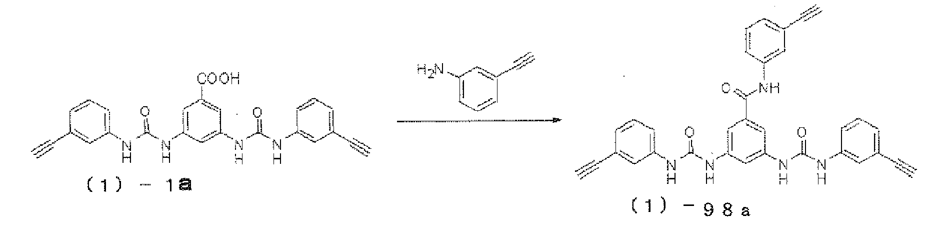

To a three-necked flask, were introduced 12 g of polyphosphoric acid and 12 g of 3-ethynylaniline, and subsequently to the mixture was introduced 54 g of (1)-la synthesized by the method of Example 6a, and the mixture was heated to 200°C and was continuously stirred for 12 hours. The obtained reaction liquid was introduced into a beaker, in which 500 mL of ion exchanged water was placed, and after the solid of the aimed product thus obtained was washed with an aqueous sodium hydrogencarbonate solution, followed washing with ion exchanged water and methanol, the solid was filtered off, thereby obtaining 30.6 g of the aimed Exemplary Compound (1)-98a (yield: 57%).

### [Comparative Example 1]

According to Example 1 in Patent Literature 2 (JP-A No. 2002-265414), 4.5 g of 5-(4-(2-phenylethynyl)phenoxy) isophthalic acid was obtained. MS:M+==358.08.

### [Comparative Example 1a]

According to Example 2 of Patent Literature (Japanese Patent Application Laid-Open No. 3-227954), 45 g of meta-ethynylbenzoic acid was obtained.
MS:M+=146.14

### < Evaluation of physical properties of polymer>

### [Example 11]

### Synthesis of polybenzimidazole containing Exemplary Compound (1)-63

To a three-necked flask, were introduced 120 g of polyphosphoric acid and 23.6 g of 3,3',4,4'-tetraaminobiphenyl, and the mixture was heated to 100°C. Subsequently, 0.68 g of Exemplary Compound (1)-63 and 16.3 g of isophthalic acid were added thereto, and heated 200°C, and the mixture was continuously stirred for 12 hours. The resultant reaction liquid was introduced into a beaker, in which 500 mL of ion exchanged water was placed, and after the solid of the aimed product thus obtained was washed with an aqueous sodium hydrogencarbonate solution, followed washing with ion exchanged water and methanol, the solid was filtered off, thereby obtaining polybenzimidazole having an average molecular weight about 30,000.

### [Example 12]

### Synthesis of polybenzimidazole containing Exemplary Compound (1)-1

The synthetic method was the same as that in Example 11, except that 0.65 g of Exemplary Compound (1)-1 was used in place of 0.68 g of Exemplary Compound (1)-63.

### [Example 13]

### Synthesis of polybenzoxazole containing Exemplary Compound (1)-1

The synthetic method was the same as that in Example 11, except that 0.65 g of Exemplary Compound (1)-1 was used in place of 0.68 g of Exemplary Compound (1)-63, and 23.8 g of 3,3'-diarninn-4,4'-dihydroxybiphenyl was used in place of 23.6 g of 3,3',4,4'-tetraaminobiphenyl.

### [Example 10a]

### Synthesis of polybenzimidazole containing Exemplary Compound (1)-63a

To a three-necked flask, were introduced 120 g of polyphosphoric acid and 22.5 g of 3,3',4,4'-tetraaminobiphenyl, and the mixture was heated to 100°C, and subsequently 16.6 g of isophthalic acid was added thereto, heated to 200°C, and the resultant mixture was continuously stirred for 12 hours. Further, to the mixture, were added 10g of polyphosphoric acid and 2.3 g of Exemplary Compound (1)-63a, with stirring continuously for 12 hours.

The obtained reaction liquid was introduced into a beaker, in which 500 mL of ion exchanged water was placed, and after the solid of the aimed product thus obtained was washed with an aqueous sodium hydrogencarbonate solution, followed washing with ion exchanged water and methanol, the solid was filtered off, thereby obtaining a polymer compound, in which Exemplary Compound (1)-68a was bonded to the terminal end of polybenzimidazole having an average molecular weight of about 30,000.

### [Example 11a]

### Synthesis of polybenzimidazole containing Exemplary Compound (1)-1a

The synthetic method was the same as that in Example 10a except that 2.2 g of Exemplary Compound (1)-la was used in place of 2.3 g of Exemplary Compound (1)-63.

### [Example 12a]

### Synthesis of polybenzoxazole containing Exemplary Compound (1)-1a

The synthetic method was the same as that in Example 10a, except that 22.7 g, of . 3,3'-diamino-4.4'-dihydroxybiphenyl was used in place of 22.5 g of 3.3',4,4'-tetraaminobiphenyl.

### [Comparative Example 2]

### Synthesis of poly benzimidazole containing 5-(4-(2-phenylethynyl)phenoxy) isophthalic acid

The synthetic method was the same as that in Example 11, except that 0.72 g of 5-(4-(2-phenylethynyl)phenoxy) isophthalic acid synthesized by the method of Comparative Example 1 was used in place of 0.65 g of (1)-1.

### [Comparative Example 2a]

### Synthesis ofpolybenzimidazole containing meta-ethynylbenzoic acid

The synthetic method was the same as that in Example 10a, except that 0.74 g of meta-ethynylbenzoic acid synthesized by the method of Comparative Example 1a was used in place of 2.3 g of Exemplary Compound (1)-63a.

### [Example 13a]

Polybenzimidazole derivatives obtained by Example 5a, Example 6a and Comparative Example 2a each were are dissolved in an amount of 2 g in 100 mL of N,N-dimethyl acetamide, the solutions each were coated by using a coil bar on a quartz glass substrate and dried, and the coated substrates were subjected to a thermal curing treatment at 400°C. The tensile strength of the polybenzimidazole films formed on the quartz glass substrate was measured by using a tensile strength meter (STROGRAPH VC-1; trade name); manufactured by Toyo Seiki Seisaku-sho Ltd. The measurement results are shown in Table 1.

**Table 1.**

| Synthetic Method of Polymer Containing Acetylene Compound | Tensile Strength [MPa] |
|---|---|
| Example 11 a | 160.9 |
| Example 12a | 160.7 |
| Comparative Example 2a | 158.9 |

### [Example 14]

Polybenzimidazole derivatives obtained by Example 5, Example 6 and Comparative Example 2 each were are dissolved in an amount of 2 g in 100 mL of N,N-dimethyl acetamide, the solutions each were coated by using a coil bar on a quartz glass substrate and dried, and the coated substrates were subjected to a thermal curing treatment at 400°C. The tensile strength of the polybenzimidazole films formed on the quartz glass substrate was measured by using a tensile strength meter (STROGRAPH VC-1; trade name); manufactured by Toyo Seiki Seisaku-sho Ltd.

**Table 2.**

| Synthetic Method of Polymer Containing Acetylene Compound | Tensile Strength [MPa] |
|---|---|
| Example 11 | 161.6 |
| Example 12 | 161.2 |
| Comparative Example 2 | 160.4 |

As is clear from Table 1 and Table 2, it can be seen that the films obtained from the polymers containing the acetylene compound obtained by the invention have excellent tensile strength.

### [Industrial Applicability]

The novel acetylene compounds provided by the present invention are introduced into a polymer and the polymer is subjected to a thermal curing treatment, thereby obtaining a thermally curable polymer capable of improving mechanical strength, heat resistance and chemical resistance.

The foregoing description of the exemplary embodiments of the present invention has been provided for the purposes of illustration and description. It is not intended to be exhaustive or to limit the invention to the precise forms disclosed. The embodiments were chosen and described in order to best explain the principles of the invention and its practical applications, thereby enabling others skilled in the art to understand the invention for various embodiments and with the various modifications as are suited to the particular use contemplated.

All publications, patent applications, and technical standards mentioned in this specification are herein incorporated by reference to the same extent as if such individual publication, patent application, or technical standard was specifically and individually indicated to be incorporated by reference. It will be obvious to those having skill in the art that many changes may be made in the above-described details of the preferred embodiments of the present invention. It is intended that the scope of the invention be defined by the following claims and their equivalents.

## Claims

1. A compound represented by the following Formula (1): 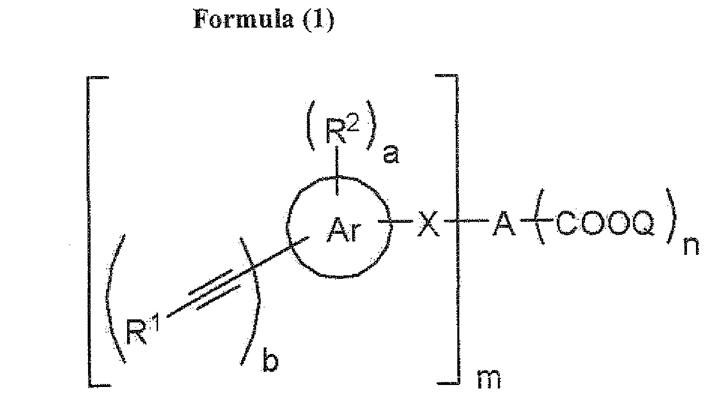 wherein, in Formula (1), the encircled Ar represents an aryl group or a heteroaryl group; X represents a divalent linking group represented by -NR(C=C)-, -NR(C=O)O-, -NR(C=O)NR'-, -(C=O)-, -(C=O)O-, -O(C=O)O-, -(C=O)S-, -NR(C=S)-, -NR(C=S)NR'-, -O(C=S)O-, -O- or -S-; R, R' and R¹each independently represent a hydrogen atom, a hydrocarbon group or a heterocyclic group; R² represents a hydrogen atom or a substituent substitutable on a benzene ring; A represents a hydrocarbon group or a heterocyclic group; Q represents a hydrogen atom, a hydrocarbon group, or a metal element capable of forming a monovalent metal salt; a represents an integer 0 or more; b represents an integer 1 or more; m represents an integer I or more; n represents and integer 1 or more; when n, m and b are 1 at the same time, X is not -(C=O)O-; and when n is 2, and both m and b are 1, X is not -O-.

2. The compound according to claim 1, wherein the compound according to claim 1 is represented by the following Formula (2): 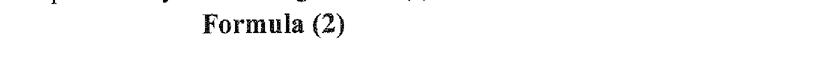 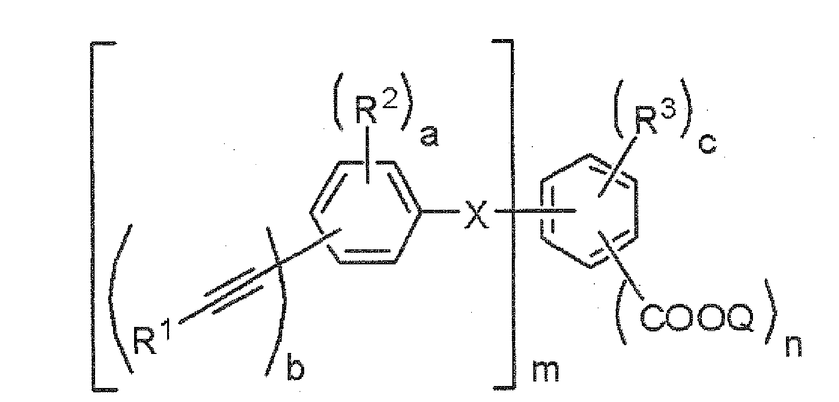 wherein, in Formula (2), X represents a divalent linking group represented by -NR(C=O)-, -NR(C=O)O-, -NR(C=O)NR'-, or -(C=O)O-; R³ represents a hydrogen atom or a functional group substitutable on the benzene ring; R¹; R²; R, R' and Q each have the same definitions as those of R¹; R²; R, R' and Q in Formula (1); a represents an integer from to 4; b represents an integer from 1 to 5; c represents an integer from 0 to 3; m represents an integer from 1 to 5; n represents an integer from 1 to 5; the sum of c, m and n is 6; and when n, m and b are 1 at the same time, X is not -(C=O)O-.

3. The compound according to claim 2, wherein, in Formula (2), m is an integer from 2 to 5; n is 1; and X represents a divalent linking group represented by -NR(C=O)-, -NR(C=O)O-, or -NR(C=O)NR'-.

4. The compound according to claim 2, wherein, in Formula (2), m is 1, and n is an integer from 2 to 5.

5. The compound according to claim 4, wherein, in Formula (2), n is 2.

6. The compound according to claim 5, wherein, in Formula (2), b is 1.

7. The compound according to claim 3, wherein, in Formula (2), m is 2.

8. The compound according to claim 2, wherein, in Formula (2), b is 1; and the substitution position of the ethynyl group substituted by R¹ is the meta-position or the para-position with respect to the linking group X.

9. The compound according to claim 2, wherein the compound represented by Formula (2) is represented by the following Formula (3): 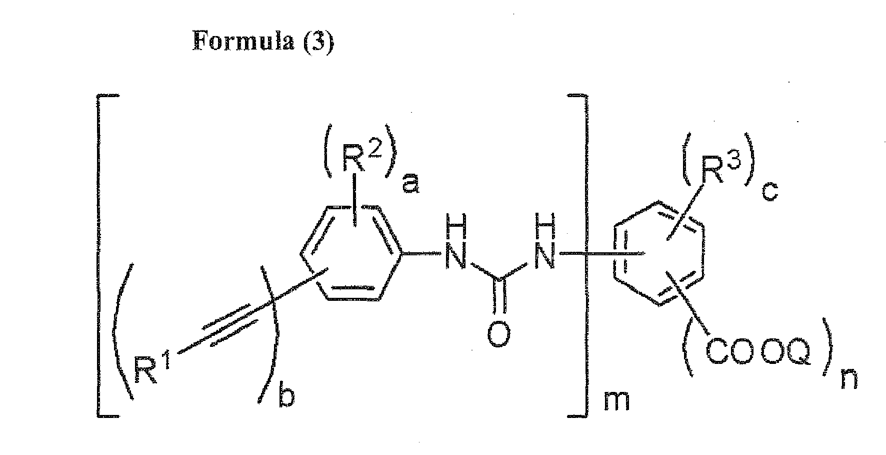 wherein, in Formula (3), R¹, R², R³, Q, a, b, c, m and n each have the same definitions as R¹, R², R³, Q, a, b, c, m and n in Formula (2).

10. The compound according to claim 2, wherein the compound represented by Formula (2) is represented by the following Formula (4): 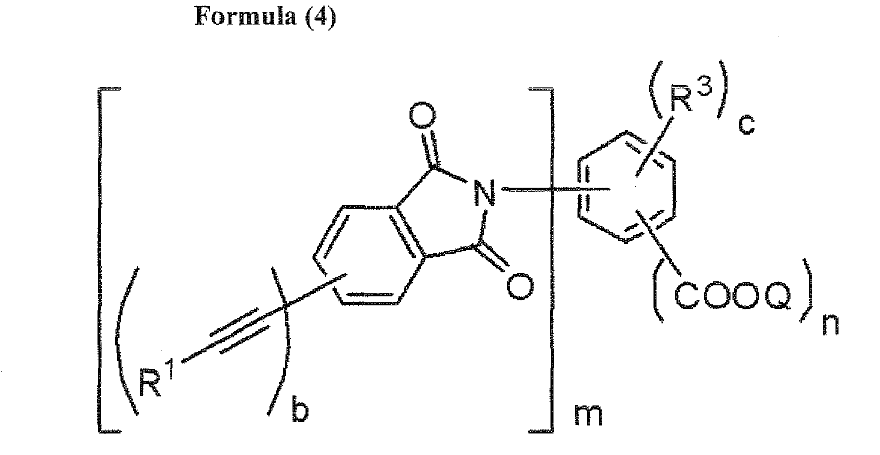 wherein, in Formula (4), b represents an integer from 1 to 4; and R¹, R³, Q, c, m and n each have the same definitions as R¹, R³, Q, c, m and n in Formula (2).

11. The compound according to claim 9, wherein, in Formula (3), m is 2, and n is 1.

12. The compound according to claim 10, wherein, in Formula (4), m is 2, and n is 1.
